(19) Europäisches Patentamt
European Patent Office
Office européen des brevets

(11) Publication number: 0 227 818 B1

(12) # EUROPEAN PATENT SPECIFICATION

(45) Date of publication of patent specification: **09.09.92**

(51) Int. Cl.5: **A61K 45/06**, A61K 9/06, A61K 37/64, //A61K31/535

(21) Application number: **86904589.8**

(22) Date of filing: **02.07.86**

(86) International application number: **PCT/US86/01377**

(87) International publication number: **WO 87/00055 (15.01.87 87/01)**

(54) **ANTIGLAUCOMA COMPOSITIONS.**

(30) Priority: **03.07.85 US 752343**

(43) Date of publication of application: **08.07.87 Bulletin 87/28**

(45) Publication of the grant of the patent: **09.09.92 Bulletin 92/37**

(84) Designated Contracting States: **AT BE CH DE FR GB IT LI LU NL SE**

(56) References cited:

**Chemical Abstracts, volume 87, no. 11, 12 September 1977, (Columbus, Ohio, US), T.J. Zimmerman et al.: "Timolol. A beta-adrenergic blocking agent for the treatment of glaucoma", see page 52, abstract no. 78551w, & Arch. Ophthalmol. 1977, 95 (4), 601-4**

**Journal of ocular Pharmacology, vol. 3, no. 4, 1987, p. 295-307**

(73) Proprietor: **SCHERING CORPORATION**
**2000 Galloping Hill Road**
**Kenilworth New Jersey 07033(US)**

(72) Inventor: **WATKINS, Robert, Wayne**
**RR 1, Box 510B**
**Great Meadows, NJ 07838(US)**
Inventor: **BARNETT, Allen**
**13 Flanders Road**
**Pine Brook, NJ 07058(US)**

(74) Representative: **von Kreisler, Alek, Dipl.-Chem. et al**
**Deichmannhaus am Hauptbahnhof**
**W-5000 Köln 1(DE)**

EP 0 227 818 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Medicinal Research Reviews, vol. 5, no. 4, 1985, p. 483-531

Journal of Cardivascular Pharmacology,, 15, suppl. 2, 1990, s.24-s.28

Survey of Ophthalmology, vol. 31, no. 5, 1987, p. 307-327

The New England Journal of Medicine, vol. 319, no. 23, p. 1517-1525

**Description**

BACKGROUND OF THE INVENTION

Glaucoma is an ocular disease complex associated with an elevated pressure within the eye (i.e., intraocular pressure, IOP). As a result of the elevated IOP, damage to the optic nerve head resulting in irreversible loss of visual function may ensue. Untreated, this condition may eventually lead to blindness.

Ocular hypertension, i.e., the condition of elevated intraocular pressure without optic nerve head damage or characteristic glaucomatous visual field loss, is now believed by the majority of ophthalmologists to represent the earliest phase in the onset of glaucoma.

A number of the drugs presently employed to treat glaucoma ace not entirely satisfactory, particularly in the earliest course of the disease when the side effects they produce are often worse than the symptoms of the disease.

Epinephrine, used as a topical solution, must be utilized cautiously in patients with high blood pressure, diabetes, hyperthyroidism and cerebral artereosclerosis due to the possibility of systemic action.

Timolol, a clinically utilized, topically applied agent for lowering intraocular pressure, must be used with caution in patients in whom Beta-adrenergic blockade may be undesirable. Systemic absorption of topically administered timolol and systemic Beta-blockade are responsible for the contraindication of timolol therapy for glaucoma in patients with compromised pulmonary function and in patients who cannot tolerate its systemic cardiovascular action.

Pilocarpine, a topical drug, although considered systemically harmless and quite effective, may cause considerable local difficulties. Pupillary constriction causes the eye to lose its ability to adapt from light to dark. Accommodation may become stimulated so that the patient's refraction is sometimes incorrect and vision becomes blurred. The drug itself may cause a local vasodilation and red eyes. Irritation is common.

Carbonic anhydrase inhibitors have been used systemically but they have a number of disadvantages. While effective in lowering intraocular pressure, they often cause a numbness and tingling, gastrointestinal upsets and, frequently, depression, lethargy, a loss of appetite, and general malaise.

EP-A-0036351 attempts to overcome these difficulties by the topical administration of an alkali metal salt of a carbonic anhydrase inhibitor.

EP-A-0099239 and EP-A-0114333 describe certain angiotensin converting enzyme inhibitors as being useful in controlling the intraocular pressure.

The present invention provides a new approach in the therapy for reducing and controlling the elevated intraocular pressure associated with glaucoma.

The invention patented in its pharmaceutical composition aspect is a topical ophthalmologically acceptable composition useful for reducing and/or controlling the elevated intraocular pressure associated with glaucoma which comprises a pharmaceutically acceptable angiotensin converting enzyme inhibitor and a pharmaceutically acceptable beta adrenergic blocker in combination with an ophthalmologically acceptable carrier. Preferably the composition comprises for an amount of from 0.005 $\mu$g to 50 $\mu$g of the ACE inhibitor an amount of from 5 $\mu$g to 500$\mu$g of the beta adrenergic blocker.

According to a modification of the invention, pharmaceutical compositions are comprised which contain either the beta adrenergic blocker or the ACE inhibitor for combined use of these active components.

The invention patented in a further aspect is a kit comprising in separate containers in a single package pharmaceutical compositions for use in combination to reduce and control the elevated intraocular pressure associated with glaucoma in humans which comprises in one container a topical ophthalmological pharmaceutical composition comprising an angiotensin converting enzyme inhibitor and in the second container, a topical ophthalmological pharmaceutical composition comprising a beta adrenergic blocker.

Preferably the compositions contain the active components in such concentration that the one composition provides 0.005 to 50$\mu$g of the angiotensin converting enzyme inhibitor per unit dose and the other composition provides 0.05 to 500$\mu$g of a beta adrenergic blocker per unit dose.

Examples of angiotensin converting enzyme (ACE) inhibitors are those disclosed in the following patents and published patent applications:

(a) U.S. Patent No. 4,105,776 to Ondetti et al. discloses proline derivatives which are angiotensin converting enzyme (ACE) inhibitors and have the general formula

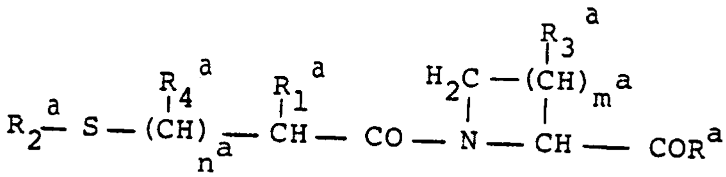

wherein
$R^a$ is hydroxy, $NH_2$ or lower alkoxy;
$R_1{}^a$ and $R_4{}^a$ each is hydrogen, lower alkyl, phenyl or phenyl-lower alkyl;
$R_2{}^a$ is hydrogen, lower alkyl, phenyl, substituted phenyl wherein the phenyl substituent is halo, lower alkyl or lower alkoxy, phenyl-lower alkyl, diphenyl-lower alkyl, triphenyl-lower alkyl, lower alkylthiomethyl, phenyl-lower alkylthiomethyl, lower alkanoyl-amidomethyl,

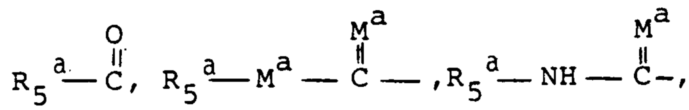

$R_6{}^a$-S-, or $R_7{}^a$;
$R_3{}^a$ is hydrogen, hydroxy or lower alkyl;
$R_5{}^a$ is lower alkyl, phenyl or phenyl-lower alkyl;
$R_6{}^a$ is lower alkyl, phenyl, substituted phenyl (wherein the phenyl substituent is halo, lower alkyl or lower alkoxy), hydroxy-lower alkyl or amino (carboxy)-lower alkyl;

$R_7{}^a$ is 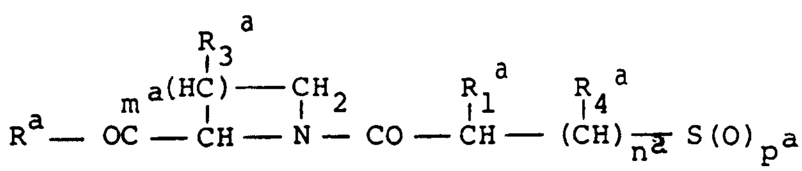

$M^a$ is O or S;
$m^a$ is 1 to 3;
$n^a$ and $p^a$ each is 0 to 2.
(b) EP-A-0097050 discloses phosphinylalkanoyl substituted prolines which have the formula

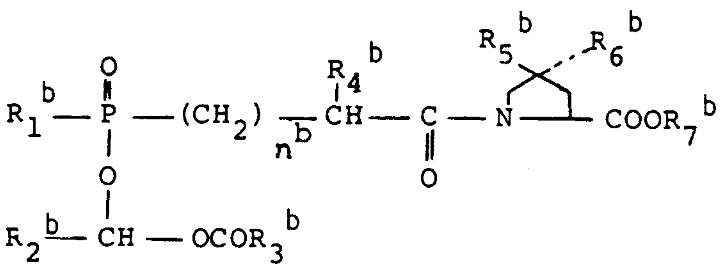

$R_1{}^b$ is alkyl, aryl, aralkyl, cycloalkyl or cycloalkylalkyl;
$R_2{}^b$ is cycloalkyl, 3-cyclohexenyl or 2-alkyl-3-cyclohexenyl;
$R_3{}^b$ is alkyl, cycloalkyl or phenyl;
$R_4{}^b$ is H or alkyl;
one of $R_5{}^b$ and $R_6{}^b$ is H and the other is alkyl-$X^b$-, phenyl-$X^b$, alkoxy, phenoxy, phenyl, cycloalkyl, alkyl or phenylalkyl; or
$R_5{}^b$ and $R_6{}^b$ together form $-X^bCH_2CH_2X^b-$, $X^b$ is S, SO or $SO_2$;
$R_7{}^b$ is H or $CH(R_2{}^b)OCOR_3{}^b$;

$n^b$ is 0 or 1;
'aryl' is phenyl opt. substituted by halo, alkyl, alkoxy, alkylthio, OH, alkanoyl, $NO_2$, amino, dialkylamino and/or $CF_3$;
alkyl has 1-10C, cycloalkyl 3-7C, alkoxy 1-8C and alkanoyl 2-9C atoms.

(c) EP-A-0083172 discloses N-substituted acetyl-L-proline derivatives which are said to be angiotensin converting enzyme inhibitors and have the formula

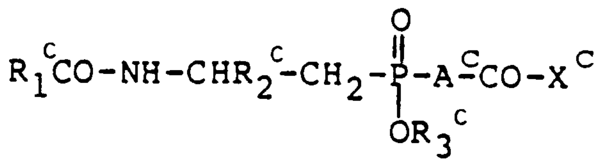

$R_1{}^c$ and $R_2{}^c$ are H, 1-7C alkyl, 1-7C haloalkyl, $(CH_2)_m c\text{-}D^c$, 1-7C aminoalkyl or a group of the formula:

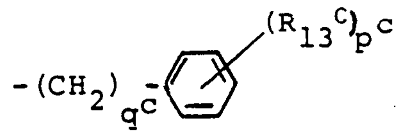

$D^c$ is cycloalkyl, furyl, thienyl or pyridinyl;
$A^c$ is $(CH_2)_n\text{-}CHR_{21}{}^c$, $NR_{22}{}^c\text{-}CHR_{23}{}^c$ or $O\text{-}CHR_{23}{}^c$;
$n^c$ is 0 or 1;
$q^c$ is 0-7;
$R_{21}{}^c$ is H, 1-7C alkyl, 1-7C haloalkyl, benzyl or phenethyl;
$R_{22}{}^c$ is H or 1-7C alkyl;
$R_{23}{}^c$ is H, 1-7C alkyl, 1-7C haloalkyl or $(CH_2)_r cD_1{}^c$;
$D_1{}^c$ is phenyl, 4-hydroxyphenyl, 3,4-dihydroxyphenyl, 3-indolyl, 4-imidazolyl, $NH_2$, SH, 1-7C alkylthio, guanidino or $CONH_2$;
$X^c$ is a group of formula $(II)^c$-$(V)^c$, or $NR_4{}^c\text{-}CHR_5{}^c\text{-}COOR_6{}^c$;

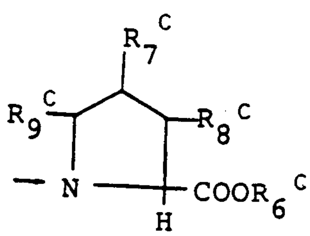

(II)$^c$

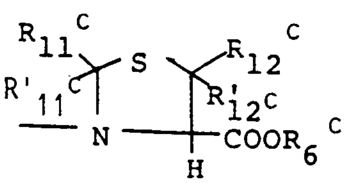

(III)$^c$

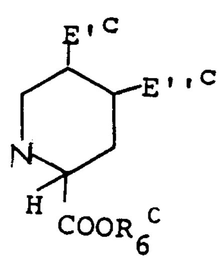

(IV)$^c$

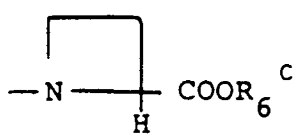

(V)$^c$

where
$R_9{}^c$ and $R_8{}^c$ are H and $R_7{}^c$ is as defined below;
$R_9{}^c$ and $R_7{}^c$ are H and $R_8{}^c$ is as defined below;
$R_7{}^c$ and $R_8{}^c$ are H and $R_9{}^c$ is as defined below;
$R_9{}^c$ and $R_8{}^c$ are H and $-CHR_7{}^c-$ is replaced by $-CR_{10}{}^cR_{10}{}^c$;
$R_9{}^c$ is H and $R_7{}^c + R_8{}^c$ form a double bond;

$R_9{}^c$ is H and $R_7{}^c$ + $R_8{}^c$ complete a fused benzene ring; or
$R_8{}^c$ is H and $R_9{}^c$ + $R_7{}^c$ complete a fused benzene ring;
$E'^c$ and $E''^c$ are H or $E'^c$ + $E''^c$ complete a fused benzene ring;
$R_7{}^c$ is H, 1-7C alkyl, halogen, keto, OH, 2-8C alkanoyl-amino, $N_3$, $NH_2$, $NR_{19}{}^cR_{20}{}^c$, $(CH_2)_m{}^c$-$D^c$, O-$CONR_{15}{}^cR_{15}{}^c$, 1-7C alkoxy, 1-7C alkylthio, or a group of formula $(VI)^c$, $(VII)^c$ or $(VIII)^c$:

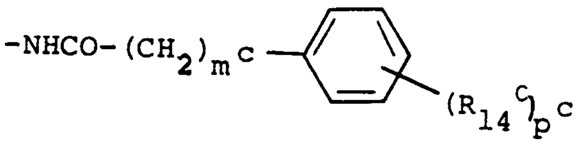

$(VI)^c$

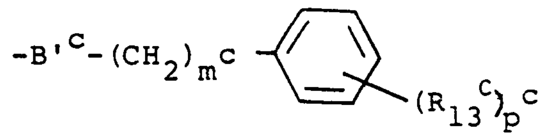

$(VII)^c$

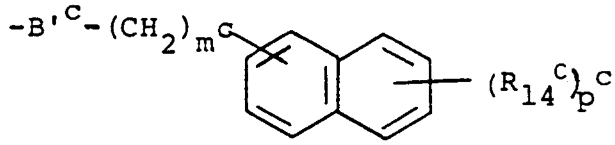

$(VIII)^c$

$B'^c$ is a bond or it is O or S;$R_8{}^c$ is keto, halogen, O-$CONR_{15}{}^cR_{15}{}^c$, 1-7C alkoxy, 1-7C alkylthio, or a group $(VII)^c$ or $(VIII)^c$ in which $B'^c$ is O or S;
$R_9{}^c$ is Keto or a group $(VII)^c$ in which $B'^c$ is a bond; $R_{10}{}^c$ is halogen or $Y^cR_{16}{}^c$;
$R_{11}{}^c$, $R'_{11}{}^c$, $R_{12}{}^c$ and $R'_{12}$ are H or 1-7C alkyl, or $R_{11}{}^c$ is a group of formula $(IX)^c$ and the other 3 are H;

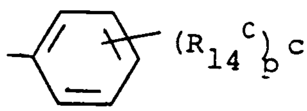

$(IX)^c$

$R_{13}{}^c$ is H, 1-4C alkyl, 1-4C alkoxy, 1-4C alkylthio, Cl, Br, F, $CF_3$, OH, Ph, PhO, PhS or $PhCH_2$;
$R_{14}{}^c$ is H, 1-4C alkyl, 1-4C alkoxy, 1-4C alkylthio, Cl, Br, F, $CF_3$ or OH;
$m^c$ is 0-3;
$p^c$ is 1-3 but it is 2 or 3 only when $R_{13}{}^c$ or $R_{14}{}^c$ is H, Me, MeO, Cl or F;
$R_{15}{}^c$ is H or 1-4C alkyl;
$Y^c$ is O or S;
$R_{16}{}^c$ is 1-4C alkyl or group $(VII)^c$ in which $B'^c$ is a bond; or the two $R_{16}{}^c$ groups complete a 5-or 6-membered ring in which the C atoms may each bear 1 or 2 alkyls having 1-4C atoms;
$R_4{}^c$ is H, 1-7C alkyl, cycloalkyl or $(CH_2)_r{}^cPh$; $R_5{}^c$ is H, 1-7C alkyl or $(CH_2)_r{}^c$-$D_1{}^c$;
$r^c$ is 1-4;
$R_3{}^c$ and $R_6{}^c$ are H, 1-7C alkyl, $PhCH_2$, PhCH or $CHR_{17}{}^c$-O-$COR_{18}{}^c$;
$R_{17}{}^c$ is H, 1-7C alkyl or Ph;
$R_{18}{}^c$ is H, 1-7C alkyl, 1-7C alkoxy or Ph; or
$R_{17}{}^c$ + $R_{18}{}^c$ form $(CH_2)_2$, $(CH_2)_3$, -CH=CH- or o-phenylene;
$R_{19}{}^c$ is 1-7C alkyl, benzyl or phenethyl; and
$R_{20}{}^c$ is H or chosen as for $R_{19}{}^c$.
(d) EP-A-0 012 401 discloses carboxyalkyl dipeptide derivatives which are said to be angiotensin converting enzyme inhibitors and have the formula

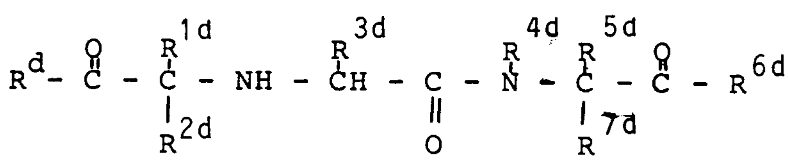

wherein

$R^d$ and $R^{6d}$ are the same or different and are hydroxy, lower alkoxy, lower alkenoxy, dilower alkylamino lower alkoxy (dimethylaminoethoxy), acylamino lower alkoxy (acetylaminoethoxy), acyloxy lower alkoxy (pivaloyloxymethoxy), aryloxy, such as phenoxy, arloweralkoxy, such as benzyloxy, substituted aryloxy or substituted arloweralkoxy wherein the substituent is methyl, halo or methoxy, amino, lower alkylamino, dilower alkylamino, hydroxyamino, arloweralkylamino such as benzylamino;

$R^{1d}$ is hydrogen, alkyl of from 1 to 20 carbon atoms which include branched and cyclic and unsaturated (such as allyl) alkyl groups, substituted lower alkyl wherein the substituent can be halo, hydroxy, lower alkoxy, aryloxy such as phenoxy, amino, diloweralkylamino, acylamino, such as acetamido and benzamido, arylamino, guanidino, imidazolyl, indolyl, mercapto, lower alkylthio, arylthio such as phenylthio, carboxy or carboxamido, carboloweralkoxy, aryl such as phenyl or naphthyl, substituted aryl such as phenyl wherein the substituent is lower alkyl, lower alkoxy or halo, arloweralkyl, arloweralkenyl, heteroarloweralkyl or heteroarloweralkenyl such as benzyl, styryl or indolyl ethyl, substituted arloweralkyl, substituted arloweralkenyl, substituted heteroarloweralkyl, or substituted heteroarloweralkenyl, wherein the substituent(s) is halo, dihalo, lower alkyl, hydroxy, lower alkoxy, amino, aminomethyl, acylamino (acetylamino or benzoylamino) diloweralkylamino, lower alkylamino, carboxyl, haloloweralkyl, cyano or sulfonamido;

arloweralkyl or heteroarloweralkyl substituted on the alkyl portion by amino or acylamino (acetylamino or benzoylamino);

$R^{2d}$ and $R^{7d}$ are the same or different and are hydrogen or lower alkyl;

$R^{3d}$ is hydrogen, lower alkyl, phenyl lower alkyl, aminomethyl phenyl lower alkyl, hydroxy phenyl lower alkyl, hydroxy lower alkyl, acylamino lower alkyl (such as benzoylamino lower alkyl, acetylaminoloweralkyl), aminoloweralkyl, dimethylaminoloweralkyl, haloloweralkyl, guanidinoloweralkyl, imidazolylloweralkyl, indolylloweralkyl, mercaptoloweralkyl, lower alkylthioloweralkyl;

$R^{4d}$ is hydrogen or lower alkyl;

$R^{5d}$ is hydrogen, lower alkyl, phenyl, phenylloweralkyl, hydroxyphenylloweralkyl, hydroxyloweralkyl, aminoloweralkyl, guanidinoloweralkyl, imidazolylloweralkyl, indolyloweralkyl, mercaptoloweralkyl or lower alkylthioloweralkyl;

$R^{4d}$ and $R^{5d}$ may be connected together to form an alkylene bridge of from 2 to 4 carbon atoms, an alkylene bridge of from 2 to 3 carbon atoms and one sulfur atom, an alkylene bridge of from 3 to 4 carbon atoms containing a double bond or an alkylene bridge as above substituted with hydroxy, lower alkoxy, lower alkyl or dilower alkyl;

and the pharmaceutically acceptable salts thereof.

(d) U.S. Patent No. 4,462,943 discloses carboxyalkyl amino acid derivatives of substituted prolines which are angiotensin converting enzyme inhibitors and have the formula

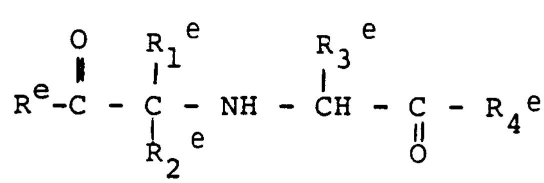

$R_4{}^e$ is a substituted proline of the formula

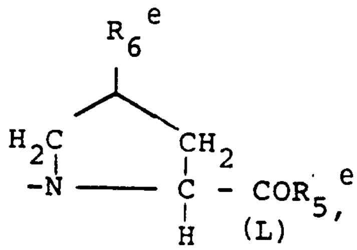

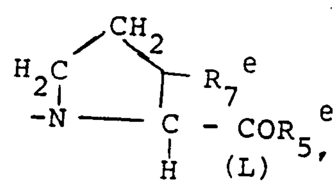

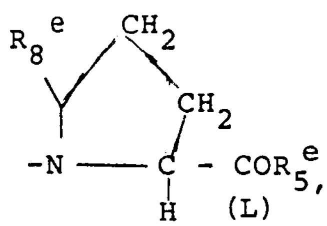

or

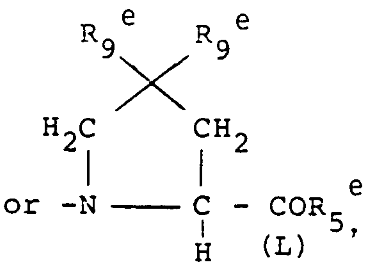

$R_6^e$ is halogen, keto, azido,

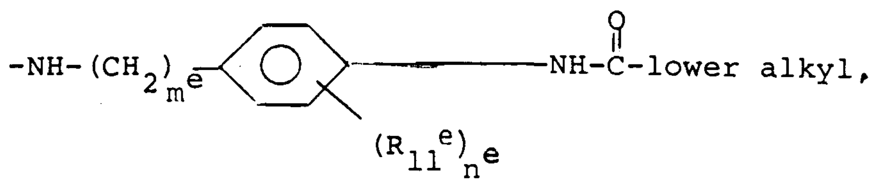

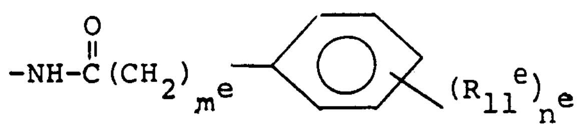

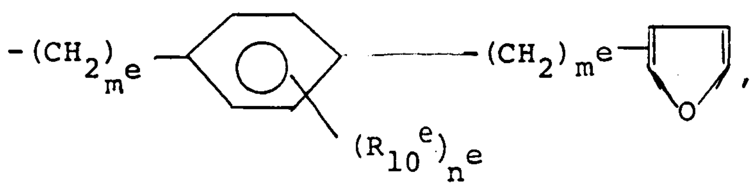

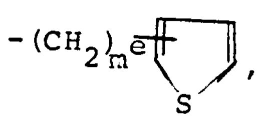

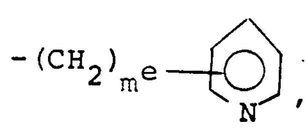

a 1- or 2-naphthyl of the formula

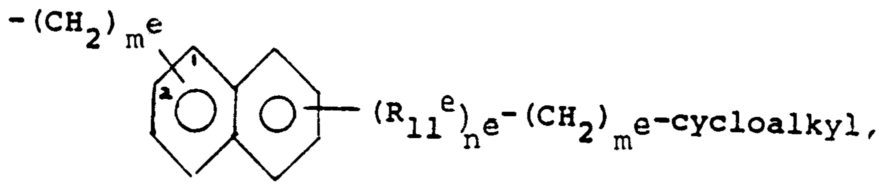

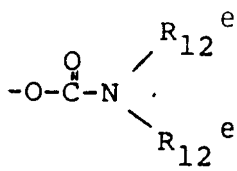,

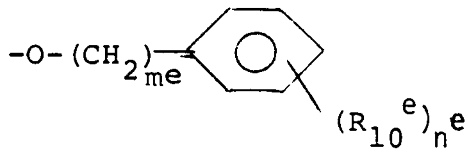

a 1- or 2-naphthyloxy of the formula

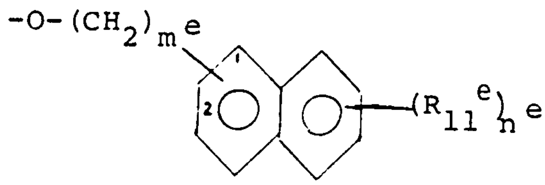

-S-lower alkyl,

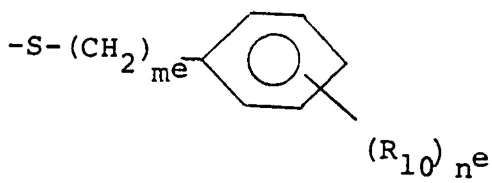

or a 1- or 2-naphthylthio of the formula

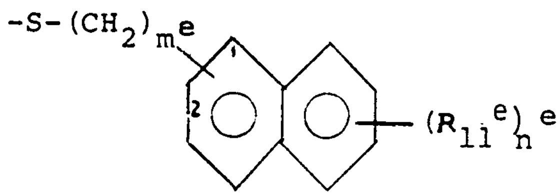

$R_7{}^e$ is keto, halogen,

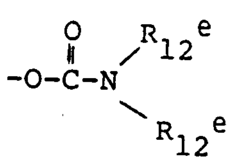,

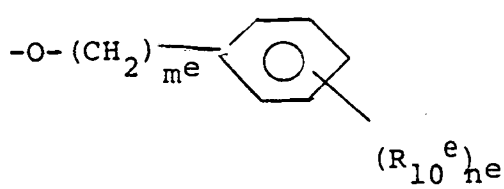

or 1- or 2-naphthloxy of the formula

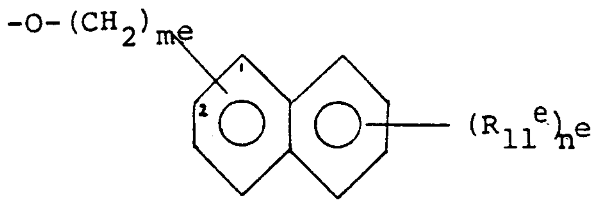

-S-lower alkyl,

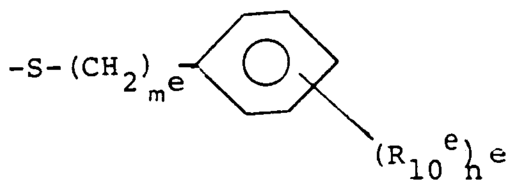

or a 1- or 2-naphthylthio of the formula

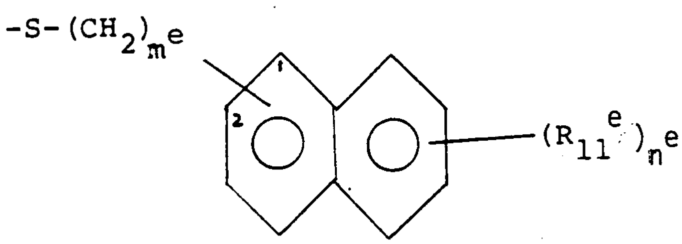

$R_8{}^e$ is keto or

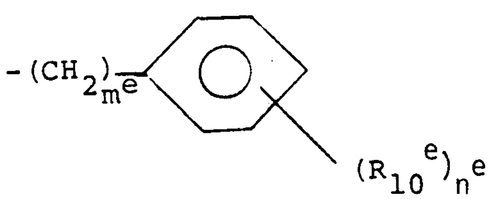

$R_9{}^e$ is halogen or $-Y^e-R_{13}{}^e$.

$m^e$ is zero, one, two, or three.

$R_{10}{}^e$ is hydrogen, lower alkyl of 1 to 4 carbon atoms, lower alkoxy of 1 to 4 carbon atoms, lower alkylthio of 1 to 4 carbon atoms, chloro, bromo, fluoro, trifluoromethyl, hydroxy, phenyl, phenoxy, phenylthio, or phenylmethyl,

$R_{11}{}^e$ is hydrogen, lower alkyl of 1 to 4 carbon atoms, lower alkoxy of 1 to 4 carbon atoms, lower alkylthio of 1 to 4 carbon atoms, bromo, fluoro, trifluoromethyl, or hydroxy.

$n^e$ is one, two or three provided that $n^e$ is more than one only if $R_{10}{}^e$ or $R_{11}{}^e$ is hydrogen, methyl, methoxy, chloro, or fluoro.

$R_{12}{}^e$ is hydrogen or lower alkyl of 1 to 4 carbon atoms,

$Y^e$ is oxygen or sulfur,

$R_{13}{}^e$ is lower alkyl of 1 to 4 carbon atoms.

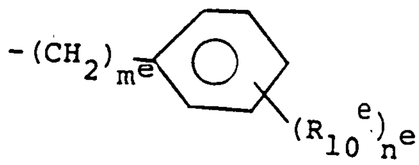

or the $R_{13}^e$ group join to complete an unsubstituted 5- or 6-membered ring or said ring in which one or more of the carbon atoms has a lower alkyl of 1 to 4 carbon atoms, or a di(lower alkyl of 1 to 4 carbon atoms) substituent,
$R^e$ and $R_5^e$ are independently selected from hydroxy, lower alkoxy, di(lower alkyl)-amino-lower alkoxy, such as dimethylaminoethoxy, lower alkyl-carbonyl-amino-lower alkoxy, such as acetylaminoethoxy, lower alkyl-carbonyloxy-lower alkoxy, such as pivaloyloxymethoxy,

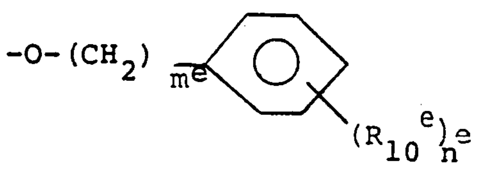

wherein $m^e$, $n^e$ and $R_{10}^e$ are as defined above, amino, lower alkyl-amino, di(lower alkyl)-amino, hydroxyamino, benzylamino, or phenethylamino, $R_1^e$ is hydrogen, lower alkyl,

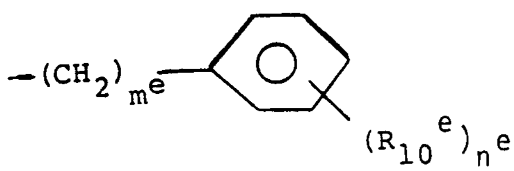

halo substituted lower alkyl, hydroxy substituted lower alkyl,
$-(CH_2)_q^e$-cycloalkyl, $-(CH_2)_q^e$ carboxy, $-(CH_2)_q^e$-S-lower alkyl,

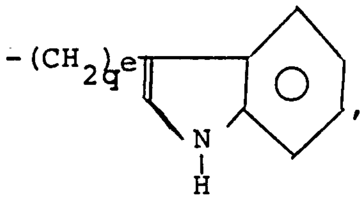,
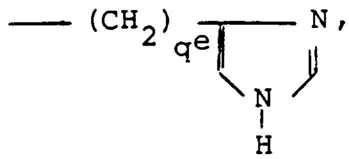,

$-(CH_2)_{q^e}$-guanidinyl, $-(CH_2)_q-NH_2$,

$-(CH_2)_{q^e}-N(\text{lower alkyl})_2$,

$-(CH_2)_{q^e}-NH-\overset{O}{\overset{\|}{C}}-\text{lower alkyl}$,

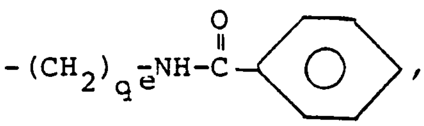,

$-(CH_2)_{q^e}-SH$,

$-(CH_2)_{q^e}-\text{lower alkoxy}$,

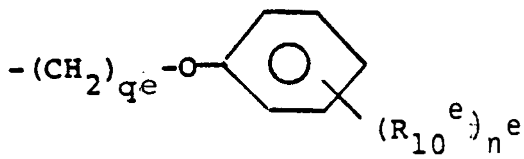

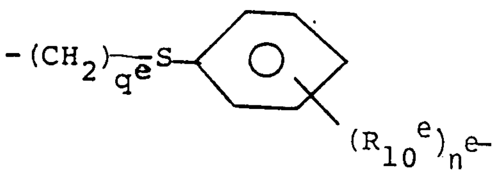

$-(CH_2)_{q^e}-\overset{O}{\overset{\|}{C}}-NH_2$

$-(CH_2)_{q^e}-\overset{O}{\overset{\|}{C}}-\text{lower alkoxy}$, or

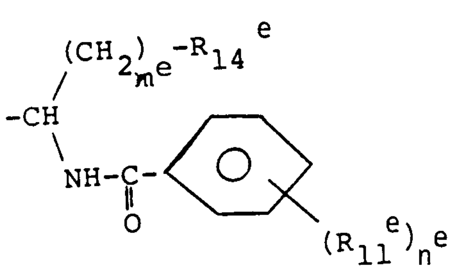

wherein $m^e$, $n^e$, $R_{10}{}^e$ and $R_{11}{}^e$ are as defined above, $R_{14}{}^e$ is lower alkyl, cycloalkyl, or

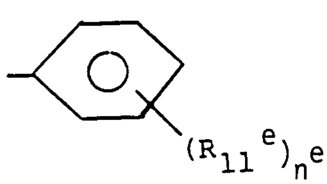

and $q^e$ is an integer from 1 to 4,
$R_2{}^e$ is hydrogen or lower alkyl,
$R_3{}^e$ is hydrogen, lower alkyl, halo substituted lower alkyl, hydroxy substituted lower alkyl, $-(CH_2)q^e-NH_2$, $-(CH_2)q^e-N=(\text{lower alkyl})_2$, $-(CH_2)q^e$-guanidinyl, $-(CH_2)q^e-SH$, $-(CH_2)q^e-S-$

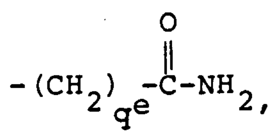

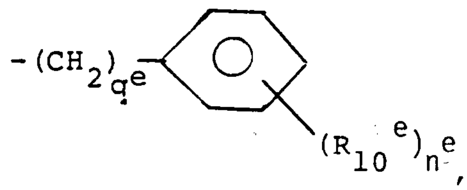

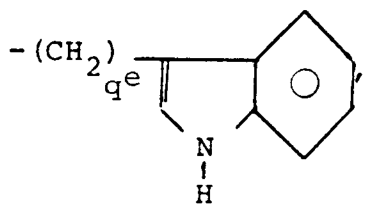

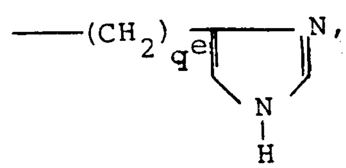

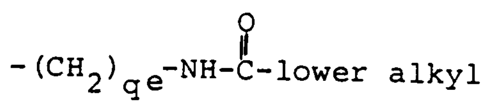

or

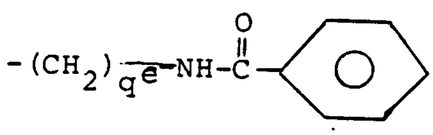

wherein $R_{10}{}^e$, $n^e$ and $q^e$ are as defined above.
(f) U.S. Patent No. 4,470,973
discloses substituted peptides which are angiotensin converting enzyme inhibitors and have the formula

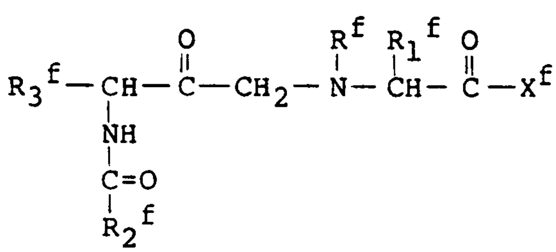

$X^f$ is an amino or imino acid of the formula

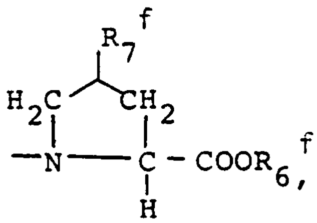

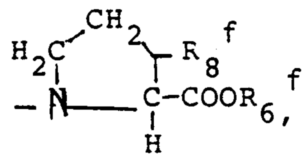

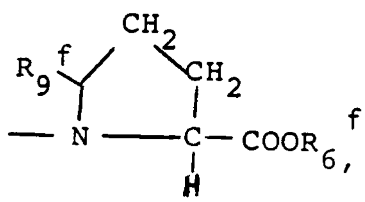

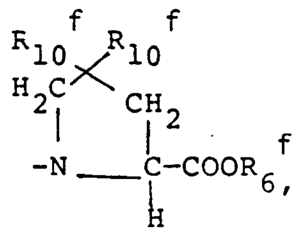

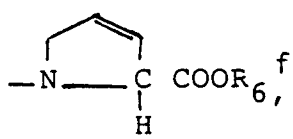

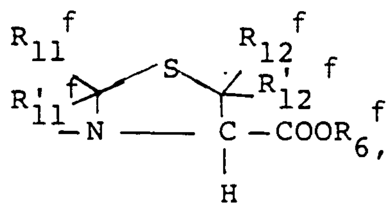

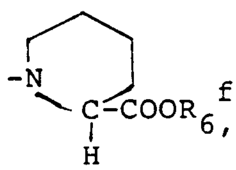

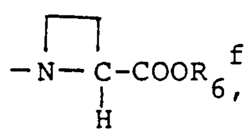

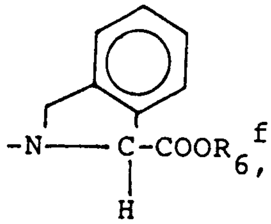

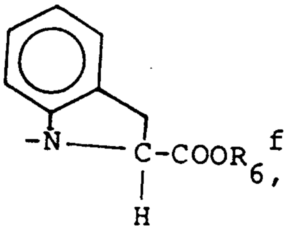

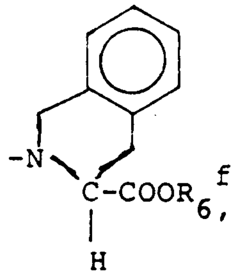

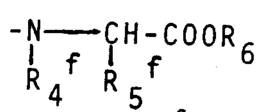, or

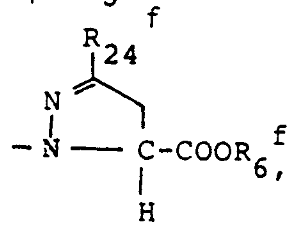

$R_7{}^f$ is hydrogen, lower alkyl, halogen, keto, hydroxy

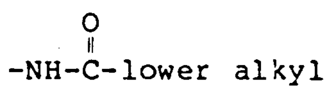

azido, amino,

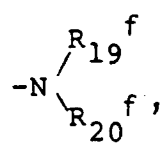

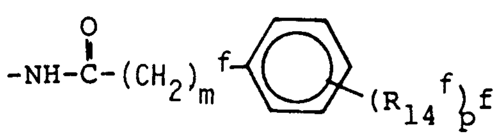

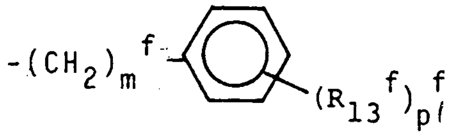 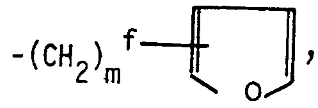

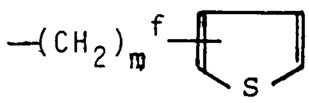 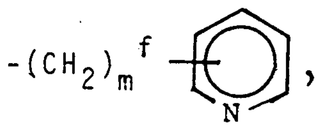

a 1- or 2-naphthyl of the formula

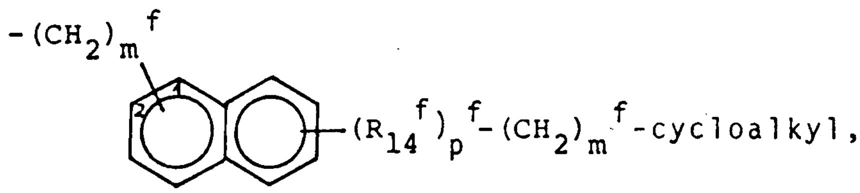

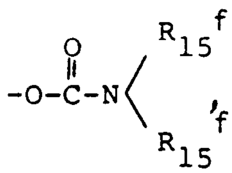 -O-lower alkyl,

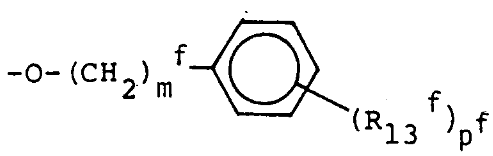

a 1- or 2-naphthyloxy of the formula

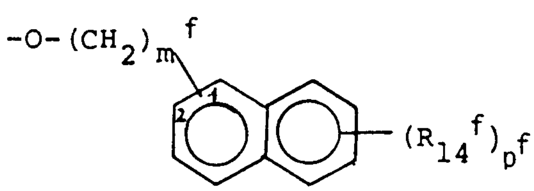

-S-lower alkyl,

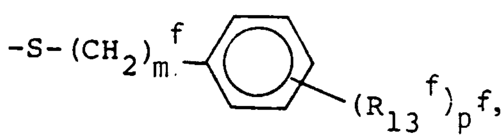

or a 1- or 2-naphthylthio of the formula

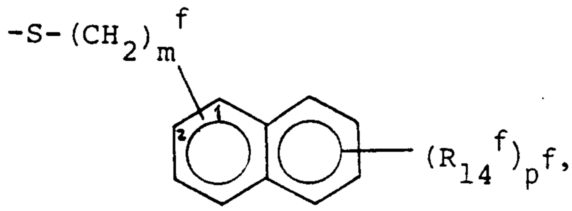

$R_8^f$ is keto, halogen.

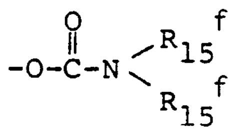

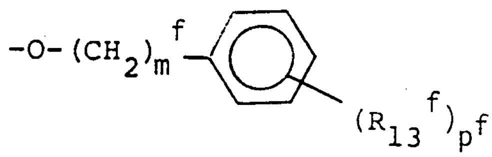

-O-lower alkyl, a 1- or 2-naphthyloxy of the formula

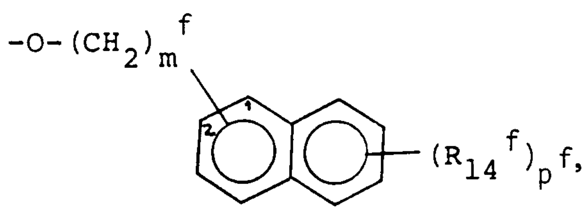

-S-lower alkyl

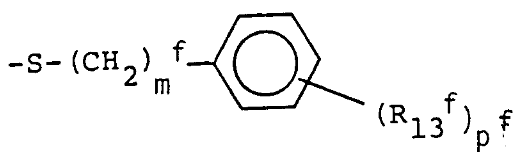

or a 1- or 2-naphthylthio of the formula

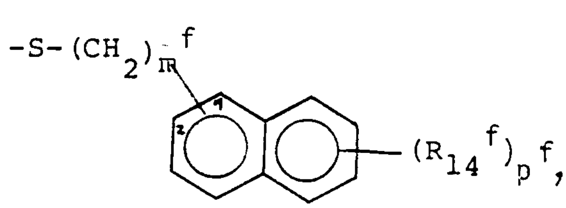

$R_9^f$ is keto or

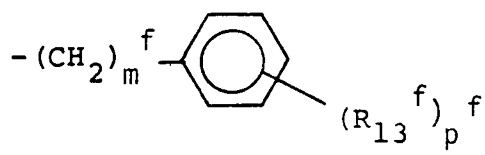

$R_{10}^f$ is halogen or $-Y^f-R_{16}^f$.

$R_{11}^f$, $R_{11'}^f$, $R_{12}^f$ and $R_{12'}^f$ are independently selected from hydrogen and lower alkyl or $R_{11'}^f$,$R_{12}^f$ and $R_{12'}^f$ are hydrogen and $R_{11}^f$ is

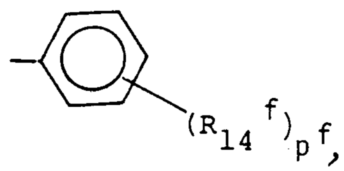

$R_{13}^f$ is hydrogen, lower alkyl of 1 to 4 carbon atoms, lower alkoxy of 1 to 4 carbon atoms, lower alkylthio of 1 to 4 carbon atoms, chloro, bromo, fluoro, trifluoromethyl, hydroxy, phenyl, phenoxy, phenylthio, or phenylmethyl. $R_{14}^f$ is hydrogen, lower alkyl of 1 to 4 carbon atoms, lower alkoxy of 1 to 4 carbon atoms, lower alkylthio of 1 to 4 carbon atoms, chloro, bromo, fluoro, trifluoromethyl, or hydroxy.

$m^f$ is zero, one, two, three, or four.

$p^f$ is one, two or three provided that p is more than one only if $R_{13}^f$ or $R_{14}^f$ is hydrogen, methyl, methoxy, chloro, or fluoro.

$R_{15}^f$ is hydrogen or lower alkyl of 1 to 4 carbon atoms.

$Y^f$ is oxygen or sulfur.

$R_{16}^f$ is lower alkyl of 1 to 4 carbon atoms.

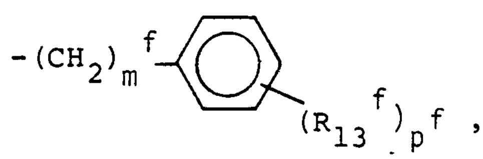

or the $R_{16}^f$ groups join to complete an unsubstituted 5- or 6-membered ring or said ring in which one or more of the carbon atoms has a lower alkyl of 1 to 4 carbon atoms or a di(lower alkyl of 1 to 4 carbon atoms) substituent.

$R_4^f$ is hydrogen, lower alkyl.

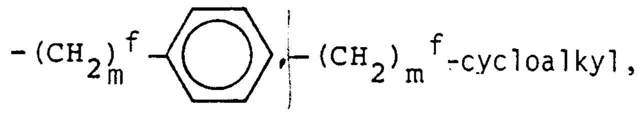

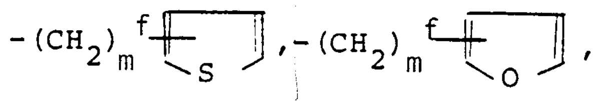

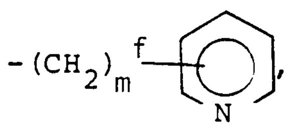 or 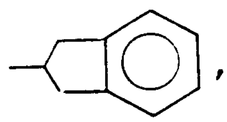

$R_5{}^f$ is hydrogen, lower alkyl,

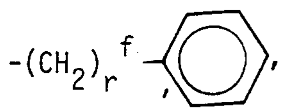 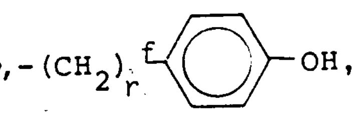

$-(CH_2)_r-OH$, 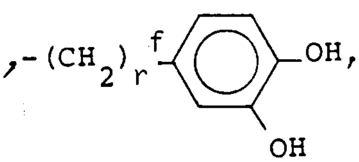

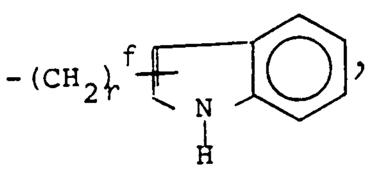 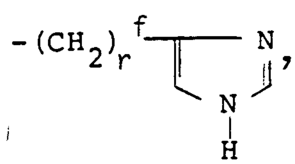

$-(CH_2)_r{}^f-NH_2-(CH_2)_r{}^f-SH,-(CH_2)_r{}^f$-S-lower alkyl,

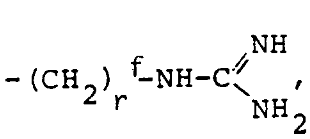 or 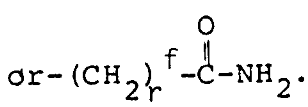

$r^f$ is an integer from 1 to 4.
$R_{19}{}^f$ is lower alkyl, benzyl, or phenethyl.
$R_{20}{}^f$ is hydrogen, lower alkyl, benzyl or phenethyl.
$R^f$ is hydrogen, lower alkyl, cycloalkyl,

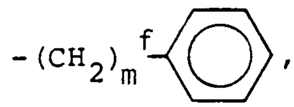

$-(CH_2)_2-NH_2$, $-(CH_2)_3-NH_2$, $-(CH_2)_4-NH_2$,
$-(CH_2)_2-OH$, $-(CH_2)_3-OH$, $-(CH_2)_4-OH$,
$-(CH_2)_2-SH$, $-(CH_2)_3-SH$, or $-(CH_2)_4-SH$.

$R_1^f$ is hydrogen, lower alkyl, halo substituted lower alkyl,

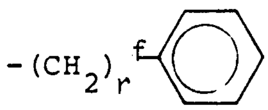, 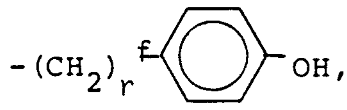,

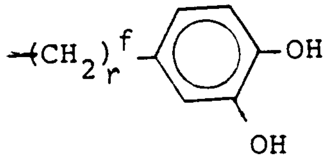, 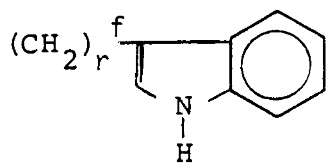

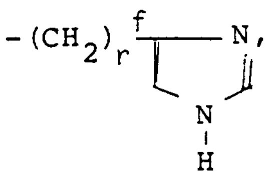, $-(CH_2)_r^f-NH_2$, $-(CH_2)_r^f-SH$,

$-(CH_2)_r^f-OH$, $-(CH_2)_r^f$-S-lower alkyl,

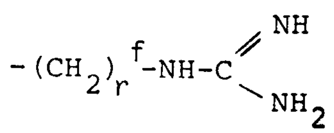 , or 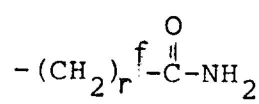

provided that $R_1^f$ is hydrogen only if $R^f$ is other than hydrogen.

$R_2^f$ is

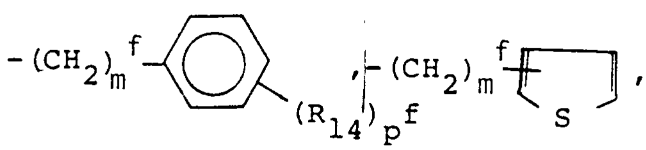,

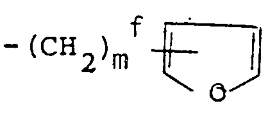, or 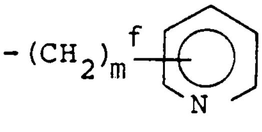

$R_3^f$ is hydrogen, lower alkyl

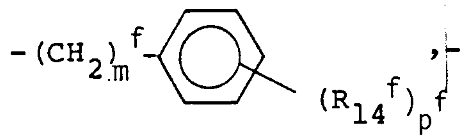, 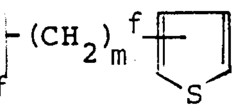,

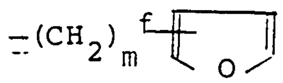, 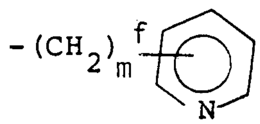, halo substituted

lower alkyl, $-(CH_2)_m{}^f$cycloalkyl, 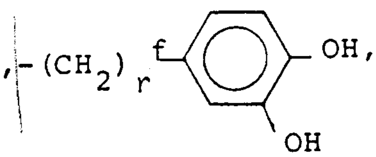

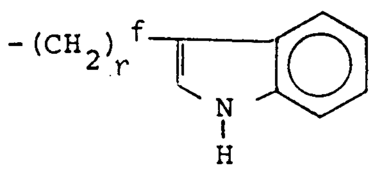, $-(CH_2)_r{}^f-OH$,

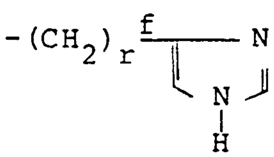, $-(CH_2)_r{}^f-NH_2$, $-(CH_2)_r{}^f-SH$,

$-(CH_2)_r{}^f$-S-lower alkyl, 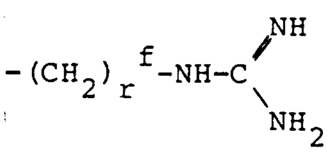, or 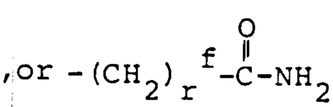

wherein $m^f$, $R_{14}{}^f$, $p^f$ and $r^f$ are as defined above.

$R_6{}^f$ is hydrogen, lower alkyl, benzyl, benzhydryl,

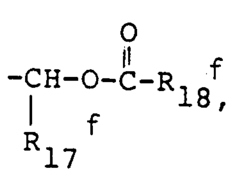, 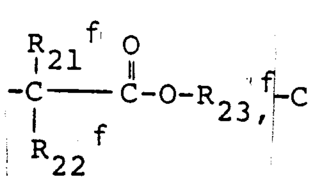, $-CH-(CH_2-OH)_2$,

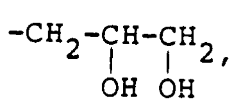, $-(CH_2)_2-N(CH_3)_2$, or 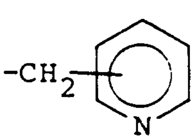

$R_{17}{}^f$ is hydrogen, lower alkyl, cycloalkyl, or phenyl.

$R_{18}{}^f$ is hydrogen, lower alkyl, lower alkoxy, or phenyl or $R_{17}{}^f$ and $R_{18}{}^f$ taken together are $-(CH_2)_2-$,

$-(CH_2)_3-$, $-CH=CH-$, or

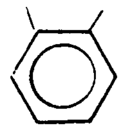

,

$R_{21}^{f}$ and $R_{22}^{f}$ are independently selected from hydrogen and lower alkyl.
$R_{23}^{f}$ is lower alkyl.
$R_{24}^{f}$ is hydrogen, lower alkyl,

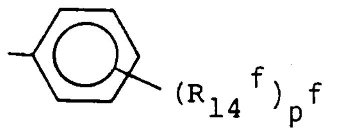

,

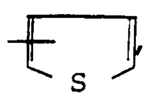

,

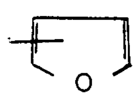

, or

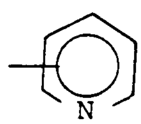

GB-A- 2095682 discloses N-substituted-N-carboxyalkyl aminocarbonyl alkyl glycine derivatives which are said to be angiotensin converting enzyme inhibitors and have the formula

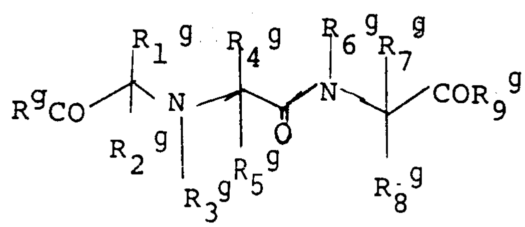

wherein either

(A) $R^g$ and $R_9^g$ are OH, 1-6C alkoxy, 2-6C alkenyloxy, di-(1-6C alkyl)amino-(1-6C) alkoxy, 1-6C hydroxyalkoxy, acylamino-(1-6C)alkoxy, acyloxy-(1-6C)alkoxy, aryloxy, aryloxy-(1-6C)alkoxy, $NH_2$, mono- or di-(1-6C alkyl)amino, hydroxyamino or aryl-(1-6C)alkylamino;

$R_1^g$-$R_5^g$, $R_7^g$ and $R_8^g$ are 1-20C alkyl, 2-20C alkenyl, 2-20C alkynyl, aryl, aryl-(1-6C) alkyl having 7-12C or heterocyclyl-(1-6C)alkyl having 7-12 atoms;

$R_6^g$ is cycloalkyl, polycycloalkyl, partly saturated cycloalkyl or polycycloalkyl, cycloalkyl-(1-6C)alkyl having 3-20C, 6-10C aryl, aryl-(1-6C)alkyl, aryl-(2-6C)alkenyl or aryl-(2-6C) alkynyl; or

$R_2^g$ and $R_3^g$ together with the C and N atoms to which they are attached or $R_3^g$ and $R_5^g$ together with the N and C atoms to which they are attached form an N-heterocycle containing 3-5C or 2-4C and an S atom;

all alkyl, alkenyl and alkynyl are optionally substituted by OH, 1-6C alkoxy, S4, 1-6C alkylthio, $NH_2$, mono- or di(1-6C alkyl)amino, halogen or $NO_2$;

all cycloalkyl groups (including poly and partially unsaturated) are optionally substituted by halogen, 1-6C hydroxyalkyl, 1-6C alkoxy, amino-(1-6C alkyl)amino, di-(1-6C alkyl)amino, SH, 1-6C alkylthio, $NO_2$ or $CF_3$; and aryl groups are optionally substituted by OH, 1-6C alkoxy, $NH_2$, mono- or di-(1-6C alkyl) amino, SH, 1-6C alkylthio, 1-6C hydroxyalkyl, 1-6C aminoalkyl, 1-6C thioalkyl, $NO_2$, halogen, $CF_3$, $OCH_2O$, ureido or guanidino; or

(B) $R^g$ and $R_9^g$ are H or 1-6C alkoxy;

$R_1{}^g$ and $R_2{}^g$ are H, 1-6C alkyl, aryl-(1-6C) alkyl having 7-12C atoms or heterocyclyl-(1-6C) alkyl having 6-12C atoms;
$R_3{}^g$-$R_5{}^g$, $R_7{}^g$ and $R_8{}^g$ are H or 1-6C alkyl;
$R_6{}^g$ is cycloalkyl, polycycloalkyl, partly saturated cycloalkyl or polycycloalkyl, cycloalkyl-(1-6C) alkyl having 3-20C atoms, aryl or aryl-(1-6C) alkyl; and
aryl has 6-10C and is optionally substituted by 1-6C alkyl, 2-6C alkenyl, 2-6C alkynyl, OH, 1-6C alkoxy, $NH_2$, mono- or di-(1-6C alkyl) amino, SH, 1-6C alkylthio, 1-6C hydroxyalkyl, 1-6C aminoalkyl, 1-6C thioalkyl, $NO_2$, halogen, $CF_3$, $OCH_2O$, ureido or guanidino.

U.S. Patent No. 4,470,972 discloses 7-carboxyalkyl-aminoacyl-1,4-dithia-7-azaspiro[4.4]nonane-8-carboxylic acids which are angiotensin converting enzyme inhibitors and have the formula

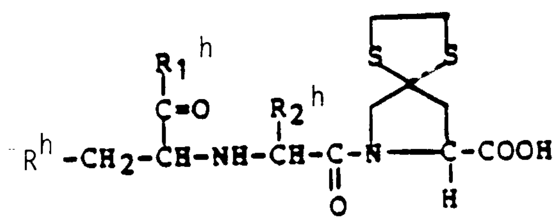

wherein:
$R^h$ is lower alkyl, benzyl, benzylthio, benzyloxy, phenylthio or phenoxy;
$R_1{}^h$ is hydroxy or lower alkoxy;
$R_2{}^h$ is hydrogen, lower alkyl or amino lower alkyl; and the pharmaceutcally acceptable salts thereof.

(i) EP-A-0 050 800 discloses carboxyalkyl dipeptides derivatives which are said to be angiotensin converting enzyme inhibitors and have the formula

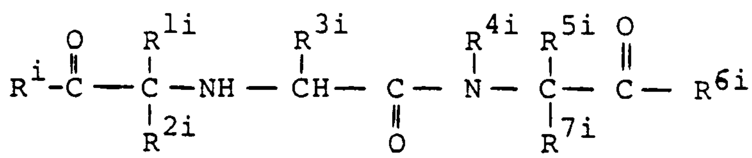

or a pharmaceutically acceptable salt thereof, wherein $R^i$ and $R^{6i}$ are the same or different and are hydroxy, lower alkoxy, lower alkenyloxy, dilower alkylamino lower alkoxy, acylamino lower alkoxy, acyloxy lower alkoxy, aryloxy, aryllower alkoxy, amino, lower alkylamino, dilower alkylamino, hydroxyamino, aryllower alkylamino, or substituted aryloxy or substituted aryllower alkoxy wherein the substitutent is methyl, halo or methoxy; $R^{1i}$ is hydrogen, alkyl of from 1 to 10 carbon atoms, substituted lower alkyl wherein the substitutent is hydroxy, lower alkoxy, aryloxy, substituted aryloxy, heteroaryloxy, substituted heteroaryloxy, amino, lower alkylamino, diloweralkylamino, acylamino, arylamino, substituted arylamino, quanidino, imidazolyl, indolyl, lower alkylthio, arylthio, substituted arylthio, carboxy, carbamoyl, lower alkoxy carbonyl, aryl, substituted aryl, aralkyloxy, substituted aralkyloxy, aralkylthio or substituted aralkylthio, wherein the aryl or heteroaryl portion of said substituted aryloxy, heteroaryloxy, arylamino, arylthio, aryl, aralkyloxy, aralkylthio group is substituted with a group selected from halo, lower alkyl, hydroxy, lower alkoxy, amino, aminomethyl, carboxyl, cyano, or sulfamoyl; $R^{2i}$ and $R^{7i}$ are the same or different and are hydrogen or lower alkyl; $R^{3i}$ is hydrogen, lower alkyl, phenyl lower alkyl, aminoethylphenyl lower alkyl, hydroxyphenyl lower alkyl, hydroxy lower alkyl, acylamino lower alkyl, amino lower alkyl, dimethylamino lower alkyl, guanidino lower alkyl, imidazolyl lower alkyl, indolyl lower alkyl, or lower alkyl thio lower alkyl; $R^{4i}$ and $R^{5i}$ are the same or different and are hydrogen, lower alkyl or $Z^i$, Or $R^{4i}$ and $R^{5i}$ taken together form a group represented by $Q^i$, $U^i$, $V^i$, $Y^i$, $D^i$ or $E^i$, wherein
$Z^i$ is

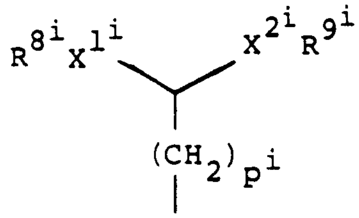

wherein $X^{1i}$ and $X^{2i}$ independent of each other are O, S or $CH_2$, $R^{8i}$ and $R^{9i}$ independent of each other are lower alkyl, lower alkenyl, lower alkynyl, cycloalkyl having 3 to 8 carbon atoms, hydroxy lower alkyl, or $-(CH_2)_{n^i}Ar^i$, wherein $n^i$ is 0, 1, 2 or 3 and $Ar^i$ is unsubstituted or substituted phenyl, furyl, thienyl or pyridyl, wherein said substituted phenyl, furyl, thienyl or pyridyl groups are substituted with at least one group that is independently selected from $C_1$ to $C_4$ alkyl, lower alkoxy, lower alkylthio, halo, $CF_3$ and hydroxy, or $R^{8i}$ and $R^{9i}$ taken together form a bridge $W^i$, wherein $W^i$ is a single bond or a methylene bridge or a substituted methylene bridge when at least one of $X^{1i}$ and $X^{2i}$ is methylene, or $W^i$ is an alkylene or substituted alkylene bridge having 2 or 3 carbon atoms, said substituted methylene bridge or said substituted alkylene bridge having one or two substituents selected from lower alkyl, aryl and aryl lower alkyl groups, and $p^i$ is 0, 1 or 2; with the proviso that at least one of $R^{4i}$ and $R^{5i}$ is $Z^i$, with the proviso that if $R^{4i}$ is $Z^i$ and $p^i$ is 0 then $X^{1i}$ and $X^{2i}$ must both be methylene, and with the proviso that if $X^{1i}$ and $X^{2i}$ are both methylene then $R^{8i}$ and $R^{9i}$ must form an alkylene bridge $W^i$;

$Q^i$ is

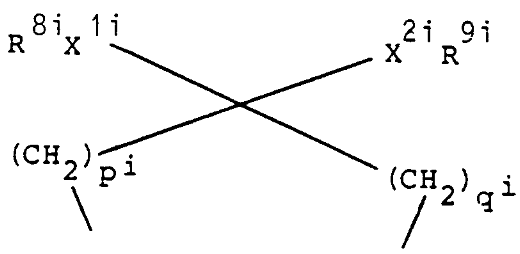

wherein $R^{8i}$, $R^{9i}$, $X^{1i}$ and $X^{2i}$ are as defined above, $p^i$ is 0, 1 or 2, $q^i$ is 0, 1 or 2, with the proviso that the sum of $p^i$ and $q^i$ must be 1, 2 or 3, with the proviso that if $p^i$ is 0 then $X^{1i}$ and $X^{2i}$ must be methylene, and with the proviso that if $X^{1i}$ and $X^{2i}$ are methylene then $R^{8i}$ and $R^{9i}$ taken together form a bridge $W^i$, wherein $W^i$ is as defined above;

$v^i$ is

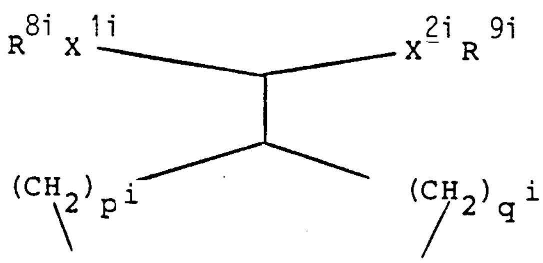

wherein $R^{8i}$, $R^{9i}$, $X^{1i}$ and $X^{2i}$ are as defined above, $p^i$ is 0, 1 or 2 and $q^i$ is 0, 1 or 2, with the proviso that the sum of $p^i$ and $q^i$ is 1, 2 or 3, with the proviso that if $X^{1i}$ and $X^{2i}$ are $CH_2$ then $R^{8i}$ and $R^{9i}$ taken together form a bridge $w^i$, wherein $w^i$ is as defined above;

$U^i$ is

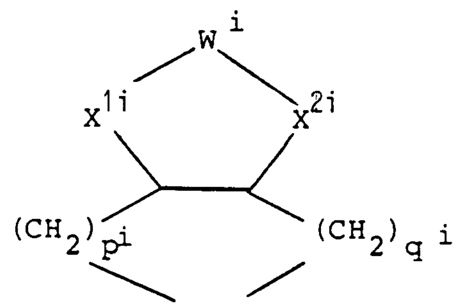

wherein $W^i$ is as defined above (except that $W^i$ may also be a methylene bridge when $X^{1i}$ and $X^{2i}$ are oxygen or sulfur), $X^{1i}$ and $X^{2i}$ are as defined above, $p^i$ is 0, 1 or 2, $q^i$ is 0, 1 or 2, with the proviso that the sum of $p^i$ and $q^i$ is 1 or 2, and with the proviso that if $p^i$ is 0, $X^{1i}$ must be $CH_2$;
$Y^i$ is

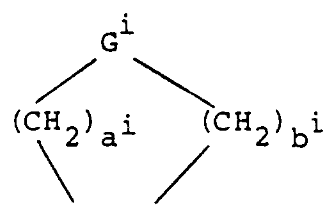

wherein $G^i$ is oxygen, sulfur or $CH_2$, $a^i$ is 2, 3 or 4 and $b^i$ is 1, 2, 3, 4 or 5, with the proviso that the sum of $a^i$ and $b^i$ is 5, 6 or 7 or
$G^i$ is $CH_2$, $a^i$ is 0, 1, 2 or 3, $b^i$ is 0, 1, 2 or 3 with the proviso that the sum of $a^i$ and $b^i$ is 1, 2 or 3, with the proviso that the sum of $a^i$ and $b^i$ may be 1, 2 or 3 only if $R^{1i}$ is lower alkyl substituted with aralkylthio or aralkyloxy;
$D^i$ is

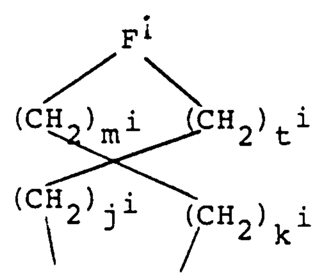

wherein $F^i$ is O or S, $j^i$ is 0, 1 or 2 and $k^i$ is 0, 1 or 2, with the proviso that the sum of $j^i$ and $k^i$ must be 1, 2 or 3, and $m^i$ is 1, 2 or 3 and $t^i$ is 1, 2 or 3, with the proviso that the sum of $m^i$ and $t^i$ must be 2, 3 or 4;
$E^i$ is

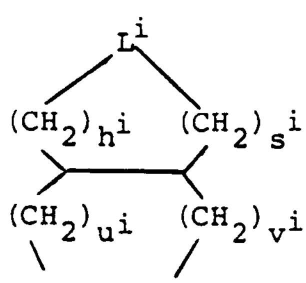

wherein $L^i$ is O or S, $u^1$ is 0, 1 or 2 and $v^i$ is 0, 1 or 2, with the proviso that the sum of $u^i$ and $v^i$ must be 1 or 2, and $h^i$ is 1 or 2 and $s^i$ is 1 or 2, with the proviso that the sum of $h^i$ and $s^i$ must be 2 or 3.

(j) EP-A-0 037 231 discloses acyl derivatives of octahydro-1H-indole-2-carboxylic acids which are said to be angiotensin converting enzyme inhibitors and have the formula

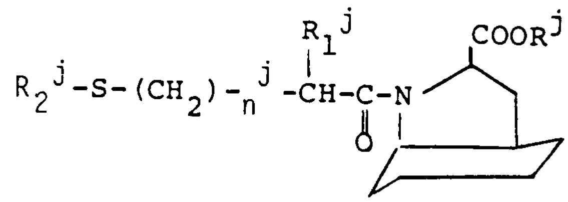

wherein $R^j$ is hydrogen or lower alkyl; $R_1{}^j$ is hydrogen, lower alkyl, or benzyl; $R_2{}^j$ is hydrogen or $R_3{}^j$-CO- wherein $R_3{}^j$ is lower alkyl, heteroaryl containing 4 to 9 carbon atoms and one or two nitrogen, oxygen or sulfur atoms; phenyl, substituted phenyl having 1 or 2 substituents selected from fluorine, chlorine, bromine, lower alkyl or alkoxy; and n is 0 or 1; wherein lower alkyl and lower alkoxy include straight or branched groups containing 1 to 4 carbon atoms, and pharmaceutically acceptable salts of the compounds when $R^j$ is hydrogen and when $R_3{}^j$ is heteroaryl containing 1 or 2 nitrogen atoms, are disclosed. Also disclosed are substituted acyl compounds of octahydro-1H-indole-2-carboxylic acid having the formula

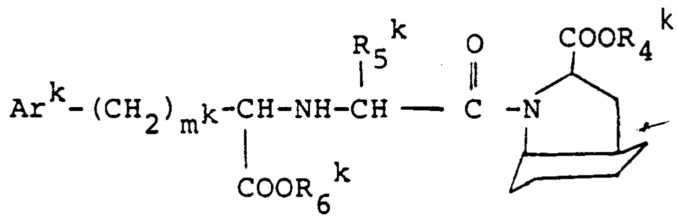

wherein $R_4{}^k$ is hydrogen or lower alkyl; $R_5{}^k$ is hydrogen, lower alkyl or benzyl; $R_6{}^k$ is hydrogen or lower alkyl; $Ar^k$ is phenyl or phenyl substituted with 1 or 2 substituents selected from fluorine, chlorine, bromine, lower alkyl, lower alkoxy, hydroxy or amino; and m is 0 to 3; wherein lower alkyl and lower alkoxy contain 1 to 4 straight or branched carbon atoms; and the pharmaceutically acceptable salts thereof.

(l) EP-A- 0 079 522 discloses N-carboxymethyl(amino) lysl-proline compounds which are said to be angiotensin converting enzyme inhibitors and have the formulae

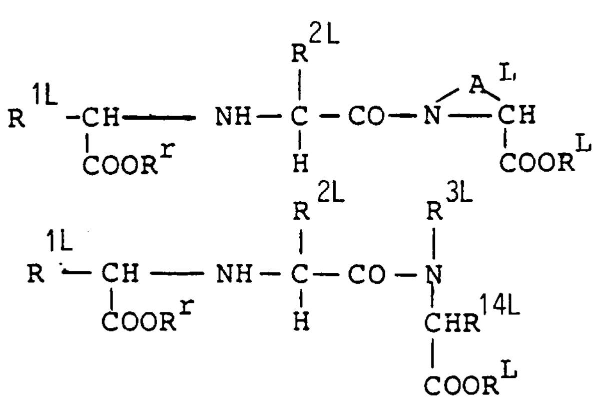

$(I)^L$

$(Ia)^L$

wherein

$R^L$ and $R^{2L}$ are independently hydrogen; loweralkyl; aralkyl; or aryl;

$R^{1L}$ is hydrogen; branched or straight chain $C_{1-12}$ alkyl and alkenyl; $C_3$-$C_9$ cycloalkyl and benzofused

alkyl; substituted loweralkyl where the substituents are halo, hydroxy loweralkoxy, aryloxy, amino, mono- or diloweralkylamino, acylamino, arylamino, guanidino, mercapto, loweralkylthio, arylthio, carboxy, carboxamido, or loweralkoxycarbonyl; aryl; substituted aryl where the substituents are loweralkyl, loweralkoxy, or halo; arloweralkyl; arloweralkenyl; heteroarloweralkyl; heteroarloweralkenyl; substituted arloweralkyl, substituted arloweralkenyl, substituted heteroarloweralkyl, or substituted heteroarloweralkenyl where the aryl and heteroaryl substituents are halo, dihalo, loweralkyl, hydroxy, loweralkoxy, amino, aminoloweralkyl, acylamino, mono- or diloweralkylamino, carboxyl, haloloweralkyl, nitro, cyano, or sulfonamido, and where the loweralkyl portion of a loweralkyl may be substituted by amino, acylamino, or hydroxyl;

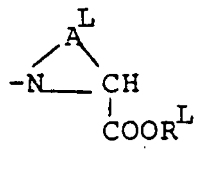 is 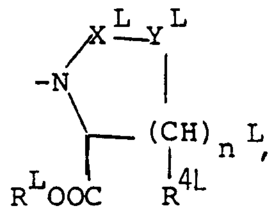 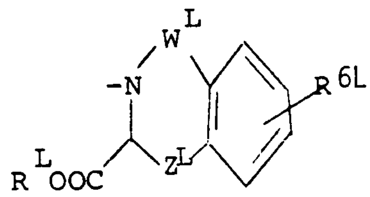

or 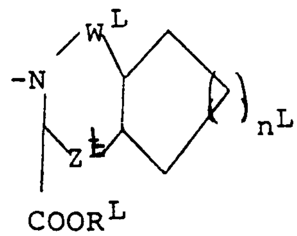

where:
$X^L$ and $Y^L$ taken together are $-CH_2-CH_2-$;

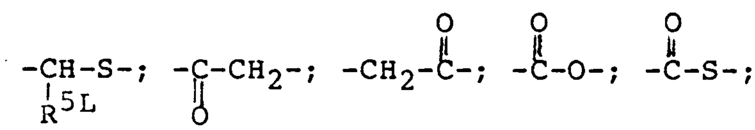

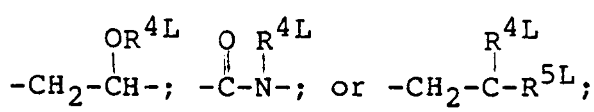

$R^{4L}$ is hydrogen; loweralkyl; aryl; substituted aryl;
$R^{5L}$ is hydrogen; loweralkyl; aryl or substituted aryl;
$n^L$ is 1 to 3;
$W^L$ is absent; $-CH_2-$; or
$Z^L$ is $-(CH_2)m^L$, where m is 0 to 2, provided that $m^L$ may not be 0 and $W^L$ may not be absent at the same time; and
$R^{6L}$ is hydrogen; loweralkyl; halo; or $OR^{4L}$;
$R^{2L}$ is $-(CH_2)_r{}^L-B^L-(CH_2)_sL-NR^{7L}R^{15L}$
where
$r^L$ and $s^L$ are independently 0 to 3;
$B^L$ is absent; -O-; -S-; or $NR^{8L}$;
where $R^{8L}$ is hydrogen; loweralkyl; alkanoyl; or aroyl; and
$R^{7L}$ is

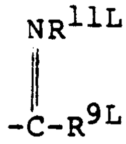 ; 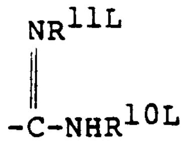 ; or 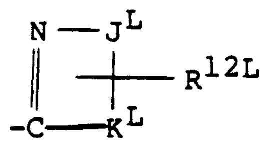

where

$R^{9L}$ is loweralkyl; aralkyl; aryl; heteroaryl; or heteroarloweralkyl and these groups are substituted by hydroxy, lower alkoxy or halo; carboxyl; carboxamido; nitromethenyl.

$R^{10L}$ is hydrogen; loweralkyl; aryl; or amidino;

$R^{11L}$ hydrogen; loweralkyl; cyano; amidino; aryl; aroyl; loweralkanoyl;

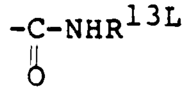

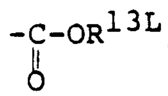

$-NO_2$; $-SO_2NH_2$;

or

$SO_2R^{13L}$;

$R^{12L}$ is hydrogen; loweralkyl; halo; aralkyl; amino; cyano; mono- or diloweralkylamino; or $OR^{4L}$;

$R^{13L}$ is hydrogen; loweralkyl; or aryl;

$R^{15L}$ is hydrogen; lower alkyl; aralkyl; or aryl.

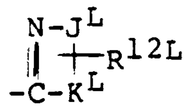

constitutes a basic heterocycle of 5 or 6 atoms or benzofused analogs thereof and optionally containing 1-3 N atoms, an oxygen-, a sulfur-atom, an SO- or an $SO_2$-group optionally substituted by amino, lower alkyl amino, diloweralkyl amino, lower alkoxy, or aralkyl groups;

$R^{3L}$ is $C_{3-8}$ cycloalkyl and benzofused $C_{3-8}$ cycloalkyl; perhydrobenzofused $C_{3-8}$ cycloalkyl; aryl; substituted aryl; heterary; substituted heteroaryl;

$R^{14L}$ is hydrogen or loweralkyl; and, a pharmaceutically acceptable salt thereof.

(m) U.S. Patent No. 4,256,761 discloses amides which are angiotensin converting enzyme inhibitors and have the general formula

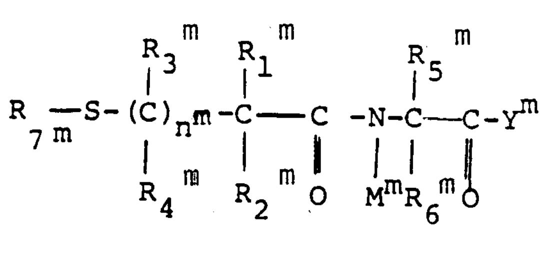

wherein

$R_1{}^m$, $R_2{}^m$, $R_3{}^m$, $R_4{}^m$, $R_5{}^m$ and $R_6{}^m$ are hydrogen, alkyl, alkenyl, alkynyl, phenyl-alkyl, and cycloalkyl, and may be the same or different,

$n^m$ is an integer from 0 to 4 inclusive,

$M^m$ is alkenyl, alkynyl, cycloalkyl, cycloalkyl-alkyl, polycycloalkyl, polycycloalkyl-alkyl, aryl, aralkyl, heteroaryl, heteroaryl-alkyl, hetero-cycloalkyl, hetero-cycloalkyl-alkyl, fused aryl-cycloalkyl, fused aryl-cycloalkyl-alkyl, fused heteroaryl-cycloalkyl, fused heteroaryl-cycloalkyl-alkyl, alkoxyalkyl, alkylthioalkyl, alkylamino-alkyl, or dialkylaminoalkyl,
$Y^m$ is hydroxy, alkoxy, amino, or substituted amino, aminoalkanoyl, aryloxy, aminoalkoxy, or hydroxyalkoxy, and
$R_7{}^m$ is hydrogen, alkanoyl, carboxyalkanoyl, hydroxyalkanoyl, aminoalkanoyl, cyano, amidino, carbalkoxy, $Z^mS$ or

$Z^mSCO$-

wherein $Z^m$ is hydrogen, alkyl, hydroxyalkyl, or the radical

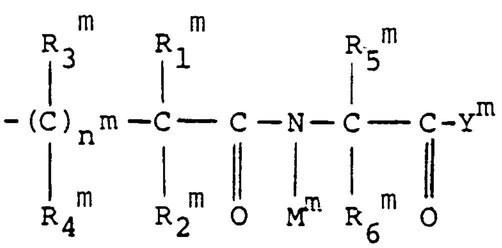

wherein $R_1{}^m$, $R_2{}^m$, $R_3{}^m$, $R_4{}^m$, $R_5{}^m$, $R_6{}^m$ $n^m$, $M^m$ and $Y^m$ are as described above; and where Y is hydroxy, their non-toxic, pharmaceutically acceptable alkali metal, alkaline earth metal, and amine salts.
(n) U.S. Patent No. 4,344,949 discloses acyl derivatives of 1,2,3,4-tetrahydro-isoquinoline-3-carboxylic acid compounds which are angiotensin converting enzyme inhibitors and have the formula

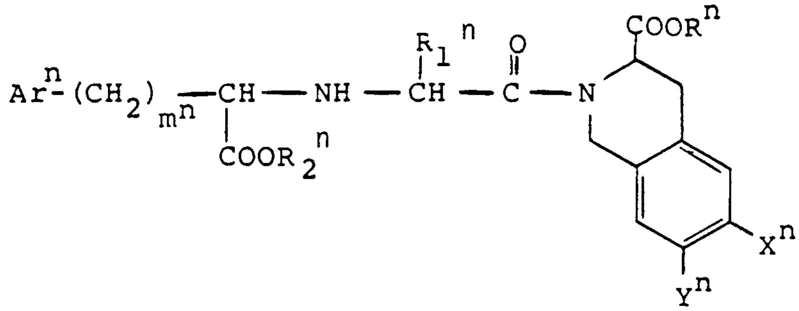

wherein $R^n$ is hydrogen, lower alkyl or aralkyl;
$R_1{}^n$ is hydrogen, lower alkyl, or benzyl; $R_2{}^n$ is hydrogen or lower alkyl, and $Ar^n$ is phenyl or phenyl substituted with 1 or 2 substituents selected from fluorine, chlorine, bromine, lower alkyl, lower alkoxy, hydroxy or amino; $X^n$ and $Y^n$ are independently hydrogen, lower alkyl, lower alkoxy, lower alkylthio, lower alkylsulfinyl, lower alkylsulfonyl, hydroxy, or $X^n$ and $Y^n$ together are methylenedioxy; $m^n$ is 0 to 3; and the pharmaceutically acceptable acid salts thereof.
(o) EP-C-0079022 discloses N-amino acyl-azabicyclooctane carboxylic acid derivatives which have the formula

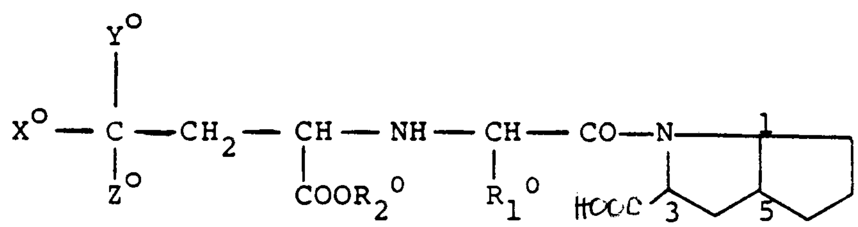

the hydrogen atoms at ring positions 1 and 5 are cis to each other and the 3-carboxy group has the endo orientation;

$R_1{}^o$ is H, allyl, vinyl or the side chain of an optionally protected naturally occurring $\alpha$-amino acid;

$R_2{}^o$ is H, 1-6C alkyl, 2-6C alkenyl or aryl(1-4C alkyl);

$Y^o$ is H or OH and $Z^o$ is H, or $Y^o$ and $Z^o$ together oxygen;

$X^o$ is 1-6C alkyl, 2-6C alkenyl, 5-9C cycloalkyl, 6-12C aryl (optionally substituted by one to three 1-4C alkyl or alkoxy, OH, halo, nitro, amino (optionally substituted by one or two 1-4C alkyl), or methylenedioxy or indol-3-yl.

(p) EP-C-0046953 discloses N-amino acyl-indoline and tetrahydro isoquinoline carboxylic acids which are angiotensin coverting enzyme inhibitors and have the formula

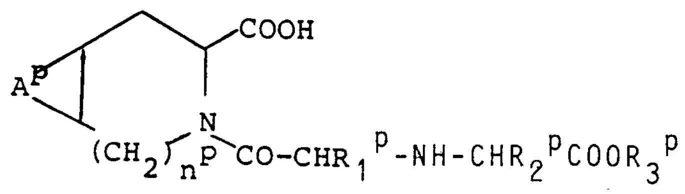

$n^p$ is 0 or 1;

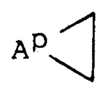

is a benzene or cyclohexane ring:

$R_1{}^p$ and $R_2{}^p$ are each 1-6C alkyl, 2-6C alkenyl, 5-7C cycloalkyl, 5-7C cycloalkenyl, 7-12C cycloalkylalkyl, optionally partially hydrogenated 6-10C aryl, 7-14C aralkyl or 5-7 membered monocyclic or 8-10 membered bicyclic heterocyclyl containing 1 or 2 S or O and/or 1-4N atoms; all $R_1{}^p$ and $R_2{}^p$ groups are optionally substituted,

$R_3{}^p$ is H, 1-6C alkyl, 2-6C alkenyl or 7-14C aralkyl.

Preferred $R_1{}^p$ is Me and $R_2{}^p$ is phenethyl optionally substituted by halogen, Me or OMe.

For purposes of the invention, a preferred ACE inhibitor is 7-[N-(1(S)-carboxy-3-phenylpropyl)-(S)-alanyl]-1,4-dithia-7-azaspiro[4.4]nonane-8(S)-carboxylic acid.

Examples of beta adrenergic blockers are bunolol, atenolol, metoprolol, nadolol, pindolol, propranolol, timolol, labetalol, betaxolol, carteolol and dilevolol and their isomers. For purposes of the invention a preferred Beta adrenergic blocker is timolol.

The preferred pharmaceutical composition of the invention comprises:

a) 7-[N-(1(S)-carboxy-3-phenylpropyl)-(S)-alanyl]-1,4-dithia-7-azaspiro[4.4]nonane-8(S)-carboxylic acid in an amount of from 0.005 $\mu$g to 50 $\mu$g; and

b) timolol in an amount of from 5$\mu$g to 500$\mu$g; in combination with an ophthalmologically acceptable carrier for topical use.

Angiotensin coverting enzyme (ACE) inhibitors are known in the art. Angiotensin II, a pressor substance, is produced in vivo by the action of angiotensin converting enzyme (ACE) on angiotensin I. Compounds capable of inhibiting the action of ACE are clinically useful for controlling the blood pressure of humans suffering from hypertension. ACE inhibitors are known in the art, and may have a variety of structures. See, for example, An. Rev. Biochem., 51, 283 (1982) and references cited herein.

It can be concluded from test results that any ACE inhibitor will possess the utility described herein; however, for purposes of the present invention the preferred ACE inhibitors are compounds which are capable of inhibiting the action of ACE by at least 50% at a concentration of 1 $\mu$M or less when tested by the following standard method:

ACE activity is determined by spectrophotometric assay of the product of the hydrolysis of the synthetic substrate, hippuryl histidyl leucine (HHL), as described by Cushman and Cheung, Biochem. Pharmacol., 20, 1637 (1971). The ACE used is prepared in a manner similar to that of Cheung and Cushman, Biochem. Biophys. Acta., 293, 451 (1973). Incubation for ACE assays is carried out at 37° C. Each 0.25 ml assay mixture contains the following components: 100 mM of potassium phosphate buffer containing 300 mM sodium chloride, 5 mM HHL and 1.87 mU enzyme at pH 8.3 and various concentrations

of inhibitors. The enzyme reaction is terminated after 60 minutes by the addition of 0.25 ml of 1N HCl. Inhibitors are dissolved in appropriate solvents. Hippuric acid solution for a standard curve is prepared in a similar manner.

Each experiment involves replicate incubations for each condition to be studied. $IC_{50}$ values (the concentration required for the 50% inhibition of ACE activity) are derived from calculated regression lines. Each experiment utilizes multiple concentrations of inhibitor.

Many ACE inhibitors are known in the art and may be prepared by known methods or by variations thereof. The ACE inhibitors of the invention may exist in isomeric form. The invention contemplates all such isomers both in pure form and admixture, including racemic mixtures.

For purposes of the invention, the term beta adrenergic blocker means a compound which by binding to beta adrenergic plasma membrane receptors reduces or eliminates sympathetic activity or blocks the effects of exogenously administered catecholamines or adrenergic drugs. See, for example, Weiner, N., Drugs That Inhibit Adrenergic Nerves and Block Adrenergic Receptors, in the Pharmaceutical Basis of Therapeutics (ed. A.G. Goodman, L.S. Goodman, A. Gilman), Macmillan Publishing, New York, 1980, 6th ed., pp. 188-197.

Examples of preferred beta adrenergic blockers are atenolol (4-[2-hydroxy-3-[(1-methylethyl)amino]-propoxy]benzeneacetamide), metoprolol (1-[4-(2-methoxyethyl)phenoxy]-3-[(1-methylethyl)amino]-2-propanol), nadolol (5-[3-[(1,1-dimethylethyl)amino]-2-hydroxypropoxy]-1,2,3,4-tetrahydro-2,3-naphthalenediol), pindolol (1-(1H-indol-4-yloxy)-3-[(1-methylethyl)amino]-2-propanol), propranolol (1-[(1-methylethyl)amino]-3-(1-naphthalenyloxy)-2-propanol), timolol (1-[(1,1-dimethylethyl)amino]-3-[(4-morpholinyl-1,2,5-thiadiazol-3-yl)-oxy]-2-propanol), labetalol (2-hydroxy-5-[1-hydroxy-2-[(1-methyl-3-phenylpropyl)amino]ethyl]-benzamide), betaxolol, 1-[4-[2-(cyclopropylmethoxy)ethyl]-phenoxy]-3-[(methylethyl)amino]-2-propanol, carteolol, 5-[3-[-(1,1-dimethylethyl)amino]-2-hydroxypropoxy]-3,4-dihydro-2(1H)-quinolinone, dilevolol, [R-(R,R)]-2-hydroxy-5-[1-hydroxy-2-[(1-methyl-3-phenylpropyl)amino]ethyl]-benzamide 4-methylbenzenesulfonate salt and bunolol (5-(3-tert-butylamino-2-hydroxypropoxy)-3,4-dihydro-1(2H)-naphthalinon).

The usefulness of beta adrenergic blockers for lowering intraocular pressure is known in the art. Thus, the beta adrenergic blocker timolol, is currently approved by the U.S. Food and Drug Administration for topical intraocular use for the treatment of glaucoma. It is marketed in two dose strengths, i.e., 0.25% and 0.5%. As previously stated, this agent must be used with caution in a defined patient population because of recognized untoward side effects (see Physicians Desk Reference for Ophthalmology, 11th edition, 1983, p. 126, Medical Economics Co. Inc., Oradell, N.J. 07649).

In the following paragraphs certain aspects of the invention are discussed in more detail. The pharmaceutical compositions discussed therein are either compositions comprising a beta adrenergic blocker together with an ACE inhibitor or are compositions comprising either a beta adrenergic blocker or an ACE inhibitor for combined use of the beta adrenergic blocker and the ACE inhibitor. The compositions are for topical ophthalmic use. Preferably the compositions are in the form of solutions, but other known ophthalmic compositions such as for example gels and inserts are contemplated as well.

In the discussion the compositions are defined by the amount of active components per dosage unit. Preferred are compositions in the form of solutions for which the dosage unit is one drop (50µl). Thus, for example, a suitable composition is a 0.25% solution (w/v) which is known to contain 2.5 mg of the active component per ml or 125 µg per 50 µl (1 drop). The concentration of the active components in other types of formulations can be established by known methods. It is to be noted that in the treatment of ocular pressure the actual dose needed to give a certain reduction of the intraocular pressure does not only depend on the potency of the active component or combination of components but also on the age, size and condition of the individual being treated as well as on the severity of the disease state, especially the magnitude of the intraocular pressure which is to be reduced.

In the following discussion the term compound A stands for 7-[N-(1(S)-carboxy-3-phenylpropyl)-(S)-alanyl]-1,4-dithia-7-azaspiro[4.4]nonane-8(S)-carboxylic acid.

One aspect of the present invention is to provide pharmaceutical compositions for reduction in intraocular pressure by combined use of a beta adrenergic blocker and an ACE inhibitor whereby the dosage of either one of the components is lower than the dosage used in monotherapy using either the beta adrenergic blocker or the ACE inhibitor alone. The reduction of the IOP provided by this combination therapy is at least as large as obtained by the known monotherapy. The use of diminished doses of the components results in a reduction of severity and frequency of side effects related to the components.

The compositions according to this aspect of the invention provide preferably from 50 µg to 250 µg per dosage unit of the beta adrenergic blocker and from 0.025µg to 5µg per dosage unit of the ACE inhibitor, especially from 50µg to 125µg per dosage unit of the beta adrenergic blocker and 0.5µg to 1.0µg per dosage unit of the ACE inhibitor.

For example a reduction in intraocular pressure equivalent to that obtained by use of the approved clinical dose of timolol may be obtained by use of a lower dose of timolol when such lower dose is combined with an effective amount of compound A. It is anticipated that the use of the diminished dosage of the components especially of timolol will result in a reduction of severity and frequency of related side effects. The composition preferably provides per dosage unit from 50µg to 250µg, especially 50µg timolol and from 0.025µg to 5µg in particular 0.05 to 0.5µg of compound A.

Another aspect of the invention is to provide compositions for reduction in intraocular pressure by combined use of a beta adrenergic blocker and an ACE inhibitor whereby subthreshold doses of both components are provided.

Herein the term "subthreshold intraocular pressure reducing concentration" of ACE inhibitor e.g. 7-[N-(1(S)-carboxy-3-phenylpropyl)-(S)-alanyl]-1,4-dithia-7-azaspiro[4.4]-nonane-8(S)-carboxylic acid (compound A), means an amount less than the threshold concentration of 0.001% (w/v). Similarly, the term "subthreshold intraocular pressure reducing concentration" of beta adrenergic blocker, e.g. timolol, means an amount less than 0.25% (w/v). The threshold concentration of other ACE inhibitors is the minimum concentration of the respective ACE inhibitor providing the effect of the 0.001% solution of compound A. The threshold concentration of other beta adrenergic blockers is the minimum concentration of the respective beta adrenergic blocker providing the effect of the 0.25% timolol solution.

Those skilled in the art will appreciate that the "subthreshold intraocular pressure reducing concentration" will consist of a range of concentrations, and that there will be a lower limit to said concentration below which the present invention will not operate. For purposes of this invention, this lower limit may be considered to be about 5% of the threshold concentration of the particular component.

Accordingly for the preferred formulations the term subthreshold dose can be defined as one drop (50µl) of a solution of a concentration between threshold concentration and 5% of the threshold concentration. If compositions other than solutions are utilized the threshold dose can be defined by appropriate terms.

The subthreshold intraocular pressure reducing concentration and dose respectively that is actually utilized, whether for an angiotensin converting enzyme inhibitor or for a particular beta adrenergic blocker will depend on, inter alia, the potency of each particular material, the combination being administered and the age, size and condition of the patient being treated as well as on the severity of th disease state.

The clinical implication of this aspect of the invention is that a combination of the two drugs will result in clinically significant lowering of IOP without the systemic side effects e.g. bradycardia and bronchoconstriction which occur with IOP-lowering effective concentrations of beta adrenergic blockers. No significant beta adrenergic blocker related side effects are projected for the combination.

The compositions according to this aspect of the invention provide preferably 5 µg to 125 µg (especially 50 µg to 100 µg) per dosage unit of the beta adrenergic blocker and 0.005 µg to 1 µg (especially 0.05 µg-0.5 µg) per dosage unit of the ACE inhibitor.

The IOP can be significantly reduced for example by pharmaceutical compositions providing between 6.25 µg and 125 µg (preferably 50 µg) per dosage unit of timolol and between 0.025 µg and 0.5 µg (especially 0.05 µg to 0.5 µg) per dosage unit of compound A.

Another aspect of the present invention is to provide pharmaceutical compositions for reducing the intraocular pressure by combined use of a beta adrenergic blocker and an ACE inhibitor whereby the dosage of the one component is the dosage needed to give the maximum lowering of IOP attainable from monotherapy using this component alone and the dosage of the other component is in the range of subthreshold dosages as defined above. In particular the compositions according to this aspect of the invention provide the dosage of the beta adrenergic blocker needed to give the maximum lowering of IOP attainable from monotherapy using this beta adrenergic blocker and a subthreshold dosage of the ACE inhibitor.

It is to be noted that the actual dosage needed to give the maximum lowering of IOP attainable in monotherapy depends on the patient and the degree of IOP.

The pharmaceutical compositions according to this aspect of the invention give significantly increased lowering of IOP pressure.

The clinical implication of this is that patients whose elevated IOP (e.g. glaucoma) is not adequately controlled by maximum recommended concentrations of standard therapy e.g. timolol, can be treated with the combination of an ACE inhibitor and a beta adrenergic blocker to achieve greater lowering of IOP than that attainable by either treatment alone. Such treatment will result in greater clinical benefit with no increase in side effects.

The compositions according to this aspect of the invention preferably provide from 25 µg to 500 µg (preferably 50-250µg) per dosage unit of the beta adrenergic blocker and from 0.005 to 1 µg (preferably

0.025-0.5μg) per dosage unit of the ACE inhibitor. For example compositions according to this aspect of the invention provide 50 to 250 μg preferably 250 μg timolol per dosage unit and 0.005μg to 0.5μg, preferably 0.005 μg to 0.05 μg of compound A per dosage unit.

The intraocular pressure (IOP) lowering effects of the compositions of the invention have been shown by the following procedure wherein timolol as the representative of the beta adrenergic blockers and 7-[N-(1(S)-carboxy-3-phenylpropyl)-(S)-alanyl]-1,4-dithia-7-azaspiro[4.4]nonane-8(S)-carboxylic acid (referred to as compound A) as the representative of the angiotensin converting enzyme inhibitors:

Male New Zealand white rabbits weighing 1.3 to 1.8 kg and having a normal IOP (18-23 mmHg) were allowed a 1 week acclimation period prior to starting conditioning and drug studies. Food and water were allowed ad libitum. The rabbits were maintained on a light cycle of 12 h darkness (7 PM to 7 AM) followed by 12 h of light. Animals were conditioned to their handlers and the laboratory setting and IOP measurements for at least one 4 h period before being used to study drug effects.

MEASUREMENT OF IOP

Rabbits were restrained in a cloth sack approximately 2 min prior to IOP determinations. The left lower eyelid was retracted forming a pouch and 1 drop of the local anesthetic proparacaine (0.05% w/v) was irrigated over the cornea. The eyelid was then held closed over the eye about 1 min. IOP was usually monitored 1 h before dosing, at zero time (immediately prior to dosing), and hourly for the next 4 h. Only rabbits with IOP's of 18-23 mmHg and showing no sign of ocular irritation were used in drug studies.

IOP, in mmHg, was determined in triplicate with a McKag-Marg applanation tonometer and the average reading was recorded. The tonometer had previously been calibrated against direct manometric determination of IOP in living eyes of barbiturate-anesthetized rabbits. Internal calibration signals of the McKay-Marg unit were checked after every eight readings or less.

Generally, 16 or more rabbits selected at random were assigned to a placebo or one of several concurrently run drug groups. Prior to drug administration and at each IOP determination, the cornea, iris, and conjunctivae were grossly examined for signs of irritation. Pupil diameter was also examined but detailed measurements were not made.

DRUG PREPARATION AND ADMINISTRATION

Drug solution were prepared fresh daily no more than 1 hr prior to their administration. One percent solutions (concentrations refer to active material, w/v%) were prepared using one of two vehicles (vehicle 1 or PBS, described below). Lower concentrations were prepared by serial dilution with the appropriate vehicle. The pH of these isotonic solutions was 7.4 or 7.0. Fifty μl of test solution was applied with a pipette to the left eye of conscious rabbits. The method of administration was otherwise identical to that described above for a proparacaine. Rabbits treated with vehicle 1 or compound A solutions were also dosed with 50μl of PBS. Animals treated with timolol were also dosed with either 50μl of vehicle 1 or compound A solutions.

The data (mean I SEM) were analyzed using analysis of variance and Dunnett's or Duncan's comparisons and are expressed as peak changes in IOP. Concentrations always refer to active materials and not to their salt form.

| Composition of Vehicles Used to Dissolve Compounds for Topical Administration | | |
|---|---|---|
| Material | mg/ml | |
| | Vehicle 1[a] | PBS[b] |
| Sodium phosphate, diabasic | 10.4 | 10.4 |
| Sodium phosphate, monobasic | 2.4 | 2.4 |
| Chlorobutanol | 5.0 | |
| Hydroxypropyl ethylcellulose | 5.0 | |
| Purified distilled water qs. ad. | 1.0 ml | |
| 0.9% NaCl for injection qs. ad. | | 1.0 ml |
| pH | 7.4 | 7.0 |

a) used for compound A
b) Phosphate buffered saline, the vehicle used for timolol

The following results have been obtained:

| Treatment | | Peak Change in IOP (mmHg) |
|---|---|---|
| Vehicle 1 | alone | -.05±0.5 |
| PBS -0.5±0.5 | " | |
| 0.5% Timolol[(2)] | alone | -3.0±0.4[(1)] |
| 0.00001% compound A | alone | -1.2±0.4 |
| 0.0001% " " | " | -1.7±0.8 |
| 0.001% " " | " | -3.3±0.2[(1)] |
| 0.5% Timolol plus | | |
| 0.00001% compound A | | -6.2±0.6[(1)(3)] |
| 0.0001% " " | | -6.2±0.6[(1)(3)] |
| 0.001% " " | | -6.2±0.6[(1)(3)] |

| | |
|---|---|
| 0.1% Timolol plus | |
| 0.00001% compound A | -4.8±0.4(1)(3) |
| 0.0001% " " | -6.3±0.4(1)(3) |
| 0.001% " " | -7.0±0.4(1)(3) |

(1) Statistically significant reduction of IOP vs placebo treatments.
(2) In this study the concentration of timolol which produced the maximum reduction of IOP in applying timolol as the only IOP reducing agent.
(3) Significantly different from response to either timolol or compound A alone.

None of the rabbits treated with vehicles, timolol, compound A or timolol in combination with compound A showed any signs of acute ocular irritation. Pupillary diameters were not affected.

This study establishes that combined administration of ACE inhibitors, e.g. compound A and beta adrenergic blockers, e.g. timolol leads to greater acute efficacy in reducing IOP than can be obtained with either agent alone. It can be concluded that combined administration of ACE inhibitors, e.g. compound A and beta adrenergic blockers, e.g. timolol is a novel and potentially useful approach for controlling elevated IOP associated with the human simple glaucoma syndrome.

The pharmaceutical compositions of the invention (ACE inhibitors and beta adrenergic blockers) are administered in the form of ophthalmic pharmaceutical compositions adapted for topical administration to the eye; such as solutions, suspensions, ointments and solid inserts. Formulations of the invention may contain the following amounts of each constituent:

ACE inhibitor from 0.00001 to 0.1% (w/v) and especially 0.001 to 0.01% of medicament. As a unit dosage form, an amount of ACE inhibitor from between 0.005µg to 50 µg, preferably 0.05µg to 10µg., and especially 0.5µg to 5µg of the active composition is applied to the human eye.

Beta adrenergic blocker from 0.001 to 1% (w/v) and especially 0.01 to 0.5% of medicament. As a unit dosage form, an amount of beta adrenergic blocker from between 0.5µg to 500µg and preferably 5µmg to 250µg of the active composition is applied to the human eye. Other concentrations may be employed provided the dose is effective in lowering the intraocular pressure. For example, suitable concentrations of the active components in preferred compositions can be calculated on the basis of the above detailed discussion of various aspects of the invention. Individual dosage requirements, i.e. the amount of each dose and the frequency of administration will depend on the severity of the disease and the response of the patient.

In the preferred method of the invention, it is anticipated that both active ingredients, i.e. ACE inhibitor and beta adrenergic blocker, will be administered simultaneously and be contained in one pharmaceutical dosage form, each component being present in the dosage form in its own respective preferred concentration.

Where utilized herein, the term "controlling the elevated intraocular pressure associated with glaucoma" means the regulation, attenuation and modulation of increased intraocular tension which is the primary diagnostic symptom of the disease, glaucoma. The term also means that the diminution, in the otherwise elevated intraocular pressure, obtained by the practice of the invention is maintained for a significant period of time as for example, between consecutive doses of the composition of the invention.

To prepare suitable dosage forms, the active compositions may be conveniently admixed with a non-toxic pharmaceutically acceptable carrier suitable for topical ophthalmolgic administration. Typical of such pharmaceutically acceptable carriers are, for example, water, mixtures of water and watermiscible solvents such as lower alkanols or vegetable oils, petroleum based jelly, and including also from 0.5 to 5% by weight of hyroxyethyl cellulose, ethyl oleate, carboxymethyl cellulose, polyvinylpyrrolidone, and other water soluble ophthalmologically acceptable non-toxic polymers, for example, cellulose derivatives such as methyl cellulose, alkali metal carboxymethyl cellulose, hydroxyethyl cellulose, hydroxypropyl cellulose, hydroxypropylmethyl cellulose; acrylates such as polyacrylic acids salts, ethylacrylates; polyacrylamides; natural products such as gelatin, alginates, pectins, tragacanth, karaya, chondrus, agar, acacia; the starch derivatives such as starch acetate, hydroxyethyl starch ethers, hydroxypropyl starch, as well as other synthetic derivatives such as polyvinyl alcohol, polyvinyl pyrrolidone, polyvinyl methyl ether, polyethylene oxide, neutralized carbopol and xanthan gum and mixtures of these polymers. The pharmaceutical preparation may also contain non-toxic auxiliary substances such as emulsifying, preserving, wetting and bodying agents, as for example, polyethylene glycols 200, 300, 400 and 600, carbowaxes 1,000, 1,500, 4,000, 6,000 and 10,000, antibacterial components such as quarternary ammonium compounds; phenylmercuric salts known to have cold sterilizing properties and which are non-injurious in use; thimerosal, methyl and propyl paraben, benzyl alcohol, phenyl ethanol; buffering ingredients such as alkali metal chloride, borate, acetate, gluconate buffers; antioxidants such as sodium metabisulfite, butylated hydroxyanisol (BHA) and butylated hydroxytoluene (BHT); and other conventional ingredients such as sorbitan monolaurate, triethanolamine, oleate, polyoxyethylene sorbitan monopalmitylate, dioctyl alkali metal sulfosuccinate, monothioglycerol and ethylenediamine tetracetic acid.

Additionally, suitable ophthalmic vehicles can be used as carrier media for the present purpose including conventional phosphate buffer vehicle systems, isotonic boric acid vehicles, isotonic alkaki chloride vehicles and tris.

The pharmaceutical preparation may also be in the form of a solid insert. For example, one may use a solid water soluble polymer as the carrier for the medicament. Inserts that are known in the art that are suitable for this use include those described in British Patent 15611, and in United States Patents 3,993,071; 3,986,510; 3,868,445; and 3,867,510. Solid water insoluble inserts, such as those prepared from ethylene vinyl acetate copolymer, may also be utilized.

The compositions of the invention may include additional therapeutic agents in addition to the ACE inhibitor and beta adrenergic blocker. For example antibiotics, anesthetics as well as other IOP lowering agents may be present.

The pharmaceutical preparation may be prepared according to known methods.

Example:

| OPHTHALMIC SOLUTION | |
|---|---|
| INGREDIENTS | WEIGHT % |
| 7-[N-(1(S)-carboxy-3-phenylpropyl) -S-alanyl]-1,4-dithia-7-azaspiro[4.4] nonane-8(S)-carboxylic acid | 0.0001-0.001 |
| Timolol | 0.1 - 0.5 |
| Benzalkonium Chloride | 0.01 |
| Dibasic Sodium Phosphate, Anhydrous | 1.04 |
| Monobasic Sodium Phosphate, Monohydrate | 0.24 |
| Sodium Chloride (q.s. to adjust to isotonicity) | -- |
| Purified Water q.s. to make | 1.0 ml |
| The ingredients are mixed in a suitable mixer. | |

## Claims

**1.** A topical ophthalmologically acceptable composition useful for reducing and/or controlling the elevated intraocular pressure associated with glaucoma which comprises an ophthalmologically acceptable angiotensin converting enzyme inhibitor and an ophthalmologically acceptable beta adrenergic blocker in combination with an ophthalmologically acceptable carrier for topical use.

**2.** The composition according to claim 1 which comprises an angiotensin converting enzyme inhibitor having the formula

(a)

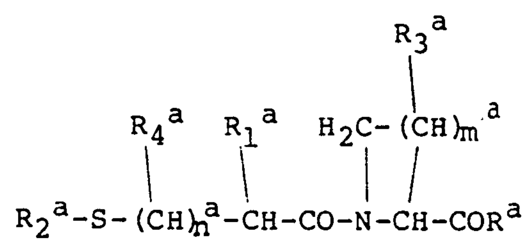

wherein

$R^a$ is hydroxy, $NH_2$ or lower alkoxy;

$R_1{}^a$ and $R_4{}^a$ each is hydrogen, lower alkyl, phenyl or phenyl-lower alkyl;

$R_2{}^a$ is hydrogen, lower alkyl, phenyl, substituted phenyl wherein the phenyl substituent is halo, lower alkyl or lower alkoxy, phenyl-lower alkyl, diphenyl-lower alkyl, triphenyl-lower alkyl, lower alkylthiomethyl, phenyl-lower alkylthiomethyl, lower alkanoyl-amidomethyl,

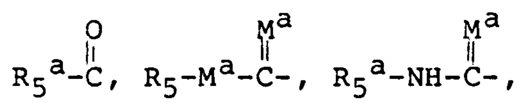

$R_6{}^a$-S-, or $R_7{}^a$;

$R_3{}^a$ is hydrogen, hydroxy or lower alkyl;

$R_5{}^a$ is lower alkyl, phenyl or phenyl-lower alkyl;

$R_6{}^a$ is lower alkyl, phenyl, substituted phenyl (wherein the phenyl substituent is halo, lower alkyl or lower alkoxy), hydroxy-lower alkyl or amino (carboxy)-lower alkyl;

$R_7{}^a$ is

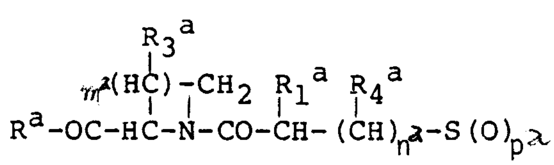

$M^a$ is O or S;

$m^a$ is 1 to 3;

$n^a$ and $p^a$ each is 0 to 2;

(b) having the formula

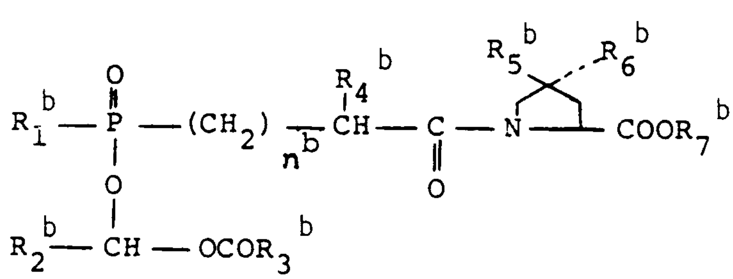

$R_1{}^b$ is alkyl, aryl, aralkyl, cycloalkyl or cycloalkylalkyl;

$R_2{}^b$ is cycloalkyl, 3-cyclohexenyl or 2-alkyl-3-cyclohexenyl;

$R_3{}^b$ is alkyl, cycloalkyl or phenyl;

$R_4{}^b$ is H or alkyl;

One of $R_5{}^b$ and $R_6{}^b$ is H and the other is alkyl-$X^b$-, phenyl-$X^b$-alkoxy, phenoxy, phenyl, cycloalkyl,

alkyl or phenylalkyl; or
$R_5{}^b$ and $R_6{}^b$ together form $-X^bCH_2CH_2X^b-$, $X^b$ is S, SO or $SO_2$;
$R_7{}^b$ is H or $CH(R_2{}^b)OCOR_3{}^b$;
$n^b$ is O or 1;
'aryl' is phenyl optionally substituted by halo, alkyl, alkoxy, alkylthio, OH, alkanoyl, $NO_2$, amino, dialkylamino and/or $CF_3$;
alkyl has 1-10C, cycloalkyl 3-7C, alkoxy 1-8C and alkanoyl 2-9C-atoms;
(c) having the formula

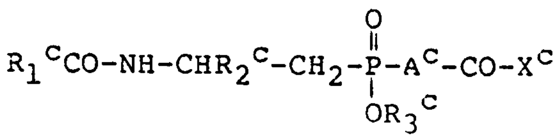

$R_1{}^c$ and $R_2{}^c$ are H, 1-7C alkyl, 1-7C haloalkyl, $(CH_2)_m{}^c-D^c$, 1-7C aminoalkyl or a group of the formula:

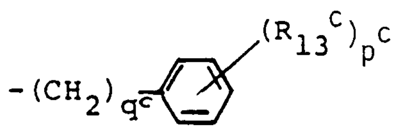

$D^c$ is cycloalkyl, furyl, thienyl or pyridinyl;
$A^c$ is $(CH_2)_n-CHR_{21}{}^c$, $NR_{22}{}^c-CHR_{23}{}^c$ or $O-CHR_{23}{}^c$;
$n^c$ is 0 or 1;
$q^c$ is 0-7;
$R_{21}{}^c$ is H, 1-7C alkyl, 1-7C haloalkyl, benzyl or phenethyl;
$R_{22}{}^c$ is H or 1-7C alkyl;
$R_{23}{}^c$ is H, 1-7C alkyl, 1-7C haloalkyl or $(CH_2)_r{}^cD_1{}^c$;
$D_1{}^c$ is phenyl, 4-hydroxyphenyl, 3,4-dihydroxyphenyl, 3-indolyl, 4-imidazolyl, $NH_2$, SH, 1-7C alkylthio, guanidino or $CONH_2$;
$X^c$ is a group of formula $(II)^c$-$(V)^c$, or $NR_4{}^c-CHR_5{}^c-COOR_6{}^c$;

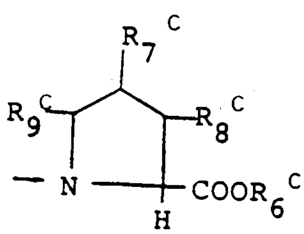

$(II)^c$

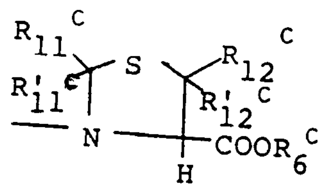

$(III)^c$

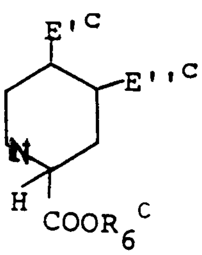

$(IV)^c$

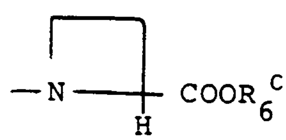

$(V)^c$

where
$R_9^c$ and $R_8^c$ are H and $R_7^c$ is as defined below;
$R_9^c$ and $R_7^c$ are H and $R_8^c$ is as defined below;
$R_7^c$ and $R_8^c$ are H and $R_9^c$ is as defined below;
$R_9^c$ and $R_8^c$ are H and $-CHR_7^c-$ is replaced by $-CR_{10}^cR_{10}c$;
$R_9^c$ is H and $R_7^c$ + $R_8^c$ form a double bond;
$R_9^c$ is H and $R_7^c$ + $R_8^c$ complete a fused benzene ring; or
$R_8^c$ is H and $R_9^c$ + $R_7^c$ complete a fused benzene ring;
$E'^c$ and $E''^c$ are H or $E'^c$ + $E''^c$ complete a fused benzene ring;
$R_7^c$ is H, 1-7C alkyl, halogen, keto, OH, 2-8C alkanoyl-amino, $N_3$, $NH_2$, $NR_{19}^cR_{20}^c$, $(CH_2)_m^c$-$D^c$, O-$CONR_{15}^cR_{15}^c$, 1-7C alkoxy, 1-7C alkylthio, or a group of formula $(VI)^c$, $(VII)^c$ or $(VIII)^c$:

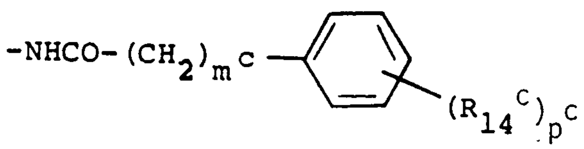

$(VI)^c$

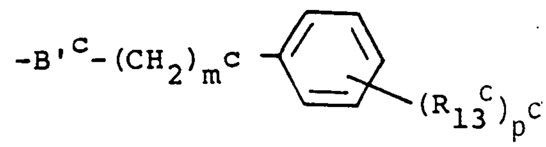

$(VII)^c$

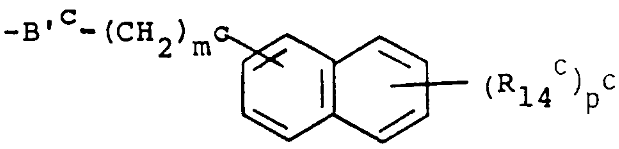

$(VIII)^c$

$B'^c$ is a bond or it is O or S;$R_8^c$ is keto, halogen, O-$CONR_{15}^cR_{15}^c$, 1-7C alkoxy, 1-7C alkylthio, or a group $(VII)^c$ or $(VIII)^c$ in which $B'^c$ is O or S;
$R_9^c$ is keto or a group $(VII)^c$ in which $B'^c$ is a bond;
$R_{10}^c$ is halogen or $Y^cR_{16}^c$;
$R_{11}^c$, $R'_{11}{}^c$, $R_{12}^c$ and $R'_{12}{}^c$ are H or 1-7C alkyl, or $R_{11}^c$ is a group of formula $(IX)^c$ and the other 3 are H;

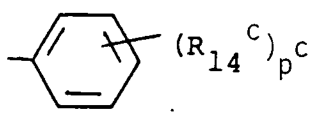

$(IX)^c$

$R_{13}^c$ is H, 1-4C alkyl, 1-4C alkoxy, 1-4C alkylthio, Cl, Br, F, $CF_3$, OH, Ph, PhO, Phs or $PhCH_2$;
$R_{14}^c$ is H, 1-4C alkyl, 1-4C alkoxy, 1-4C alkylthio, Cl, Br, F, $CF_3$ or OH;
$m^c$ is 0-3;
$p^c$ is 1-3 but it is 2 or 3 only when $R_{13}^c$ or $R_{14}^c$ is H, Me, MeO, Cl or F;
$R_{15}^c$ is H or 1-4C alkyl;
$Y^c$ is O or S;
$R_{16}^c$ is 1-4C alkyl or group $(VII)^c$ in which $B'^c$ is a bond; or the two $R_{16}^c$ groups complete a 5-or 6-membered ring in which the C atoms may each bear 1 or 2 alkyls having 1-4C atoms;
$R_4^c$ is H, 1-7C alkyl, cycloalkyl or $(CH_2)_r^c$Ph;
$R_5^c$ is H, 1-7C alkyl or $(CH_2)_r^c$-$D_1^c$;
$r^c$ is 1-4;
$R_3^c$ and $R_6^c$ are H, 1-7C alkyl, $PhCH_2$, PhCH or $CHR_{17}^c$-O-$COR_{18}^c$;
$R_{17}^c$ is H, 1-7C alkyl or Ph;
$R_{18}^c$ is H, 1-7C alkyl, 1-7C alkoxy or Ph; or $R_{17}^c$ + $R_{18}^c$ form $(CH_2)_2$, $(CH_2)_3$, -CH=CH- or o-phenylene;
$R_{19}^c$ is 1-7C alkyl, benzyl or phenethyl; and
$R_{20}^c$ is H or chosen as for $R_{19}^c$;
(d) having the formula

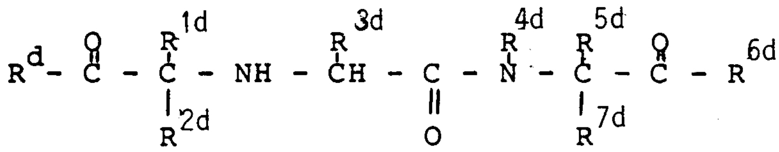

wherein

$R^d$ and $R^{6d}$ are the same or different and are hydroxy, lower alkoxy, lower alkenoxy, dilower alkylamino lower alkoxy (dimethylaminoethoxy), acylamino lower alkoxy (acetylaminoethoxy), acyloxy lower alkoxy (pivaloyloxymethoxy), aryloxy, such as phenoxy, arloweralkoxy, such as benzyloxy, substituted aryloxy or substituted arloweralkoxy wherein the substituent is methyl, halo or methoxy, amino, lower alkylamino, dilower alkylamino, hydroxyamino, arloweralkylamino such as benzylamino;

$R^{1d}$ is hydrogen, alkyl of from 1 to 20 carbon atoms which include branched and cyclic and unsaturated (such as allyl) alkyl groups, substituted loweralkyl wherein the substituent can be halo, hydroxy, lower alkoxy, aryloxy such as phenoxy, amino, dilower alkylamino, acylamino, such acetamido and benzamido, arylamino, guanidino, imidazolyl, indolyl, mercapto, lower alkylthio, arylthio such as phenylthio, carboxy or carboxamido, carboloweralkoxy, aryl such as phenyl or naphthyl, substituted aryl such as phenyl wherein the substituent is lower alkyl, lower alkoxy or halo, arloweralkyl, arloweralkenyl, heteroarloweralkyl or heteroarloweralkenyl such as benzyl, styryl or indolyl ethyl, substituted arloweralkyl, substituted arloweralkenyl, substituted heteroarloweralkyl, or substituted heteroarloweralkenyl, wherein the substituent(s) is halo, dihalo, lower alkyl, hydroxy, lower alkoxy, amino, aminomethyl, acylamino (acetylamino or benzoylamino) diloweralkylamino, lower alkylamino, carboxyl, haloloweralkyl, cyano or sulfonamido; arloweralkyl or heteroarloweralkyl substituted on the alkyl portion by amino or acylamino (acetylamino or benzoylamino);

$R^{2d}$ and $R^{7d}$ are the same or different and are hydrogen or lower alkyl;

$R^{3d}$ is hydrogen, lower alkyl, phenyl lower alkyl, aminomethyl phenyl lower alkyl, hydroxy phenyl lower alkyl, hydroxy lower alkyl, acylamino lower alkyl (such as benzoylamino lower alkyl, acetylaminoloweralkyl), aminoloweralkyl, dimethylaminoloweralkyl, haloloweralkyl, guanidinoloweralkyl, imidazolylloweralkyl, indolylloweralkyl, mercaptoloweralkyl, loweralkylthioloweralkyl;

$R^{4d}$ is hydrogen or lower alkyl;

$R^{5d}$ is hydrogen, lower alkyl, phenyl, phenylloweralkyl, hydroxyphenylloweralkyl, hydroxyloweralkyl, aminoloweralkyl, guanidinoloweralkyl, imidazolylloweralkyl, indolylloweralkyl, mercaptoloweralkyl or loweralkylthio loweralkyl;

$R^{4d}$ and $R^{5d}$ may be connected together to form an alkylene bridge of from 2 to 4 carbon atoms, an alkylene bridge of from 2 to 3 carbon atoms and one sulfur atom, an alkylene bridge of from 3 to 4 carbon atoms containing a double bond or an alkylene bridge as above substituted with hydroxy, lower alkoxy, lower alkyl or dilower alkyl; and the pharmaceutically acceptable salts thereof;

(e) having the formula

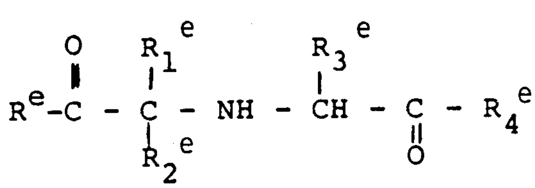

$R_4{}^e$ is a substituted proline of the formula

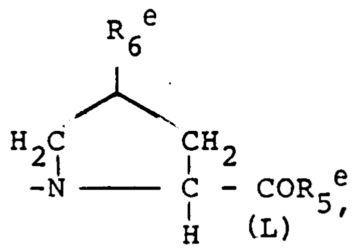

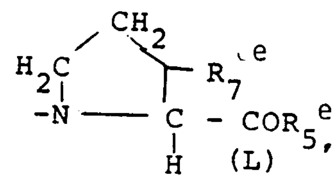

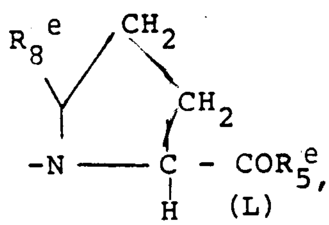

or

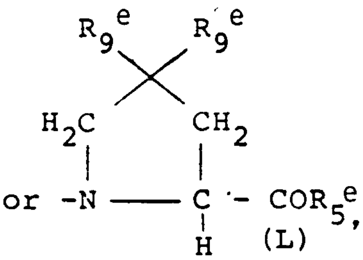

$R_6{}^e$ is halogen, keto, azido,

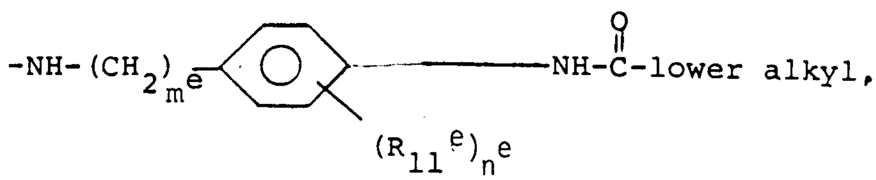

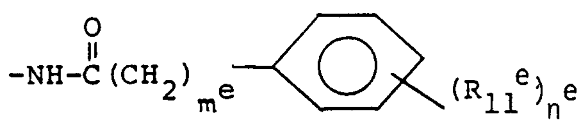

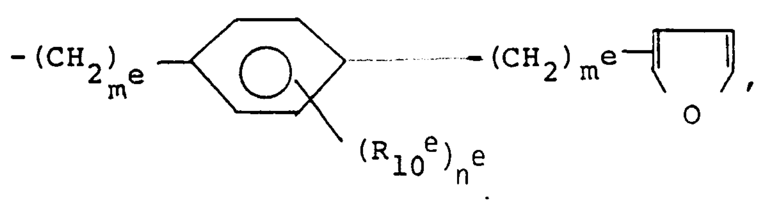

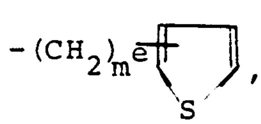

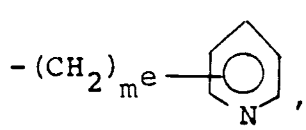

a 1- or 2-naphthyl of the formula

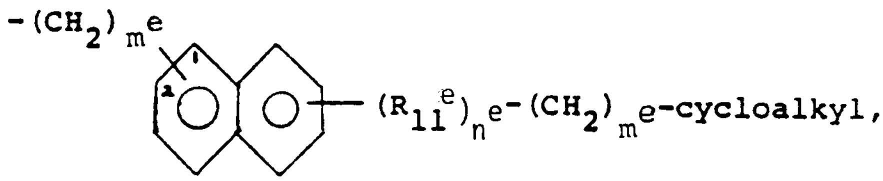

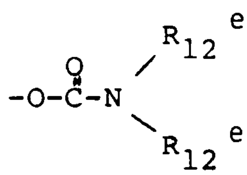

,

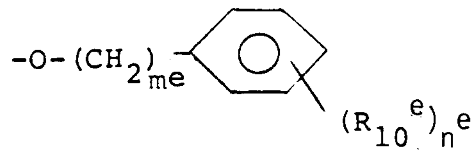

a 1- or 2-naphthyloxy of the formula

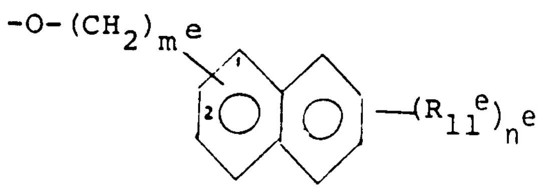

-S-lower alkyl,

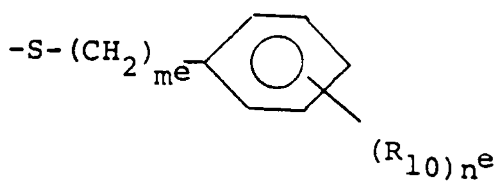

or a 1- or 2-naphthylthio of the formula

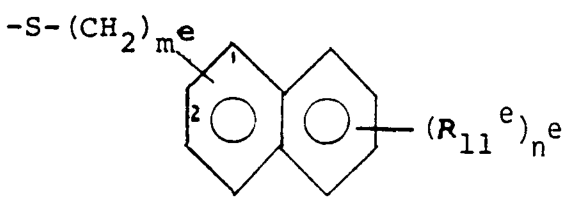

$R_7{}^e$ is keto, halogen,

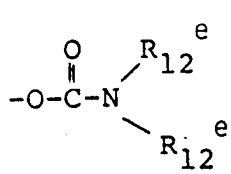

,

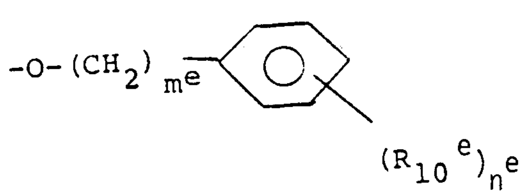

a 1- or 2-naphthloxy of the formula

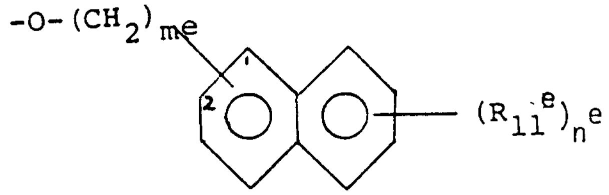

-S-lower alkyl,

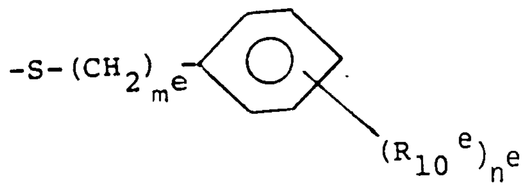

or a 1- or 2-naphthylthio of the formula

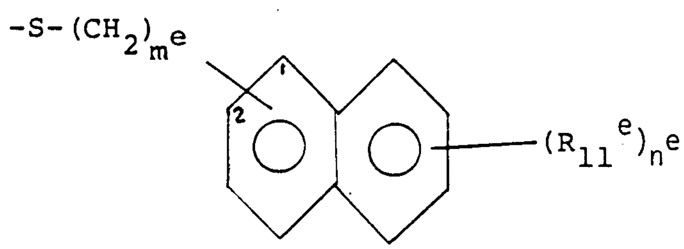

$R_8{}^e$ is keto or

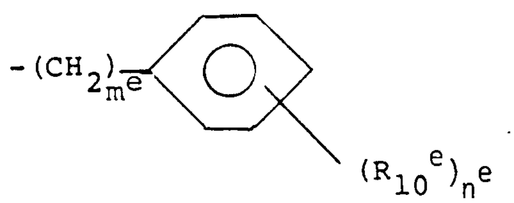

$R_9{}^e$ is halogen or $-Y^e-R_{13}{}^e$.
$m^e$ is zero, one, two, or three.
$R_{10}{}^e$ is hydrogen, lower alkyl of 1 to 4 carbon atoms, lower alkoxy of 1 to 4 carbon atoms, lower alkylthio of 1 to 4 carbon atoms, chloro, bromo, fluoro, trifluoromethyl, hydroxy, phenyl, phenoxy, phenylthio, or phenylmethyl.
$R_{11}{}^e$ is hydrogen, lower alkyl of 1 to 4 carbon atoms, lower alkoxy of 1 to 4 carbon atoms, lower alkylthio of 1 to 4 carbon atoms, bromo, fluoro. trifluoromethyl, or hydroxy.
$n^e$ is one, two or three provided that n is more than one only if $R_{10}{}^e$ or $R_{11}{}^e$ is hydrogen, methyl, methoxy, chloro, or fluoro.
$R_{12}{}^e$ is hydrogen or lower alkyl of 1 to 4 carbon atoms.
$Y^e$ is oxygen or sulfur.
$R_{13}{}^e$ is lower alkyl of 1 to 4 carbon atoms.

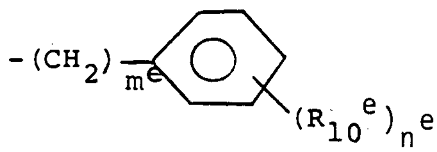

or the $R_{13}^e$ group join to complete an unsubstituted 5- or 6-membered ring or said ring in which one or more of the carbon atoms has a lower alkyl of 1 to 4 carbon atoms, or a di(lower alkyl of 1 to 4 carbon atoms) substituent.

$R^e$ and $R_5^e$ are independently selected from hydroxy; lower alkoxy, di(lower alkyl)-amino-lower alkoxy, such as dimethylaminoethoxy, lower alkyl-carbonyl-amino-lower alkoxy, such as acetylaminoethoxy, lower alkyl-carbonyloxy-lower alkoxy, such as pivaloyloxymethoxy.

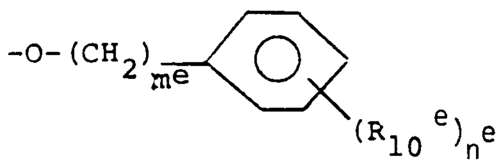

wherein $m^e$, $n^e$ and $R_{10}^e$ are as defined above, amino, lower alkyl-amino, di(lower alkyl)-amino, hydroxyamino, benzylamino, or phenethylamino. $R_1^e$ is hydrogen, lower alkyl,

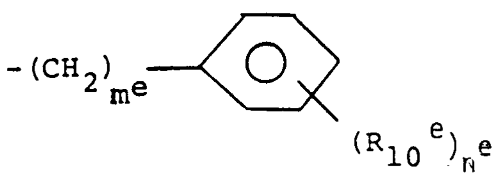

halo substituted lower alkyl, hydroxy substituted lower alkyl, $-(CH_2)_q^e$-cycloalkyl, $-(CH_2)_q^e$-carboxy, $-(CH_2)_q^e$-S-lower alkyl,

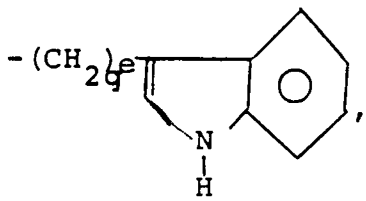

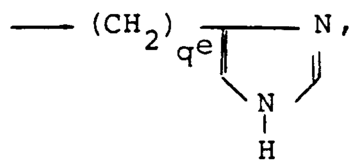

$-(CH_2)_q{}^e$-guanidinyl, $-(CH_2)_q{}^e-NH_2$

$-(CH_2)_q{}^e-N(\text{lower alkyl})_2$

$-(CH_2)_q{}^e-NH-\overset{O}{\overset{\|}{C}}-\text{lower alkyl}$,

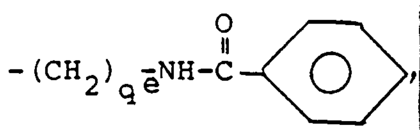, $-(CH_2)_q{}^e-SH$,

$-(CH_2)_q{}^e$-lower alkoxy,

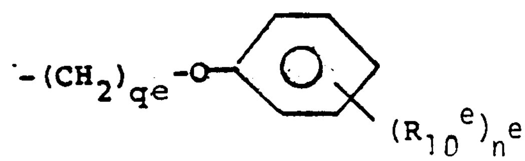

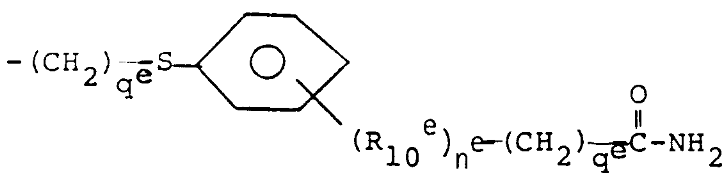

$-(CH_2)_q{}^e-\overset{O}{\overset{\|}{C}}-\text{lower alkoxy}$, or

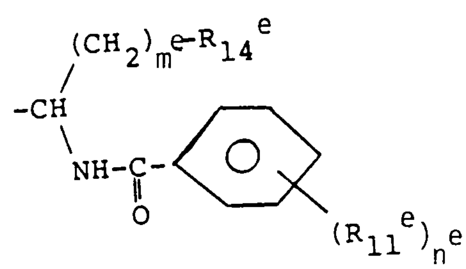

wherein $m^e$, $n^e$, $R_{10}{}^e$ and $R_{11}{}^e$ are as defined above, $R_{14}{}^e$ is lower alkyl, cycloalkyl, or

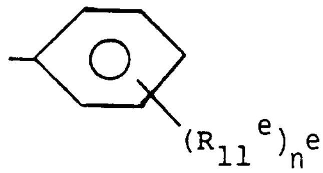

and $g^e$ is an integer from 1 to 4.

$R_2^e$ is hydrogen or lower alkyl.

$R_3^e$ is hydrogen, lower alkyl, halo substituted lower alkyl, hydroxy substituted lower alkyl, $-(CH_2)_q{}^e-NH_2$, $-(CH_2)_q{}^e$-N-(lower alkyl)$_2$, $-(CH_2)_q{}^e$-quanidinyl, $-(CH_2)_q{}^e$-SH, $-(CH_2)_q{}^e$-S-lower alkyl,

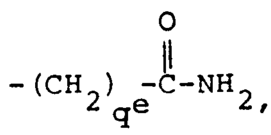

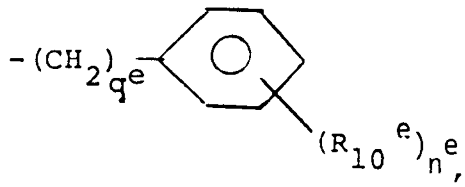

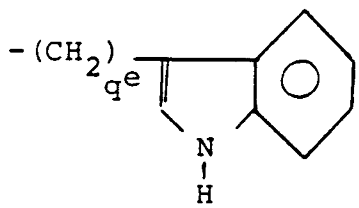

,

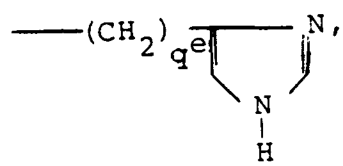

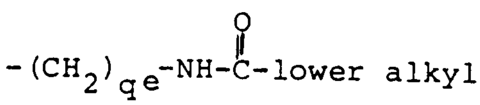

or

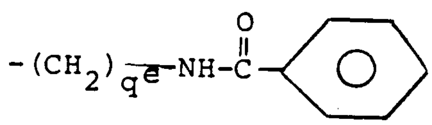

wherein $R_{10}{}^e$, $n^e$ and $q^e$ are as defined above;

(f) having the formula

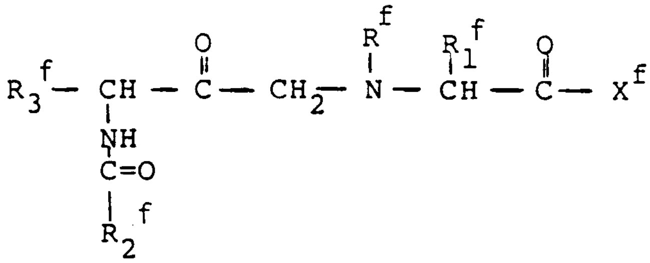

$X^f$ is an amino or imino acid of the formula

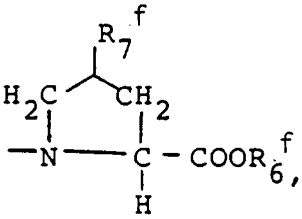

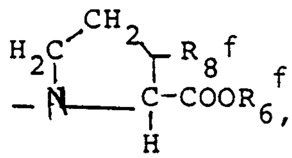

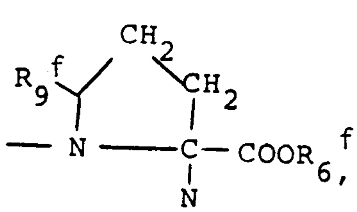

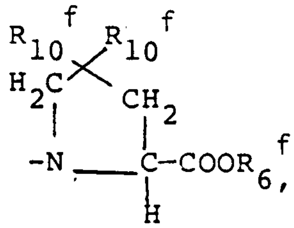

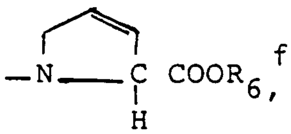

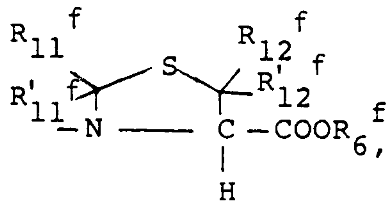

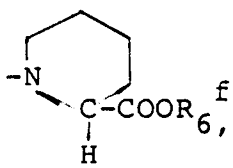

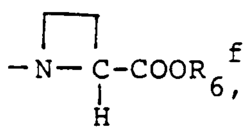

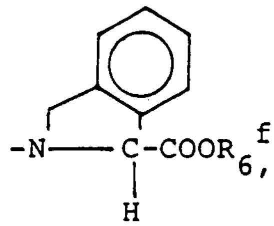

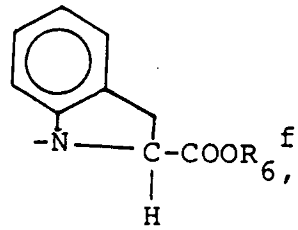

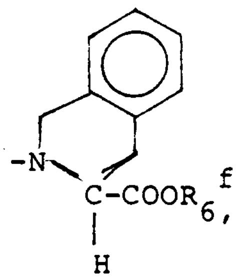

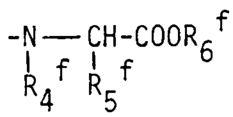

or

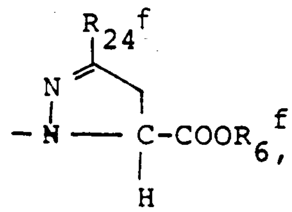

$R_7^f$ is hydrogen, lower alkyl, halogen, keto, hydroxy,

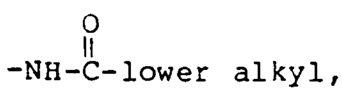

azido, amino,

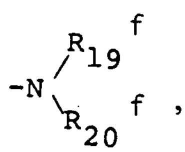

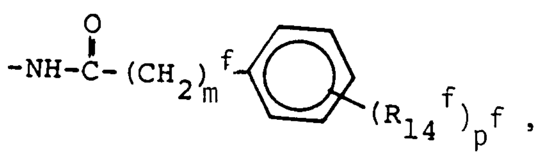

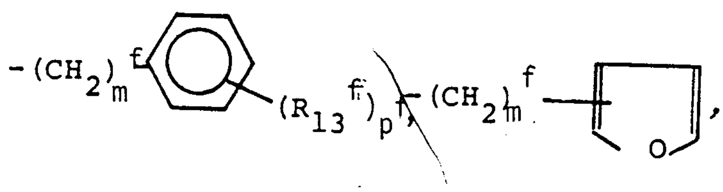

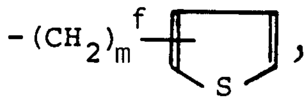

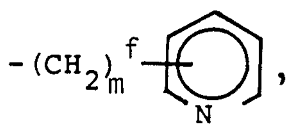

a 1- or 2-naphthyl of the formula

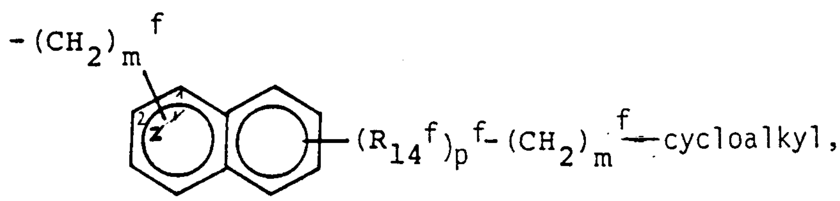

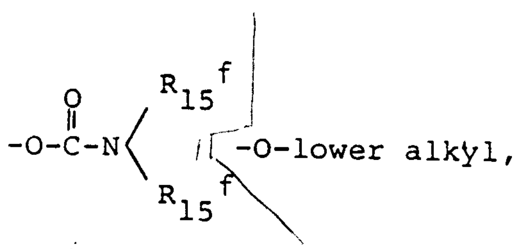

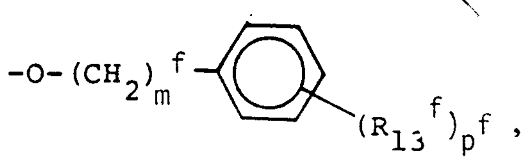

a 1- or 2-naphthyloxy of the formula

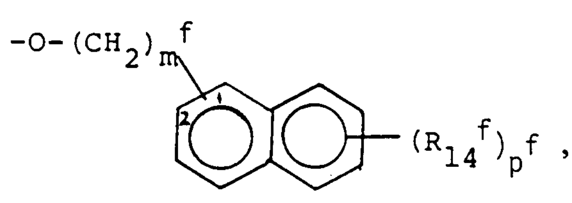

-S-lower alkyl,

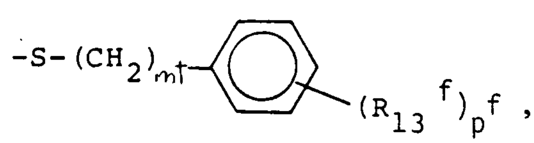

or a 1- or 2-naphthylthio of the formula

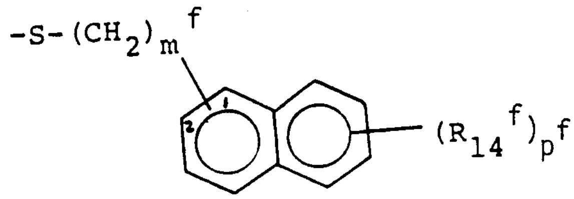

$R_8{}^f$ is keto, halogen,

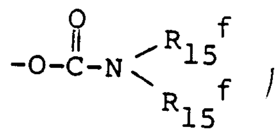

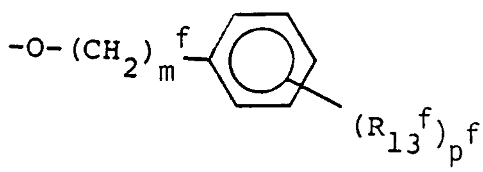

-O-lower alkyl, a 1- or 2-naphthyloxy of the formula

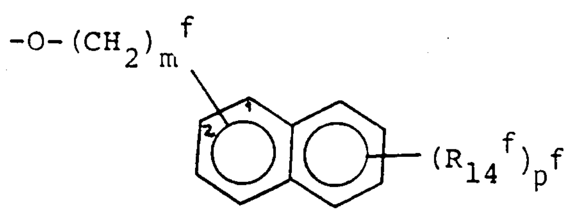

-S-lower alkyl,

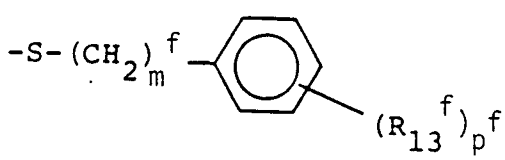

or a 1- or 2-naphthylthio of the formula

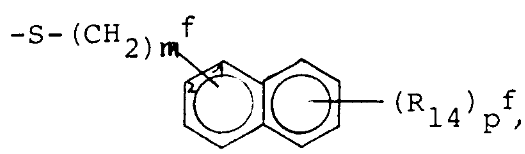

$R_9{}^f$ is keto or

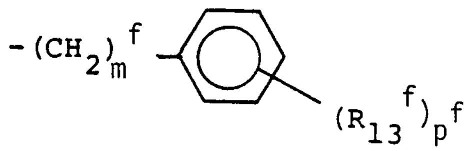

$R_{10}{}^f$ is halogen or $-Y^f-R_{16}{}^f$

$R_{11}{}^f$, $R'_{11}{}^f$, $R_{12}{}^f$ and $R'_{12}{}^f$ are independently selected from hydrogen and lower alkyl or $R'_{11}{}^f$,$R_{12}{}^f$ and $R'_{12}{}^f$ are hydrogen and $R_{11}{}^f$ is

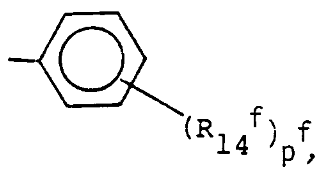

$R_{13}{}^f$ is hydrogen, lower alkyl of 1 to 4 carbon atoms, lower alkoxy of 1 to 4 carbon atoms, lower alkylthio of 1 to 4 carbon atoms, chloro, bromo, fluoro, trifluoromethyl, hydroxy, phenyl, phenoxy, phenylthio, or phenylmethyl.

$R_{14}{}^f$ is hydrogen, lower alkyl of 1 to 4 carbon atoms, lower alkoxy of 1 to 4 carbon atoms, lower alkylthio of 1 to 4 carbon atoms, chloro, bromo, fluoro, trifluoromethyl, or hydroxy.

$m^f$ is zero, one, two, three, or four.

$p^f$ is one, two or three provided that $p^f$ is more than one only if $R_{13}{}^f$ or $R_{14}{}^f$ is hydrogen, methyl, methoxy, chloro, or fluoro.

$R_{15}{}^f$ is hydrogen or lower alkyl of 1 to 4 carbon atoms.

$Y^f$ is oxygen or sulfur.

$R_{16}{}^f$ is lower alkyl of 1 to 4 carbon atoms.

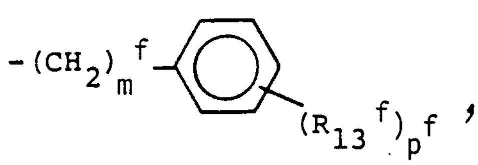

or the $R_{16}{}^f$ groups join to complete an unsubstituted 5- or 6-membered ring or said ring in which one or more of the carbon atoms has a lower alkyl of 1 to 4 carbon atoms or a di(lower alkyl of 1 to 4 carbon atoms) substituent.

$R_4{}^f$ is hydrogen, lower alkyl,

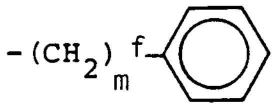, $-(CH_2)_m^f$-cycloalkyl,

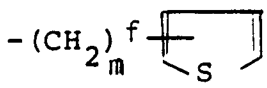, 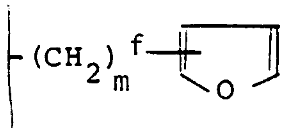,

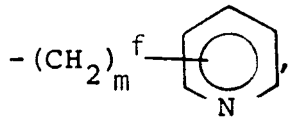, or 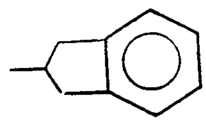

$R_5^f$ is hydrogen, lower alkyl,

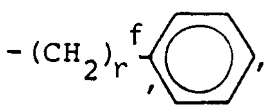, 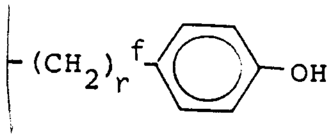,

$-(CH_2)_r^f-OH$, 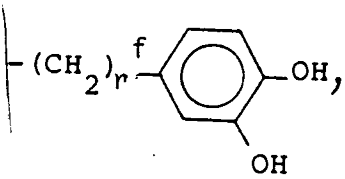,

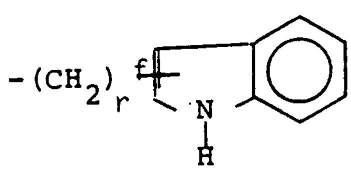, 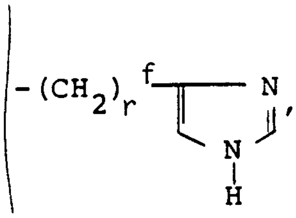,

$-(CH_2)_r^f-NH_2-(CH_2)_r^f-SH$, $-(CH_2)_r^f$-S-lower alkyl,

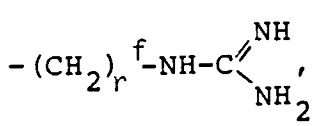, or 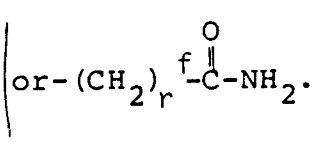.

$r^f$ is an integer from 1 to 4.

$R_{19}{}^f$ is lower alkyl, benzyl, or phenethyl.
$R_{20}{}^f$ is hydrogen, lower alkyl, benzyl or phenethyl.
$R^f$ is hydrogen, lower alkyl, cycloalkyl,

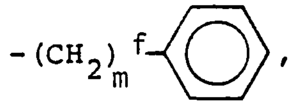,

$-(CH_2)_2-NH_2$, $-(CH_2)_3-NH_2$, $-(CH_2)_4-NH_2$, $-(CH_2)_2-OH$, $-(CH_2)_3-OH$, $-(CH_2)_4-OH$, $-(CH_2)_2-SH$, $-(CH_2)_3-SH$, or $-(CH_2)_4-SH$.
$R_1{}^f$ is hydrogen, lower alkyl, halo substituted lower alkyl,

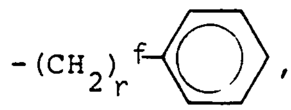, 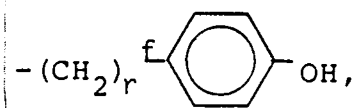,

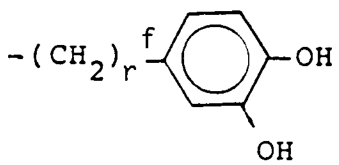, 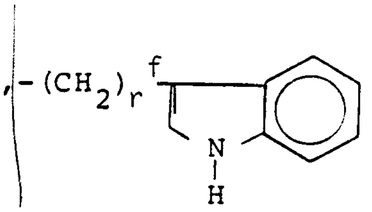,

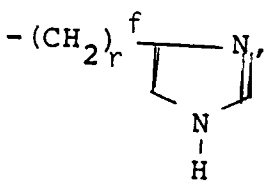, $-(CH_2)_r{}^f-NH_2$, $-(CH_2)_r{}^f-SH$,

$-(CH_2)_r{}^f-OH$, $-(CH_2)_r{}^f-S\text{-lower alkyl}$,

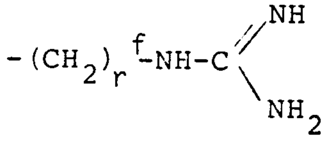, or 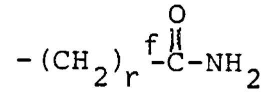

provided that $R_1{}^f$ is hydrogen only if $R^f$ is other than hydrogen.
$R_2{}^f$ is

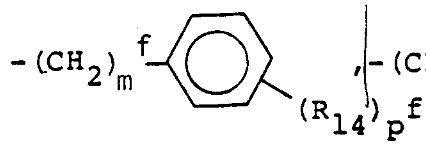, 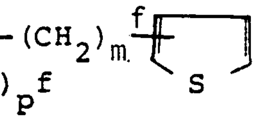,

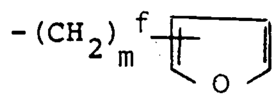, or 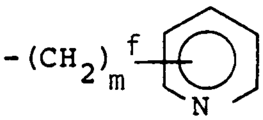

$R_3{}^f$ is hydrogen, lower alkyl,

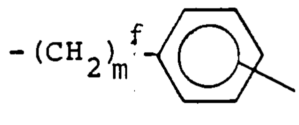, 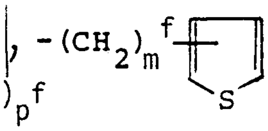,

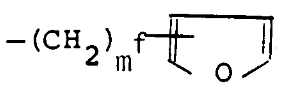, 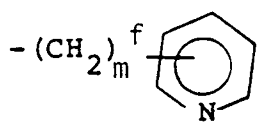, halo substituted

lower alkyl, $-(CH_2)_m{}^f$-cycloalkyl, 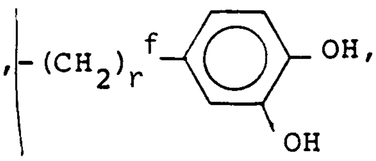

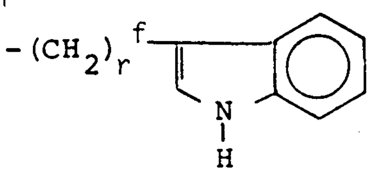, $-(CH_2)_r{}^f$-OH,

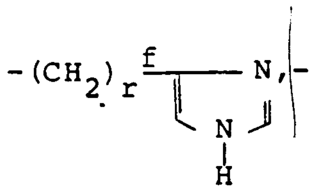, $-(CH_2)_r{}^f$-$NH_2$, $-(CH_2)_r{}^f$-SH,

$-(CH_2)_r{}^f$-S-lower alkyl, 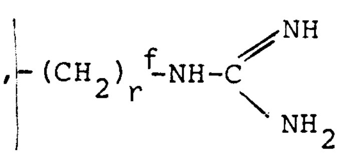, or 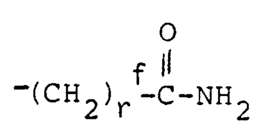

wherein $m^f$, $R_{14}{}^f$, $p^f$ and $r^f$ are as defined above.
$R_6{}^f$ is hydrogen, lower alkyl, benzyl, benzhydryl,

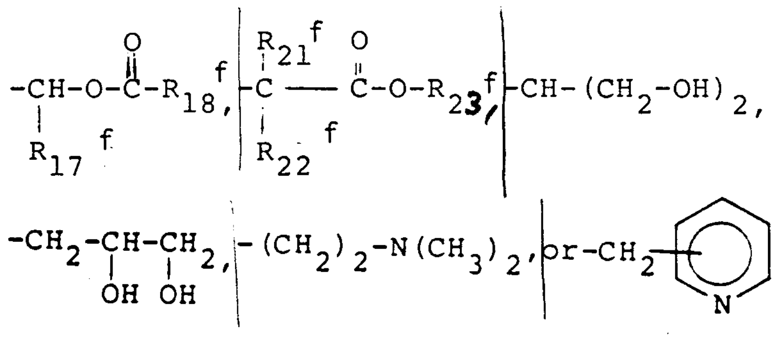

$R_{17}{}^{f}$ is hydrogen, lower alkyl, cycloalkyl, or phenyl.

$R_{18}{}^{f}$ is hydrogen, lower alkyl, lower alkoxy, or phenyl or $R_{17}{}^{f}$ and $R_{18}{}^{f}$ taken together are $-(CH_2)_2-$, $-(CH_2)_3-$, $-CH=CH-$, or

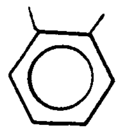,

$R_{21}{}^{f}$ and $R_{22}{}^{f}$ are independently selected from hydrogen and lower alkyl.

$R_{23}{}^{f}$ is lower alkyl.

$R_{24}{}^{f}$ is hydrogen, lower alkyl.

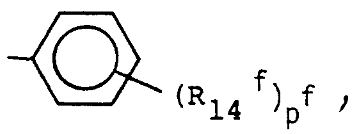

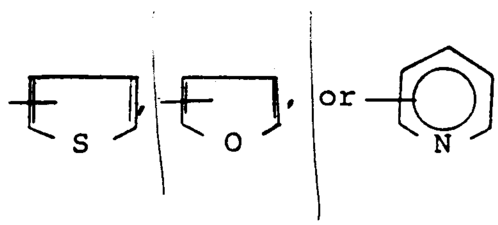

(g) having the formula

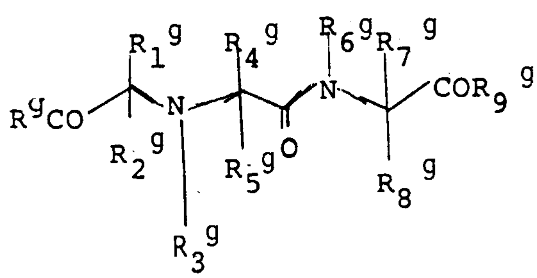

wherein either

(A) $R^g$ and $R_9{}^g$ are OH, 1-6C alkoxy, 2-6C alkenyloxy, di-(1-6C alkyl)amino-(1-6C) alkoxy, 1-6C hydroxyalkoxy, acylamino-(1-6C)alkoxy, acyloxy-(1-6C)alkoxy, aryloxy, aryloxy-(1-6C)alkoxy, $NH_2$, mono- or di-(1-6C alkyl)amino, hydroxyamino or aryl-(1-6C)alkylamino;

$R_1{}^g$-$R_5{}^g$, $R_7{}^g$ and $R_8{}^g$ are 1-20C alkyl, 2-20C alkenyl, 2-20C alkynyl, aryl, aryl-(1-6C) alkyl having 7-12C or heterocyclyl-(1-6C)alkyl having 7-12C atoms;

$R_6{}^g$ is cycloalkyl, polycycloalkyl, partly saturated cycloalkyl or polycycloalkyl, cycloalkyl-(1-6C)-alkyl having 3-20C, 6-10C aryl, aryl-(1-6C)alkyl, aryl-(2-6C)alkenyl or aryl-(2-6C) alkynyl; or

$R_2{}^g$ and $R_3{}^g$ together with the C and N atoms to which they are attached or $R_3{}^g$ and $R_5{}^g$ together with the N and C atoms to which they are attached form an N-heterocycle containing 3-5C or 2-4C and an S atom;

all alkyl, alkenyl and alkynyl are optionally substituted by OH, 1-6C alkoxy, SH, 1-6C alkylthio, $NH_2$, mono- or di(1-6C alkyl)amino, halogen or $NO_2$;

all cycloalkyl groups (including poly and partially unsaturated) are optionally substituted by halogen, 1-6C hydroxyalkyl, 1-6C alkoxy, amino-(1-6C alkyl)amino, di-(1-6C alkyl)amino, SH, 1-6C alkylthio, $NO_2$ or $CF_3$; and

aryl groups are optionally substituted by OH, 1-6C alkoxy, $NH_2$, mono- or di-(1-6C alkyl) amino, SH, 1-6C alkylthio, 1-6C hydroxyalkyl, 1-6C aminoalkyl, 1-6C thioalkyl, $NO_2$, halogen, $CF_3$, $OCH_2O$, ureido or guanidino; or

(B) $R^g$ and $R_9{}^g$ are H or 1-6C alkoxy;

$R_1{}^g$ and $R_2{}^g$ are H, 1-6C alkyl, aryl-(1-6C) alkyl having 7-12C atoms or heterocyclyl-(1-6C) alkyl having 6-12C atoms;

$R_3{}^g$-$R^5{}^g$, $R_7{}^g$ and $R_8{}^g$ are H or 1-6C alkyl;

$R_6{}^g$ is cycloalkyl, polycycloalkyl, partly saturated cycloalkyl or polycycloalkyl, cycloalkyl-(1-6C) alkyl having 3-20C, atoms aryl or aryl-(1-6C) alkyl; and

aryl has 6-10C and is optionally substituted by 1-6C alkyl, 2-6C alkenyl, 2-6C alkynyl, OH, 1-6C alkoxy, $NH_2$, mono- or di-(1-6C alkyl) amino, SH, 1-6C alkylthio, 1-6C hydroxyalkyl, 1-6C aminoalkyl, 1-6C thioalkyl, $NO_2$, halogen, $CF_3$, $OCH_2O$, ureido or guanidino;

(h) having the formula

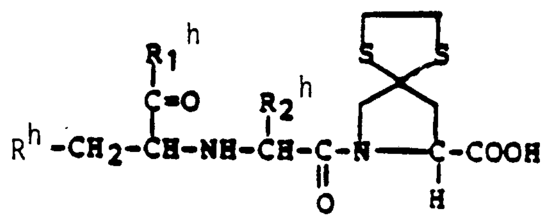

wherein:

$R^h$ is lower alkyl, benzyl, benzylthio, benzyloxy, phenylthio or phenoxy;

$R_1{}^h$ is hydroxy or lower alkoxy;

$R_2{}^h$ is hydrogen, lower alkyl or amino lower alkyl; and the pharmaceutcally acceptable salts thereof;

(i) having the formula

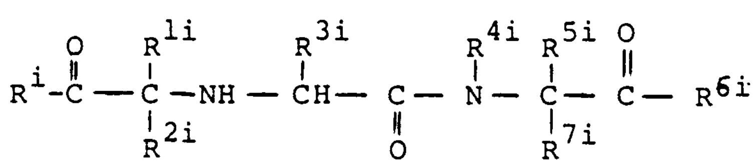

or a pharmaceutically acceptable salt thereof, wherein $R^i$ and $R^{6i}$ are the same or different and are hydroxy, lower alkoxy, lower alkenyloxy, dilower alkylamino lower alkoxy, acylamino lower alkoxy, acyloxy lower alkoxy, aryloxy, aryllower alkoxy, amino, lower alkylamino, dilower alkylamino, hydroxyamino, aryllower alkylamino, or substituted aryloxy or substituted aryllower alkoxy wherein the substituent is methyl, halo or methoxy; $R^{1i}$ is hydrogen, alkyl of from 1 to 10 carbon atoms, substituted lower alkyl wherein the substituent is hydroxy, lower alkoxy, aryloxy, substituted aryloxy, heteroaryloxy, substituted heteroaryloxy, amino, lower alkylamino, diloweralkylamino, acylamino,

arylamino, substituted arylamino, guanidino, imidazolyl, indolyl, lower alkylthio, arylthio, substituted arylthio, carboxy, carbamoyl, lower alkoxy carbonyl, aryl, substituted aryl, aralkyloxy, substituted aralkyloxy, aralkylthio or substituted aralkylthio, wherein the aryl or heteroaryl portion of said substituted aryloxy, heteroaryloxy, arylamino, arylthio, aryl, aralkyloxy, aralkylthio group is substituted with a group selected from halo, lower alkyl, hydroxy, lower alkoxy, amino, aminomethyl, carboxyl, cyano, or sulfamoyl; $R^{2i}$ and $R^{7i}$ are the same or different and are hydrogen or lower alkyl; $R^{3i}$ is hydrogen, lower alkyl, phenyl lower alkyl, aminomethylphenyl lower alkyl, hydroxyphenyl lower alkyl, hydroxy lower alkyl, acylamino lower alkyl, amino lower alkyl, dimethylamino lower alkyl, guanidino lower alkyl, imidazolyl lower alkyl, indolyl lower alkyl, or lower alkyl thio lower alkyl; $R^{4i}$ and $R^{5i}$ are the same or different and are hydrogen, lower alkyl or $Z^i$, or $R^{4i}$ and $R^{5i}$ taken together form a group represented by $Q^i$, $U^i$, $V^i$, $Y^i$, $D^i$ or $E^i$, wherein

$Z^i$ is

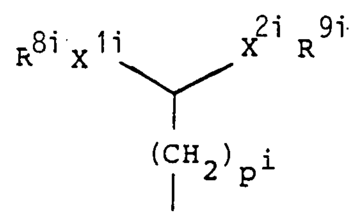

wherein $X^{1i}$ and $X^{2i}$ independent of each other are O, S or $CH_2$, $R^{8i}$ and $R^{9i}$ independent of each other are lower alkyl, lower alkenyl, lower alkynyl, cycloalkyl having 3 to 8 carbon atoms, hydroxy lower alkyl or $-(CH_2)_nAr^i$, wherein $n^i$ is 0, 1, 2 or 3 and $Ar^i$ is unsubstituted or substituted phenyl, furyl, thienyl or pyridyl, wherein said substituted phenyl, furyl, thienyl or pyridyl groups are substituted with at least one group that is independently selected from $C_1$ to $C_4$ alkyl, lower alkoxy, lower alkylthio, halo, $CF_3$ and hydroxy, or $R^{8i}$ and $R^{9i}$ taken together form a bridge $W^i$, wherein $W^i$ is a single bond or a methylene bridge or a substituted methylene bridge when at least one of $X^{1i}$ and $X^{2i}$ is methylene, or $W^i$ is an alkylene or substituted alkylene bridge having 2 or 3 carbon atoms, said substituted methylene bridge or said substituted alkylene bridge having one or two substituents selected from lower alkyl, aryl and aryl lower alkyl groups, and $p^i$ is 0, 1 or 2; with the proviso that at least one of $R^4$ and $R^5$ is $Z^i$, with the proviso that if $R^4$ is $Z^i$ and $p^i$ is 0 then $X^{1i}$ and $X^{2i}$ must both be methylene, and with the proviso that if $X^{1i}$ and $X^{2i}$ are both methylene then $R^{8i}$ and $R^{9i}$ must form an alkylene bridge $W^i$;

$Q^i$ is

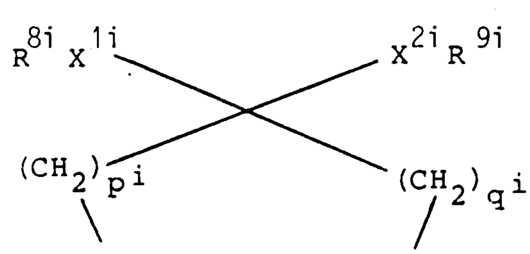

wherein $R^{8i}$, $R^{9i}$, $X^{1i}$ and $X^{2i}$ are as defined above, $p^i$ is 0, 1 or 2, $q^i$ is 0, 1 or 2, with the proviso that the sum of $p^i$ and $q^i$ must be 1, 2 or 3, with the proviso that if $p^i$ is 0 then $X^{1i}$ and $X^{2i}$ must be methylene, and with the proviso that if $X^1$ and $X^{2i}$ are methylene then $R^{8i}$ and $R^{9i}$ taken together form a bridge $W^i$, wherein $W^i$ is as defined above;

$V^i$ is

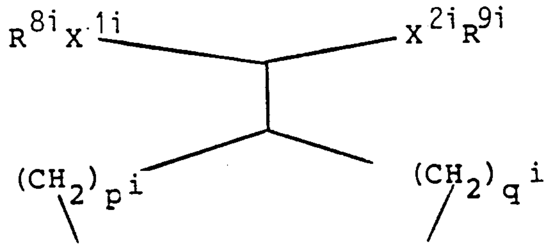

wherein $R^{8i}$, $R^{9i}$, $X^{1i}$ and $X^{2i}$ are as defined above, $p^i$ is 0, 1 or 2 and $q^i$ is 0, 1 or 2, with the proviso that the sum of $p^i$ and $q^i$ is 1, 2 or 3, with the proviso that if $X^{1i}$ and $X^{2i}$ are $CH_2$ then $R^{8i}$ and $R^{9i}$ taken together form a bridge $W^i$, wherein $W^i$ is as defined above;

$U^i$ is

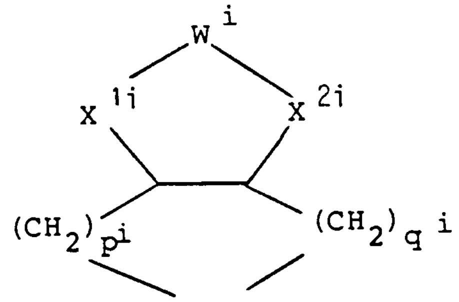

wherein $W^i$ is as defined above (except that $W^i$ may also be a methylene bridge when $X^{1i}$ and $X^{2i}$ are oxygen or sulfur), $X^{1i}$ and $X^{2i}$ are as defined above, $p^i$ is 0, 1 or i is 0, 1 or 2, with the proviso that the sum of $p^i$ and $q^i$ is 1 or 2, and with the proviso that if $p^i$ is 0, $X^{1i}$ must be $CH_2$;

$Y^i$ is

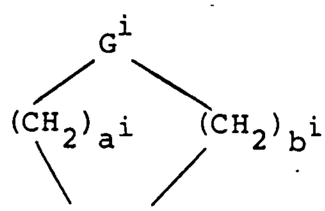

wherein $G^i$ is oxygen, sulfur or $CH_2$, $a^i$ is 2, 3 or 4 and $b^i$ is 1, 2, 3, 4 or 5, with the proviso that the sum of $a^i$ and $b^i$ is 5, 6 or 7 or

$G^i$ is $CH_2$, $a^i$ is 0, 1, 2 or 3, $b^i$ is 0, 1, 2 or 3 with the proviso that the sum of $a^i$ and $b^i$ is 1, 2 or 3, with the proviso that the sum of $a^i$ and $b^i$ may be 1, 2 or 3 only if $R^{1i}$ is lower alkyl substituted with aralkylthio or aralkyloxy;

$D^i$ is

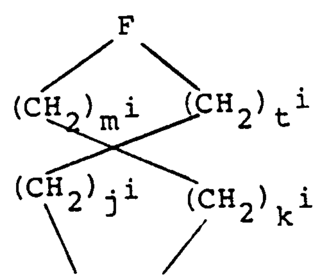

wherein $F^i$ is O or S, $j^i$ is 0, 1 or 2 and $k^i$ is 0, 1 or 2, with the proviso that the sum of $j^i$ and $k^i$ must be 1, 2 or 3, and $m^i$ is 1, 2 or 3 and $t^i$ is 1, 2 or 3, with the proviso that the sum of $m^i$ and $t^i$ must be 2, 3 or 4;
$E^i$ is

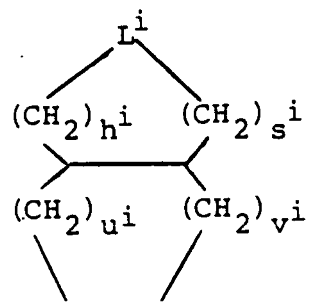

wherein $L^i$ is O or S, $u^i$ is 0, 1 or 2 and $v^i$ is 0, 1 or 2, with the proviso that the sum of $u^i$ and $v^i$ must be 1 or 2, and $h^i$ is 1 or 2 and $s^i$ is 1 or 2, with the proviso that the sum of $h^i$ and $s^i$ must be 2 or 3.
(j) having the formula

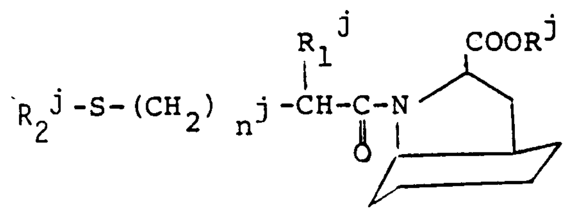

wherein $R^j$ is hydrogen or lower alkyl; $R_1{}^j$ is hydrogen,
lower alkyl, or benzyl; $R_2{}^j$ is hydrogen or

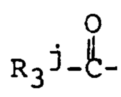

wherein $R_3{}^j$ is lower alkyl, heteroaryl containing 4 to 9 carbon atoms and one or two nitrogen, oxygen or sulfur atoms; phenyl, substituted phenyl having 1 or 2 substituents selected from fluorine, chlorine,
bromine, lower alkyl or alkoxy; and $n^j$ is 0 or 1; wherein lower alkyl and lower alkoxy include straight or branched groups containing 1 to 4 carbon atoms, and
pharmaceutically acceptable salts of the compounds when $R^j$ is hydrogen and when $R_3{}^j$ is heteroaryl containing 1 or 2 nitrogen atoms, are disclosed;

(k) having the formula

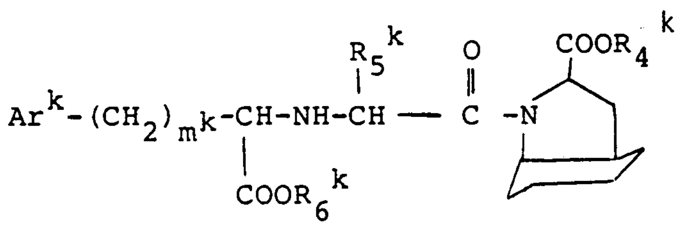

wherein $R_4{}^k$ is hydrogen or lower alkyl; $R_5{}^k$ is hydrogen, lower alkyl or benzyl; $R_6{}^k$ is hydrogen or lower alkyl;

$Ar^k$ is phenyl or phenyl substituted with 1 or 2 substituents selected from fluorine, chlorine, bromine, lower alkyl, lower alkoxy, hydroxy or amino; and m is 0 to 3; wherein lower alkyl and lower alkoxy contain 1 to 4 straight or branched carbon atoms; and the pharmaceutically acceptable salts thereof; (l) having the formulae

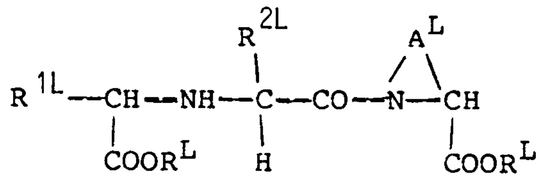

$(I)^L$

and

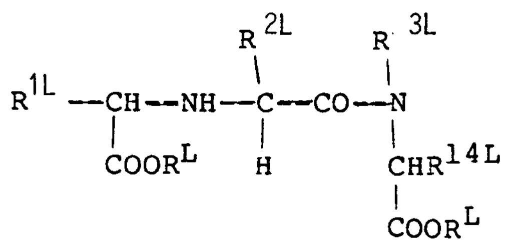

$(Ia)^L$

wherein

$R^L$ and $R^{2L}$ are independently hydrogen; loweralkyl; aralkyl; or aryl;

$R^{1L}$ is hydrogen; branched or straight chain $C_{1-12}$ alkyl and alkenyl; $C_3$-$C_9$ cycloalkyl and benzofused alkyl; substituted loweralkyl where the substituents are halo, hydroxy loweralkoxy, aryloxy, amino, mono- or diloweralkylamino, acylamino, arylamino, guanidino, mercapto, loweralkylthio, arylthio, carboxy, carboxamido, or loweralkoxycarbonyl; aryl; substituted aryl where the substituents are loweralkyl, loweralkoxy, or halo; arloweralkyl; arloweralkenyl; heteroarloweralkyl; heteroarloweralkenyl; substituted arloweralkyl, substituted arloweralkenyl, substituted heteroarloweralkyl, or substituted heteroarloweralkenyl where the aryl and heteroaryl substituents are halo, dihalo, loweralkyl, hydroxy, loweralkoxy, amino, aminoloweralklyl, acylamino, mono- or diloweralkylamino, carboxyl, haloloweralkyl, nitro, cyano, or sulfonamido, and where the loweralkyl portion of arloweralkyl may be substituted by amino, acylamino, or hydroxyl;

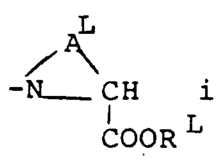

is

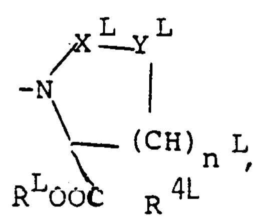

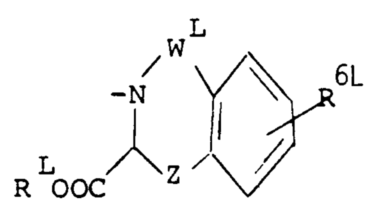

or

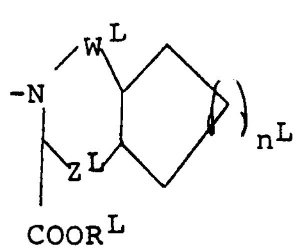

where

$X^L$ and $Y^L$ taken together are $-CH_2-CH_2-$;

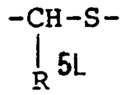; 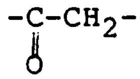; 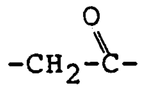; 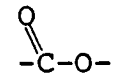; 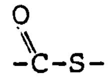;

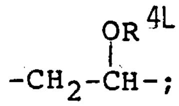; 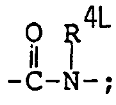; or 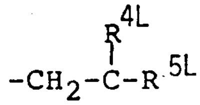;

$R^{4L}$ is hydrogen, loweralkyl; aryl; substituted aryl;
$R^{5L}$ is hydrogen; loweralkyl; aryl or substituted aryl;
$n^L$ is 1 to 3;
$w^L$ is absent; $-CH_2$; or

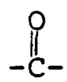;

$z^L$ is $-(CH_2)_m{}^L$, where $m^L$ is 0 to 2, provided that $m^L$ may not be O and $W^L$ may not be absent at the same time; and
$R^{6L}$ is hydrogen; loweralkyl; halo; or $OR^{4L}$;
$R^{2L}$ is $—(CH_2)_r{}^L—B^L—(CH_2)_s{}^L—NR^{7L}R^{15L}$
where
$r^L$ and $s^L$ are independently 0 to 3;
$B^L$ is absent; -O-; -S-; or $-NR^{8L}$;
where $R^{8L}$ is hydrogen; loweralkyl; alkanoyl; or aroyl; and
$R^{7L}$ is

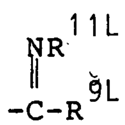; 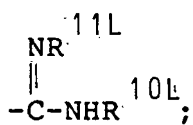; or 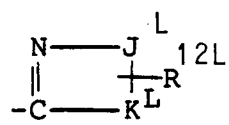

where
$R^{9L}$ is loweralkyl; aralkyl; aryl; heteroaryl; or heteroarloweralkyl and these groups are substituted by hydroxy, lower alkoxy or halo; carboxyl; carboxamido; nitromethenyl.
$R^{10L}$ is hydrogen; loweralkyl; aryl; or amidino;
$R^{11L}$ hydrogen; loweralkyl; cyano; amidino; aryl; aroyl; loweralkanoyl;

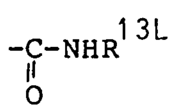;

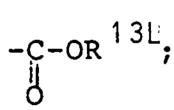;

$-NO_2$; $-SO_2NH_2$;
or
$SO_2R^{13L}$;
$R^{12L}$ is hydrogen; loweralkyl; halo; aralkyl; amino; cyano; mono- or diloweralkylamino; or $OR^{4L}$;
$R^{13L}$ is hydrogen; loweralkyl; or aryl;
$R^{15L}$ is hydrogen; lower alkyl; aralkyl; or aryl.

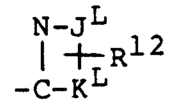

constitutes a basic heterocycle of 5 or 6 atoms or benzofused analogs thereof and optionally containing 1-3 N atoms, an oxygen-, a sulfur atom, an SO- or an $SO_2$-group optionally substituted by amino, lower alkyl amino, diloweralkyl amino, lower alkoxy, or aralkyl groups;
$R^{3L}$ is $C_{3-8}$ cycloalkyl and benzofused $C_{3-8}$ cycloalkyl; perhydrobenzofused $C_{3-8}$ cycloalkyl; aryl; substituted aryl; heteroaryl; substituted heteroaryl;
$R^{14L}$ is hydrogen or loweralkyl; and, a pharmaceutically acceptable salt thereof;
(m) having the formula

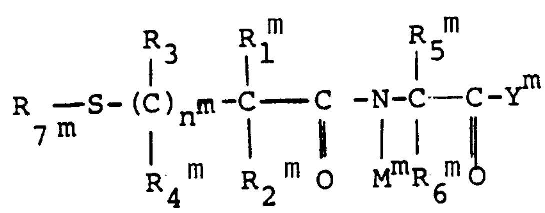

wherein
$R_1{}^m$, $R_2{}^m$, $R_3{}^m$, $R_4{}^m$, $R_5{}^m$ and $R_6{}^m$ are hydrogen, alkyl, alkenyl, alkynyl, phenyl-alkyl, and cycloalkyl, and may be the same or different,
$n^m$ is an integer from 0 to 4 inclusive,
$M^m$ is alkenyl, alkynyl, cycloalkyl, cycloalkyl-alkyl, polycycloalkyl, polycycloalkyl-alkyl, aryl, aralkyl, heteroaryl, heteroaryl-alkyl, hetero-cycloalkyl, hetero-cycloalkyl-alkyl, fused aryl-cycloalkyl, fused aryl-cycloalkyl-alkyl, fused heteroaryl-cycloalkyl, fused heteroaryl-cycloalkyl-alkyl, alkoxyalkyl, alkylthioalkyl, alkylamino-alkyl, or dialkylaminoalkyl,
$Y^m$ is hydroxy, alkoxy, amino, or substituted amino, aminoalkanoyl, aryloxy, aminoalkoxy, or hydroxyalkoxy, and
$R_7{}^m$ is hydrogen, alkanoyl, carboxyalkanoyl, hydroxyalkanoyl, aminoalkanoyl, cyano, amidino, carbalkoxy, $Z^mS$ or

$Z^mSCO$-

wherein $Z^m$ is hydrogen, alkyl, hydroxyalkyl, or the radical

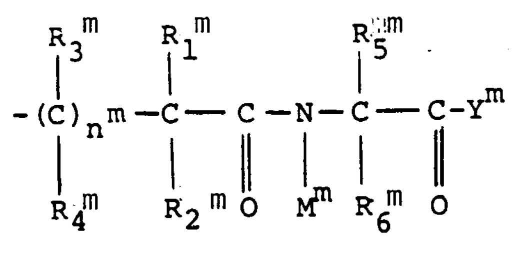

wherein $R_1{}^m$, $R_2{}^m$, $R_3{}^m$, $R_4{}^m$, $R_5{}^m$, $R_6{}^m$ $n^m$, $M^m$ and $Y^m$ are as described above; and where $Y^m$ is hydroxy, their non-toxic, pharmaceutically acceptable alkali metal, alkaline earth metal, and amine

salts; or

(n) having the formula

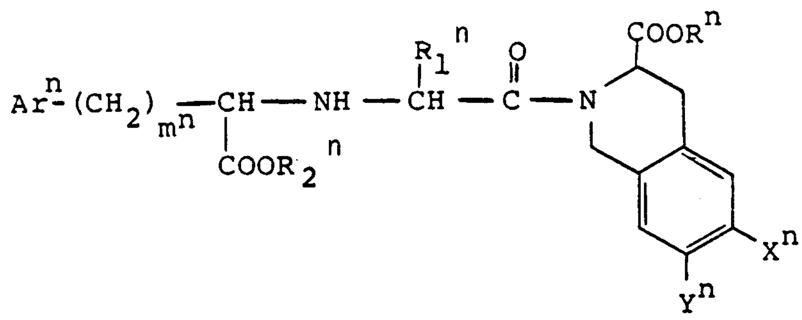

wherein $R^n$ is hydrogen, lower alkyl or aralkyl;

$R_1{}^n$ is hydrogen, lower alkyl, or benzyl; $R_2{}^n$ is hydrogen or lower alkyl, and Ar is phenyl or phenyl substituted with 1 or 2 substituents selected from fluorine, chlorine, bromine, lower alkyl, lower alkoxy, hydroxy or amino; X and Y are independently hydrogen, lower alkyl, lower alkoxy, lower alkylthio, lower alkylsulfinyl, lower alkylsulfonyl, hydroxy, or X and Y together are methylenedioxy; m is 0 to 3; and the pharmaceutically acceptable acid salts thereof;

preferably having the formula as defined in paragraph (i) especially being

7-[N-(1(S)-carboxy-3-phenylpropyl)-(S)-alanyl]-1,4-dithia-7-azaspiro[4.4]nonane-8(S)-carboxylic acid.

(o) having the formula

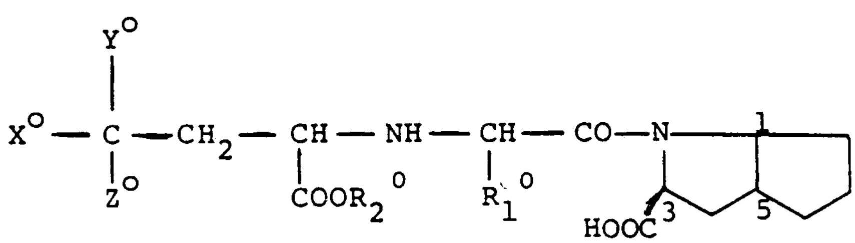

the hydrogen atoms at ring positions 1 and 5 are cis to each other and the 3-carboxy group has the endo orientation;

$R_1{}^o$ is H, allyl, vinyl or the side chain of an optionally protected naturally occurring $\alpha$-amino acid;

$R_2{}^o$ is H, 1-6C alkyl, 2-6C alkenyl or aryl(1-4C alkyl);

$Y^o$ is H or OH and $Z^o$ is H, or $Y^o$ and $Z^o$ together oxygen;

$X^o$ is 1-6C alkyl, 2-6C alkenyl, 5-9C cycloalkyl, 6-12C aryl (optionally substituted by one to three 1-4C alkyl or alkoxy, OH, halo, nitro, amino (optionally substituted by one or two 1-4C alkyl), or methylenedioxy or indol-3-yl;

(p) having the formula

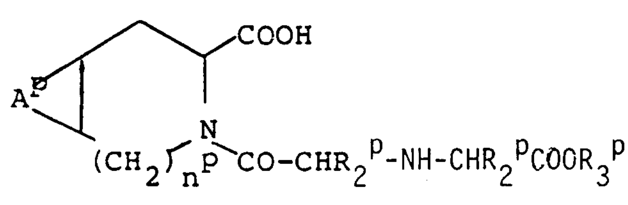

$n^p$ is 0 or 1;

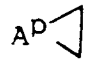

is a benzene or cyclohexane ring:

$R_1{}^p$ and $R_2{}^p$ are each 1-6C alkyl, 2-6C alkenyl, 5-7C cycloalkyl, 5-7C cycloalkenyl, 7-12C cycloalkylalkyl, optionally partially hydrogenated 6-10C aryl, 7-14C aralkyl or 5-7 membered monocyclic or 8-10 membered bicyclic heterocyclyl containing 1 or 2 S or O and/or 1-4N atoms; all $R_1{}^p$ and $R_2{}^p$ groups are optionally substituted.

$R_3{}^p$ is H, 1-6C alkyl, 2-6C alkenyl or 7-14C aralkyl;

**3.** The composition according to claim 1 or 2 wherein the beta adrenergic blocker is atenolol, metoprolol, nadolol, pindolol, propranolol, timolol, labetalol, betaxolol, carteolol, dilevolol or butonolol, preferably timolol.

**4.** The composition according to any one of claims 1 to 3 wherein for an amount of from 0.005µg to 50µg of the ACE inhibitor there is present an amount of from 5µg to 500µg of the beta adrenergic blocker, whereby the following proportions are preferred

(a) 50µg to 250µg (especially 50µg to 125µg) of the beta adrenergic blocker to 0.025µg to 5µg (especially 0.05µg to 0.5µg) of the ACE inhibitor;

(b) 5µg to 125µg (especially 50µg) of the beta adrenergic blocker to 0.005µg to 1µg (especially 0.05µg to 0.5µg) of the ACE inhibitor; and

(c) 25µg to 500µg (especially 50µg to 250µg) of the beta adrenergic blocker to 0.005µg to 1µg (especially 0.005µg to 0.05µg) of the ACE inhibitor.

**5.** A kit comprising in separate containers pharmaceutical compositions for combination therapy to reduce and/or control the elevated intraocular pressure associated with glaucoma in mammals which comprises in the one container a topical ophthalmological pharmaceutical composition comprising an ophthalmologically acceptable angiotensin converting enzyme inhibitor and in the second container, a topical ophthalmological pharmaceutical composition comprising an ophthalmologically acceptable beta adrenergic blocker.

**6.** The kit according to claim 5 wherein the ACE inhibitor is an ACE inhibitor as defined in claim 2, preferably
[7-N-(1(S)-carboxy-3-phenylpropyl)-(S)-alanyl]-1,4-dithia-7-azaspiro[4.4]nonane-8(S)-carboxylic acid.

**7.** The kit according to claim 5 or 6 wherein the beta adrenergic blocker is atenolol, metoprolol, nadolol, pindolol, propranolol, timolol, labetalol, betaxolol, carteolol, dilevolol or bunolol, preferably timolol.

**8.** The kit according to any one of claims 5 to 7 wherein for an amount of from 0.005µg to 50µg of the ACE inhibitor there is present an amount of from 5µg to 500µg of the beta adrenergic blocker, whereby the following proportions are preferred

(a) 50µg to 250µg (especially 50µg to 125µg) of the beta adrenergic blocker to 0.025µg to 5µg (especially 0.05µg to 0.5µg) of the ACE inhibitor;

(b) 5µg to 125µg (especially 50µg) of the beta adrenergic blocker to 0.005µg to 1µg (especially 0.05µg to 0.5µg) of the ACE inhibitor; and

(c) 25µg to 500µg (especially 50µg to 250µg) of the beta adrenergic blocker to 0.005µg to 1µg (especially 0.005µg to 0.05µg) of the ACE inhibitor.

**9.** A kit comprising (a) a topical ophthalmological pharmaceutical composition comprising an ophthalmologically acceptable angiotensin converting enzyme inhibitor and (b) a topical ophthalmological pharmaceutical composition comprising an ophthalmologically acceptable beta adrenergic blocker, together with (c) instructions for using the compositions for combination therapy to reduce and/or control the elevated intraocular pressure associated with glaucoma in mammals.

**10.** The kit according to claim 9 wherein the ACE inhibitor is an ACE inhibitor as defined in claim 2, preferably
[7-N-(1(S)-carboxy-3-phenylpropyl)-(S)-alanyl]-1,4-dithia-7-azaspiro[4.4]nonane-8(S)-carboxylic acid.

**11.** The kit according to claim 9 or 10 wherein the beta adrenergic blocker is atenolol, metoprolol, nadolol, pindolol, propranolol, timolol, labetalol, betaxolol, carteolol, dilevolol or bunolol, preferably timolol.

**12.** The kit according to any one of claims 9 to 11 wherein for an amount of from 0.005µg to 50µg of the ACE inhibitor there is present an amount of from 5µg to 500µg of the beta adrenergic blocker, whereby the following proportions are preferred

(a) 50µg to 250µg (especially 50µg to 125µg) of the beta adrenergic blocker to 0.025µg to 5µg (especially 0.05µg to 0.5µg) of the ACE inhibitor;

(b) 5µg to 125µg (especially 50µg) of the beta adrenergic blocker to 0.005µg to 1µg (especially 0.05µg to 0.5µg) of the ACE inhibitor; and

(c) 25µg to 500µg (especially 50µg to 250µg) of the beta adrenergic blocker to 0.005µg to 1µg (especially 0.005µg to 0.05µg) of the ACE inhibitor.

**13.** The use of an ophthalmologically acceptable ACE inhibitor and an ophthalmologically acceptable beta adrenergic blocker for the preparation of medicaments for combination therapy to reduce and/or control the elevated intraocular pressure associated with glaucoma.

**14.** The use according to claim 13 wherein the ACE inhibitor used is an ACE inhibitor as defined in claim 2, preferably
[7-N-(1(S)-carboxy-3-phenylpropyl)-(S)-alanyl]-1,4-dithia-7-azaspiro[4.4]nonane-8(S)-carboxylic acid.

**15.** The use according to claim 13 or 14 wherein the beta adrenergic blocker is atenolol, metoprolol, nadolol, pindolol, propranolol, timolol, labetalol, betaxolol, carteolol, dilevolol or bunolol, preferably timolol.

**16.** The use according to any one of claims 13 to 15 wherein for an amount of from 0.005µg to 50µg of the ACE inhibitor there is used an amount of from 5µg to 500µg of the beta adrenergic blocker, whereby the following proportions are preferred

(a) 50µg to 250µg (especially 50µg to 125µg) of the beta adrenergic blocker to 0.025µg to 5µg (especially 0.05µg to 0.5µg) of the ACE inhibitor;

(b) 5µg to 125µg (especially 50µg) of the beta adrenergic blocker to 0.005µg to 1µg (especially 0.05µg to 0.5µg) of the ACE inhibitor; and

(c) 25µg to 500µg (especially 50µg to 250µg) of the beta adrenergic blocker to 0.005µg to 1µg (especially 0.005µg to 0.05µg) of the ACE inhibitor.

**17.** Process for the preparation of a topical ophthalmologically acceptable composition useful for reducing and/or controlling the elevated intraocular pressure associated with glaucoma which comprises mixing an ophthalmologically acceptable angiotensin converting enzyme inhibitor and an ophthalmologically acceptable beta adrenergic blocker with an ophthalmologically acceptable carrier for topical use.

**18.** The process according to claim 17 wherein the ACE inhibitor is an ACE inhibitor as defined in claim 2, preferably
[7-N-(1(S)-carboxy-3-phenylpropyl)-(S)-alanyl]-1,4-dithia-7-azaspiro[4.4]nonane-8(S)-carboxylic acid.

**19.** The process according to claim 17 or 18 wherein the beta adrenergic blocker is atenolol, metoprolol, nadolol, pindolol, propranolol, timolol, labetalol, betaxolol, carteolol, dilevolol or bunolol, preferably timolol.

**20.** The process according to any one of claims 17 to 19 wherein for an amount of from 0.005µg to 50µg of the ACE inhibitor there is present an amount of from 5µg to 500µg of the beta adrenergic blocker, whereby the following proportions are preferred

(a) 50µg to 250µg (especially 50µg to 125µg) of the beta adrenergic blocker to 0.025µg to 5µg (especially 0.05µg to 0.5µg) of the ACE inhibitor;

(b) 5µg to 125µg (especially 50µg) of the beta adrenergic blocker to 0.005µg to 1µg (especially 0.05µg to 0.5µg) of the ACE inhibitor; and

(c) 25µg to 500µg (especially 50µg to 250µg) of the beta adrenergic blocker to 0.005µg to 1µg (especially 0.005µg to 0.05µg) of the ACE inhibitor.

## Patentansprüche

1. Topische, ophthalmologisch annehmbare Zusammensetzung, brauchbar zur Herabsetzung und/oder Kontrolle des mit Glaukom verbundenen erhöhten Intraokulardrucks, umfassend einen ophthalmologisch annehmbaren Inhibitor für Angiotensin umwandelndes Enzym und einen ophthalmologisch annehmbaren $\beta$-adrenergen Blocker in Kombination mit einem ophthalmologisch annehmbaren Träger zur topischen Anwendung.

2. Zusammensetzung nach Anspruch 1, umfassend einen Inhibitor für Angiotensin umwandelndes Enzym mit der Formel
   (a)

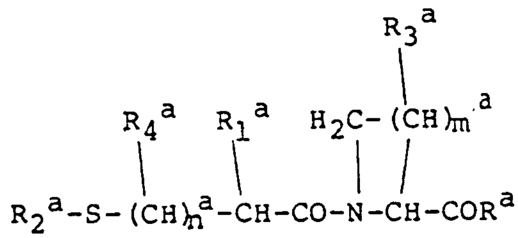

worin
$R^a$ Hydroxy, $NH_2$ oder Niederalkoxy ist;
$R_1{}^a$ und $R_4{}^a$ jeweils Wasserstoff, Niederalkyl, Phenyl oder Phenyl-Niederalkyl sind;
$R_2{}^a$ Wasserstoff, Niederalkyl, Phenyl, substituiertes Phenyl ist, wobei der Phenyl-Substituent Halogen, Niederalkyl oder Niederalkoxy, Phenyl-Niederalkyl, Diphenyl-Niederalkyl, Triphenyl-Niederalkyl, Niederalkylthiomethyl, Phenyl-Niederalkylthiomethyl, Niederalkanoylamidomethyl,
$R_5{}^a$

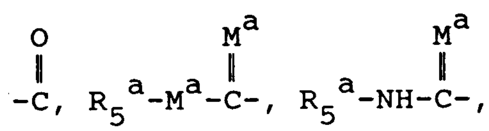

$R_6{}^a$-S- oder $R_7{}^a$ ist;
$R_3{}^a$ Wasserstoff, Hydroxy oder Niederalkyl ist;
$R_5{}^a$ Niederalkyl, Phenyl oder Phenyl-Niederalkyl ist;
$R_6{}^a$ Niederalkyl, Phenyl, substituiertes Phenyl ist (wobei der Phenyl-Substituent Halogen, Niederalkyl oder Niederalkoxy ist), Hydroxy-Niederalkyl oder Amino-(carboxy)-Niederalkyl ist; $R_7{}^a$

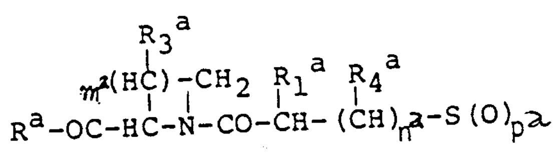

ist;
$M^a$ O oder S ist;
$m^a$ 1 bis 3 ist;
$n^a$ und $p^a$ jeweils 0 bis 2 sind:
(b) mit der Formel

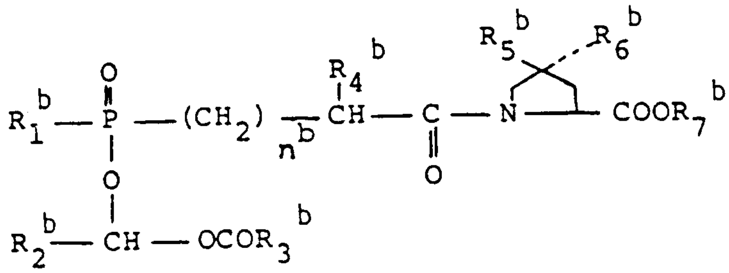

$R_1{}^b$ ist Alkyl, Aryl, Aralkyl, Cycloalkyl oder Cycloalkylalkyl;
$R_2{}^b$ ist Cycloalkyl, 3-Cyclohexenyl oder 2-Alkyl-3-cyclohexenyl;
$R_3{}^b$ ist Alkyl, Cycloalkyl oder Phenyl;
$R_4{}^b$ ist H oder Alkyl;
eines der $R_5{}^b$ und $R_6{}^b$ ist H und das andere ist Alkyl-$X^b$-, Phenyl-$X^b$-alkoxy, Phenoxy, Phenyl, Cycloalkyl, Alkyl oder Phenylalkyl; oder
$R_5{}^b$ und $R_6{}^b$ bilden gemeinsam $-X^bCH_2CH_2X^b-$, $X^b$ ist S, SO oder $SO_2$;
$R_7{}^b$ ist H oder $CH(R_2{}^b)OCOR_3{}^b$;
$n^b$ ist 0 oder 1;
"Aryl" ist Phenyl, das gegebenenfalls substituiert ist durch Halogen, Alkyl, Alkoxy, Alkylthio, OH, Alkanoyl, $NO_2$, Amino, Dialkylamino und/oder $CF_3$;
Alkyl weist 1-10, Cycloalkyl 3-7, Alkoxy 1-8 und Alkanoyl 2-9 C-Atome auf;
(c) mit der Formel

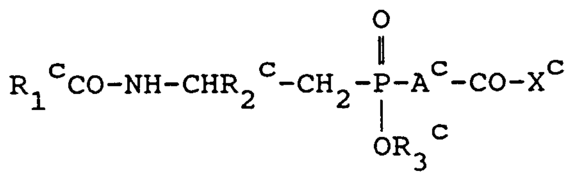

$R_1{}^c$ und $R_2{}^c$ sind H, 1-7C-Alkyl, 1-7C-Halogenalkyl, $(CH_2)_m{}^c$-$D^c$, 1-7C-Aminoalkyl oder eine Gruppe der Formel:

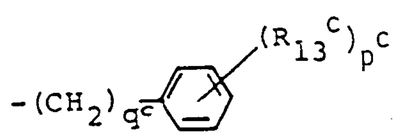

$D^c$ ist Cycloalkyl, Furyl, Thienyl oder Pyridinyl;
$A^c$ ist $(CH_2)_n$-$CHR_{21}{}^c$, $NR_{22}{}^c$-$CHR_{23}{}^c$ oder O-$CHR_{23}{}^c$;
$n^c$ ist 0 oder 1;
$q^c$ ist 0-7;
$R_{21}{}^c$ ist H, 1-7C-Alkyl, 1-7C-Halogenalkyl, Benzyl oder Phenethyl;
$R_{22}{}^c$ ist H oder 1-7C-Alkyl;
$R_{23}{}^c$ ist H, 1-7C-Alkyl, 1-7C-Halogenalkyl oder $(CH_2)_rcD_1{}^c$;
$D_1{}^c$ ist Phenyl, 4-Hydroxyphenyl, 3,4-Dihydroxyphenyl, 3-Indolyl, 4-Imidazolyl, $NH_2$, SH, 1-7C-Alkylthio, Guanidino oder $CONH_2$;
$X^c$ ist eine Gruppe der Formel $(II)^c$-$(V)^c$ oder $NR_4{}^c$-$CHR_5{}^c$-$COOR_6{}^c$;

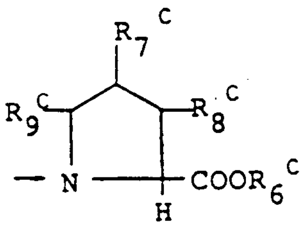

(II)$^c$

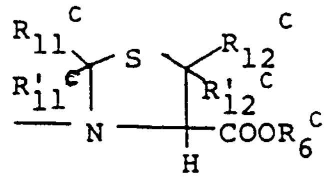

(III)$^c$

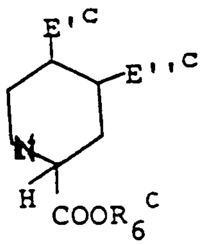

(IV)$^c$

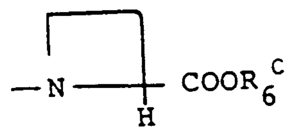

(V)$^c$

worin
$R_9{}^c$ und $R_8{}^c$ H sind und $R_7{}^c$ wie nachstehend definiert ist;
$R_9{}^c$ und $R_7{}^c$ H sind und $R_8{}^c$ wie nachstehend definiert ist;
$R_7{}^c$ und $R_8{}^c$ H sind und $R_9{}^c$ wie nachstehend definiert ist;
$R_9{}^c$ und $R_8{}^c$ H sind und $-CHR_7{}^c$- durch $-CR_{10}{}^cR_{10}{}^c$ ersetzt ist:
$R_9{}^c$ H ist und $R_7{}^c + R_8{}^c$ eine Doppelbindung bilden;
$R_9$c H ist und $R_7{}^c + R_8{}^c$ einen kondensierten Benzol-Ring vervollständigen; oder
$R_8{}^c$ H ist und $R_9{}^c + R_7{}^c$ einen kondensierten Benzol-Ring vervollständigen;
E'$^c$ und E''$^c$ H sind, oder E'$^c$ + E''$^c$ einen kondensierten Benzol-Ring vervollständigen;
$R_7{}^c$ H, 1-7C-Alkyl, Halogen, Keto, OH, 2-8C-Alkanoylamino, $N_3$, $NH_2$, $NR_{19}{}^cR_{20}{}^c$, $(CH_2)_m{}^c$-$D^c$, O-CONR$_{15}{}^c$R$_{15}{}^c$,1-7C-Alkoxy, 1-7C-Alkylthio oder eine Gruppe der Formel (VI)$^c$, (VII)$^c$ oder (VIII)$^c$ ist:

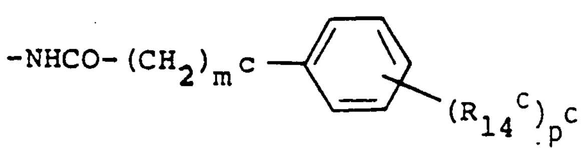

(VI)$^c$

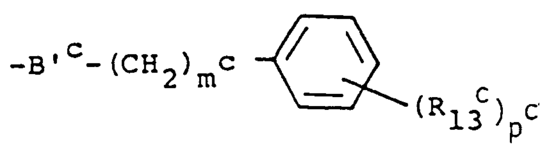

(VII)$^c$

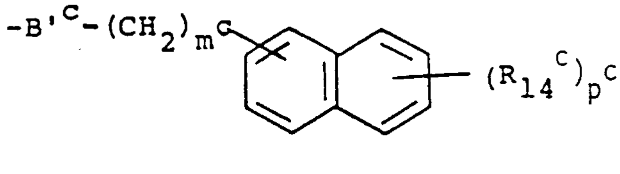

(VIII)$^c$

B'$^c$ eine Bindung ist oder O oder S; $R_8{}^c$ Keto, Halogen, O-CONR$_{15}{}^c$R$_{15}{}^c$, 1-7C-Alkoxy, 1-7C-Alkylthio oder eine Gruppe (VII)$^c$ oder (VIII)$^c$ ist, worin B'$^c$ O oder S ist;
$R_9{}^c$ Keto oder eine Gruppe (VII)$^c$ ist, worin B'$^c$ eine Bindung ist;
$R_{10}{}^c$ Halogen oder $Y^cR_{16}{}^c$ ist;
$R_{11}{}^c$, $R'_{11}{}^c$, $R_{12}{}^c$ und $R'_{12}{}^c$ H oder 1-7C-Alkyl sind, oder
$R_{11}{}^c$ eine Gruppe der Formel (IX)$^c$ ist und die anderen 3 H sind;

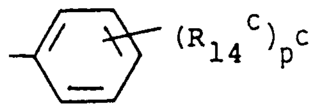

(IX)$^c$

$R_{13}{}^c$ H, 1-4C-Alkyl, 1-4C-Alkoxy, 1-4C-Alkylthio, Cl, Br, F, $CF_3$, OH, Ph, PhO, PhS oder $PhCH_2$ ist;
$R_{14}{}^c$ H, 1-4C-Alkyl, 1-4C-Alkoxy, 1-4C-Alkylthio, Cl, Br, F, $CF_3$ oder OH ist;
$m^c$ 0-3 ist;
$p^c$ 1-3 ist, jedoch 2 oder 3 nur dann, wenn $R_{13}{}^c$ oder $R_{14}{}^c$ H, Me, MeO, Cl oder F ist;
$R_{15}{}^c$ H oder 1-4C-Alkyl ist;
$Y^c$ O oder S ist;
$R_{16}{}^c$ 1-4C-Alkyl oder Gruppe (VII)$^c$ ist, worin $B'^c$ eine Bindung ist; oder die beiden $R_{16}{}^c$-Gruppen einen 5- oder 6gliedrigen Ring vervollständigen, worin die C-Atome jeweils 1 oder 2 Alkyle mit 1-4 C-Atomen tragen können;
$R_4{}^c$ H, 1-7C-Alkyl, Cycloalkyl oder $(CH_2)_r{}^cPh$ ist;
$R_5{}^c$ H, 1-7C-Alkyl oder $(CH_2)_r{}^c$-$D_1{}^c$ ist;
$r^c$ 1-4 ist;
$R_3{}^c$ und $R_6{}^c$ H, 1-7C-Alkyl, $PhCH_2$, PhCH oder $CHR_{17}{}^c$-O-$COR_{28}{}^c$ sind;
$R_{17}{}^c$ H, 1-7C-Alkyl oder Ph ist;
$R_{18}{}^c$ H, 1-7C-Alkyl, 1-7C-Alkoxy oder Ph ist; oder $R_{17}{}^c$ + $R_{18}{}^c$ $(CH_2)_2$, $(CH_2)_3$, -CH=CH- oder o-Phenylen bilden;
$R_{19}{}^c$ 1-7C-Alkyl, Benzyl oder Phenethyl ist; und $R_{20}{}^c$ H oder wie für $R_{19}{}^c$ ausgewählt ist;
(d) mit der Formel

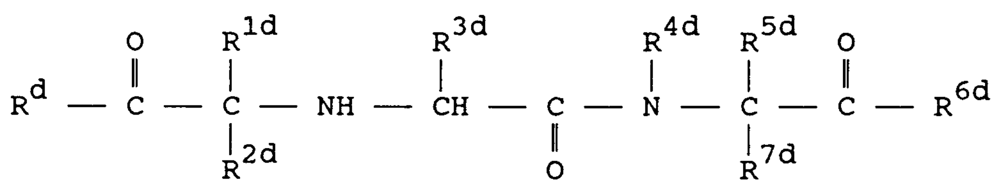

worin
$R^d$ und $R^{6d}$ gleich oder verschieden sind und Hydroxy, Niederalkoxy, Niederalkenoxy, Diniederalkylamino-niederalkoxy (Dimethylaminoethoxy), Acylamino-niederalkoxy (Acetylaminoethoxy), Acyloxy-niederalkoxy (Pivaloyloxymethoxy), Aryloxy wie etwa Phenoxy, Ar-niederalkoxy wie etwa Benzyloxy, substituiertes Aryloxy oder substituiertes Ar-niederalkoxy, worin der Substituent Methyl, Halogen oder Methoxy ist, Amino, Niederalkylamino, Diniederalkylamino, Hydroxyamino, Ar-niederalkylamino wie etwa Benzylamino sind;
$R^{1d}$ Wasserstoff ist, Alkyl mit 1 bis 20 Kohlenstoff-Atomen, darunter verzweigte und cyclische und ungesättigte (wie etwa Ally) Alkyl-Gruppen, substituiertes Niederalkyl, worin der Substituent Halogen sein kann, Hydroxy, Niederalkyloxy, Aryloxy wie etwa Phenoxy, Amino, Diniederalkylamino, Acylamino wie etwa Acetamido und Benzamido, Arylamino, Guanidino, Imidazolyl, Indolyl, Mercapto, Niederalkylthio, Arylthio wie etwa Phenylthio, Carboxy oder Carboxamido, Carbo-niederalkoxy, Aryl wie etwa Phenyl oder Naphthyl, substituiertes Aryl wie etwa Phenyl, worin der Substituent Niederalkyl, Niederalkoxy oder Halogen ist, Ar-niederalkyl, Ar-niederalkenyl, Heteroar-niederalkyl oder Heteroar-niederalkenyl wie etwa Benzyl, Styryl oder Indolylethyl, substituiertes Arniederalkyl, substituiertes Ar-niederalkenyl, substituiertes Heteroar-niederalkyl oder substituiertes Heteroar-niederalkenyl, worin der (die) Substituenten Halogen, Dihalogen, Niederalkyl, Hydroxy, Niederalkoxy, Amino, Aminomethyl, Acylamino (Acetylamino oder Benzoylamino), Diniederalkylamino, Niederalkylamino, Carboxyl, Halogenniederalkyl, Cyan oder Sulfonamido ist (sind); Ar-niederalkyl oder Heteroar-niederalkyl, das am Alkyl-Teil substituiert ist durch Amino oder Acylamino (Acetylamino oder Benzoylamino);
$R^{2d}$ und $R^{7d}$ gleich oder verschieden sind und Wasserstoff oder Niederalkyl sind;
$R^{3d}$ Wasserstoff, Niederalkyl, Phenyl-niederalkyl, Aminomethyl-phenyl-niederalkyl, Hydroxyphenyl-niederalkyl, Hydroxy-niederalkyl, Acylamino-niederalkyl (wie etwa Benzoylamino-niederalkyl,

Acetylamino-niederalkyl), Amino-niederalkyl, Dimethylamino-niederalkyl, Halogen-niederalkyl, Guanidino-niederalkyl, Imidazolyl-niederalkyl, Indolyl-niederalkyl, Mercapto-niederalkyl, Niederalkylthio-niederalkyl ist;

$R^{4d}$ Wasserstoff oder Niederalkyl ist;

$R^{5d}$ Wasserstoff, Niederalkyl, Phenyl, Phenyl-niederalkyl, Hydroxyphenyl-niederalkyl, Hydroxy-niederalkyl, Amino-niederalkyl, Guanidino-niederalkyl, Imidazolylniederalkyl, Indolyl-niederalkyl, Mercapto-niederalkyl oder Niederalkylthio-niederalkyl ist;

$R^{4d}$ und $R^{5d}$ miteinander verknüpft sein können, zur Bildung einer Alkylen-Brücke mit 2 bis 4 Kohlenstoff-Atomen, einer Alkylen-Brücke mit 2 bis 3 Kohlenstoff-Atomen und einem Schwefel-Atom, einer Alkylen-Brücke mit 3 bis 4 Kohlenstoff-Atomen, die eine Doppelbindung enthält, oder eine Alkylen-Brücke wie vorstehend, substituiert mit Hydroxy, Niederalkoxy, Niederalkyl oder Diniederalkyl; und die pharmazeutisch annehmbaren Salze davon;

(e) mit der Formel

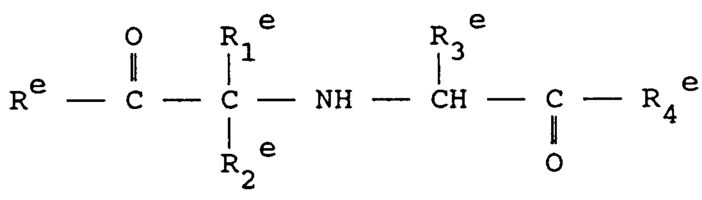

$R_4^e$ ist ein substituiertes Prolin der Formel

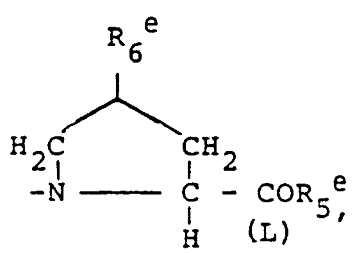

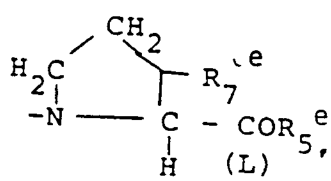

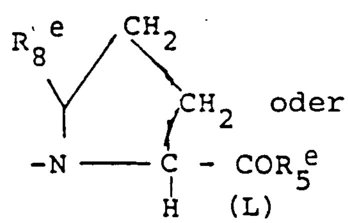

oder

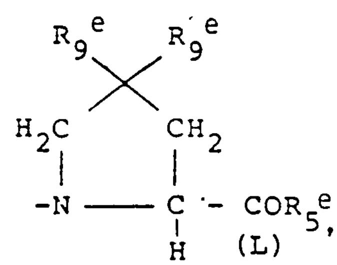

$R_6^e$ ist Halogen, Keto, Azido,

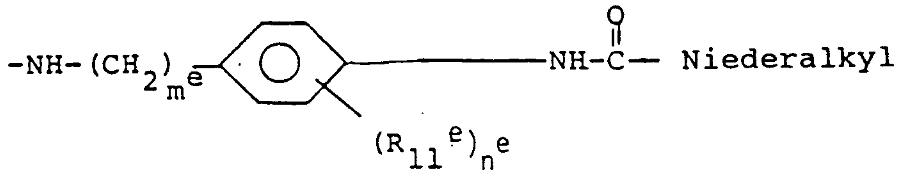

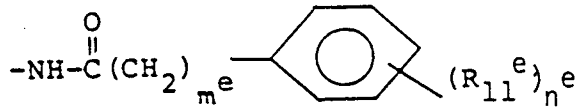

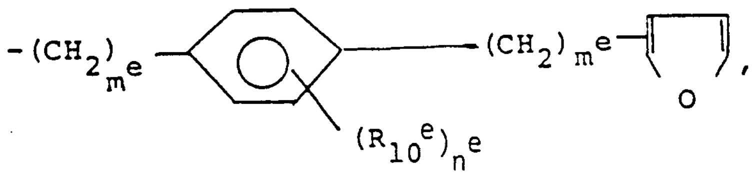

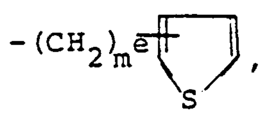

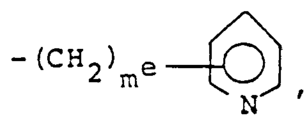

ein 1- oder 2-Naphthyl der Formel

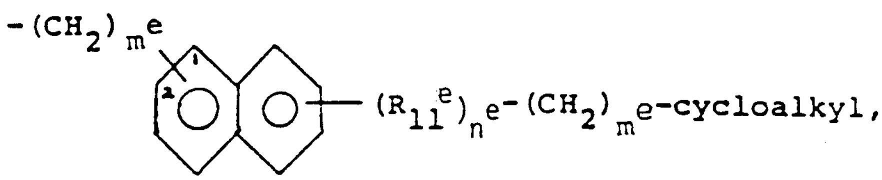

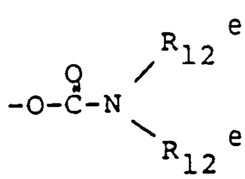

,

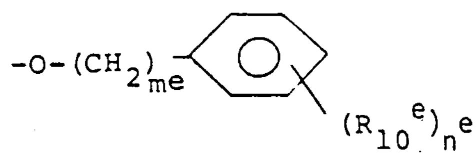

ein 1- oder 2-Naphthyloxy der Formel

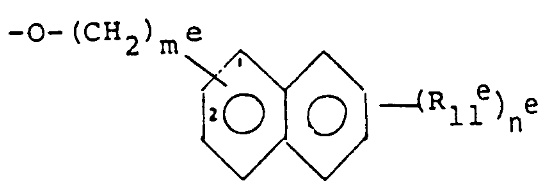

-S-Niederalkyl,

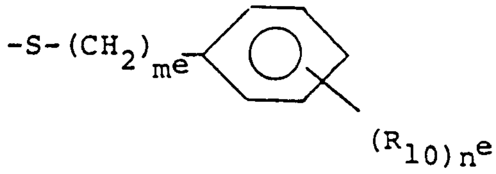

oder ein 1- oder 2-Naphthylthio der Formel

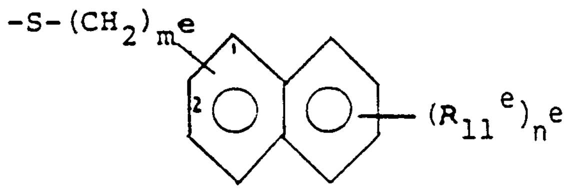

$R_7^e$ ist Keto, Halogen,

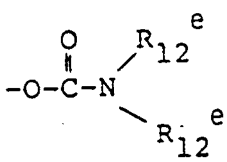

,

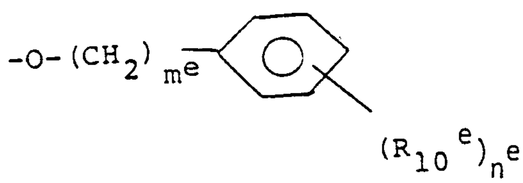

ein 1- oder 2-Naphthyloxy der Formel

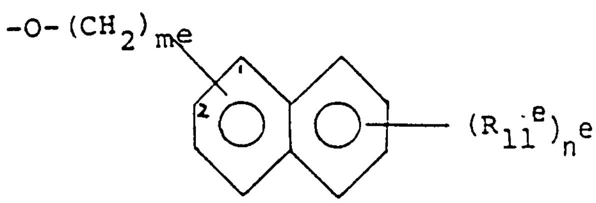

-S-Niederalkyl,

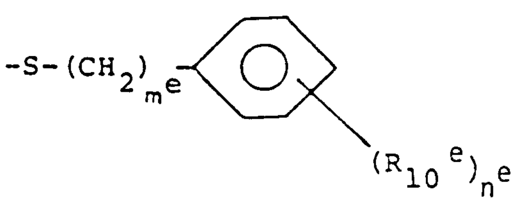

oder ein 1- oder 2-Naphthylthio der Formel

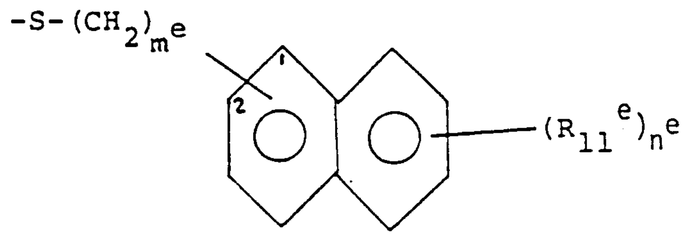

$R_8{}^e$ ist Keto oder

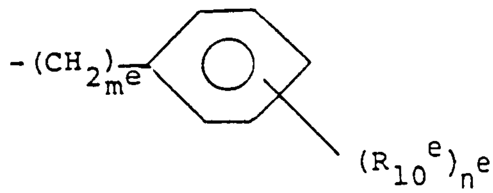

$R_9{}^e$ ist Halogen oder $-Y^e-R_{13}{}^e$;
$m^e$ ist null, eins, zwei oder drei;
$R_{10}{}^e$ ist Wasserstoff, Niederalkyl mit 1 bis 4 Kohlenstoff-Atomen, Niederalkoxy mit 1 bis 4 Kohlenstoff-Atomen, Niederalkylthio mit 1 bis 4 Kohlenstoff-Atomen, Chlor, Brom, Fluor, Trifluormethyl, Hydroxy, Phenyl, Phenoxy, Phenylthio oder Phenylmethyl;
$R_{11}{}^e$ ist Wasserstoff, Niederalkyl mit 1 bis 4 Kohlenstoff-Atomen, Niederalkoxy mit 1 bis 4 Kohlenstoff-Atomen, Niederalkylthio mit 1 bis 4 Kohlenstoff-Atomen, Brom, Fluor, Trifluormethyl oder Hydroxy;
$n^e$ ist eins, zwei oder drei, vorausgesetzt, daß n nur größer als eins ist wenn $R_{10}{}^e$ oder $R_{11}{}^e$ Wasserstoff, Methyl, Methoxy, Chlor oder Fluor ist;
$R_{12}{}^e$ ist Wasserstoff oder Niederalkyl mit 1 bis 4 Kohlenstoff-Atomen;
$Y^e$ ist Sauerstoff oder Schwefel;
$R_{13}{}^e$ ist Niederalkyl mit 1 bis 4 Kohlenstoff-Atomen;

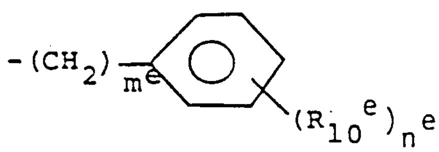

oder die $R_{13}{}^e$-Gruppen sind verknüpft, um einen unsubstituierten 5- oder 6gliedrigen Ring zu vervollständigen oder den Ring, worin eines oder mehrere der Kohlenstoff-Atome einen Niederalkylmit 1 bis 4 Kohlenstoff-Atomen oder einen Diniederalkyl-Substituenten mit 1 bis 4 Kohlenstoff-Atomen aufweist;
$R^e$ und $R_5{}^e$ sind unabhängig voneinander ausgewählt aus Hydroxy, Niederalkoxy, Diniederalkylamino-niederalkoxy wie etwa Dimetylaminoethoxy, Niederalkylcarbonylaminoniederalkoxy wie etwa Acetylaminoethoxy, Niederalkylcarbonyloxy-niederalkoxy wie etwa Pivaloyloxymethoxy;

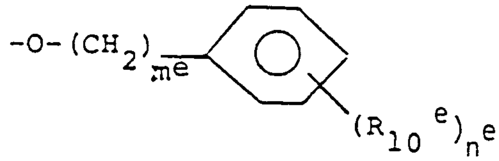

worin $m^e$, $n^e$ und $R_{10}{}^e$ wie vorstehend definiert sind, Amino, Niederalkylamino, Diniederalkylamino, Hydroxyamino, Benzylamino oder Phenethylamino;
$R_1{}^e$ ist Wasserstoff, Niederalkyl,

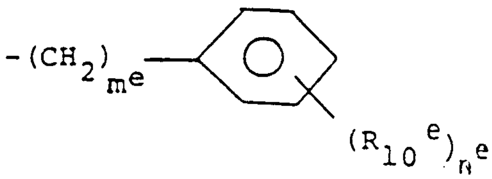

Halogen-substituiertes Niederalkyl, Hydroxy-substituiertes Niederalkyl, $-(CH_2)_q$e-Cycloalkyl, $-(CH_2)_q$e-Carboxy, $-(CH_2)_q$e-S-Niederalkyl,

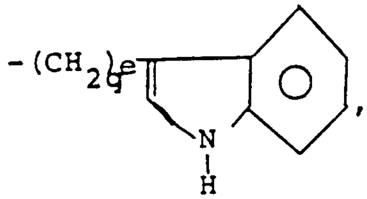, 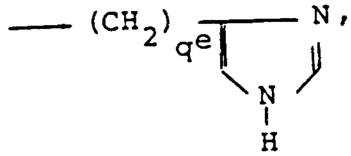

$-(CH_2)_{q^e}$-Guanidinyl, $-(CH_2)_{q^e}-NH_2$,
$-(CH_2)_{q^e}-N$(Niederalkyl)$_2$

$-(CH_2)_{q^e}-NH-\overset{O}{\overset{\|}{C}}-$Niederalkyl,

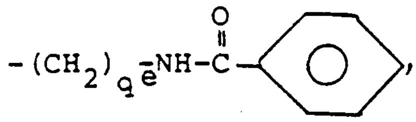, $-(CH_2)_{q^e}-SH$,

$-(CH_2)_{q^e}$-Niederalkoxy, 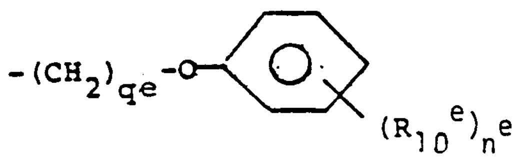

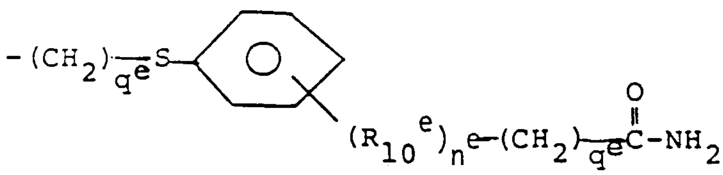

$-(CH_2)_{q^e}-\overset{O}{\overset{\|}{C}}-$Niederalkoxy oder

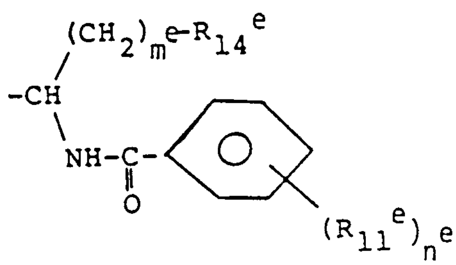

worin $m^e$, $n^e$, $R_{10}{}^e$ und $R_{11}{}^e$ wie vorstehend definiert sind, $R_{14}{}^e$ Niederalkyl, Cycloalkyl oder

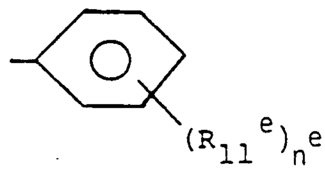

ist, und $q^e$ eine ganze Zahl von 1 bis 4 ist;

$R_2^e$ ist Wasserstoff oder Niederalkyl;

$R_3^e$ ist Wasserstoff, Niederalkyl, Halogen-substituiertes Niederalkyl, Hydroxy-substituiertes Niederalkyl, $-(CH_2)_q e-NH_2$, $-(CH_2)_q e-N(Niederalkyl)_2$, $-(CH_2)_q e$-Guanidinyl, $-(CH_2)_q e-SH$, $-(CH_2)_q e$-S-Niederalkyl,

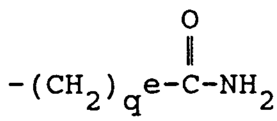

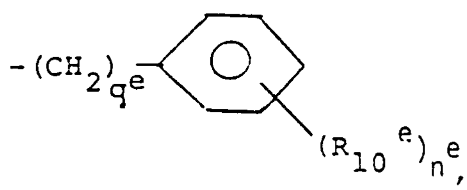

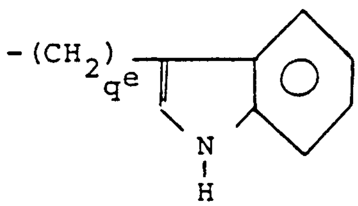

,

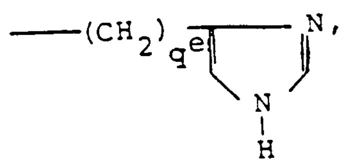

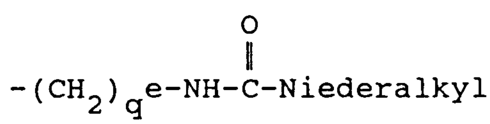 oder

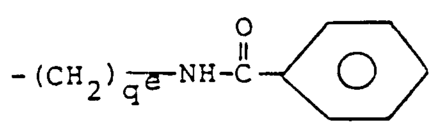

worin $R_{10}^e$, $n^e$ und $q^e$ wie vorstehend definiert sind;

(f) mit der Formel

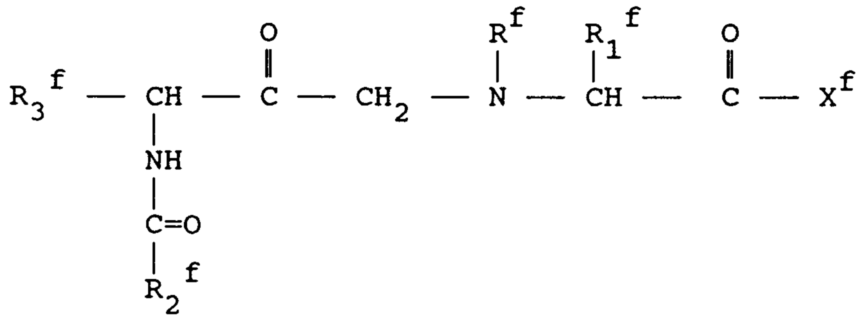

$X^f$ ist eine Amino- oder Iminosäure der Formel

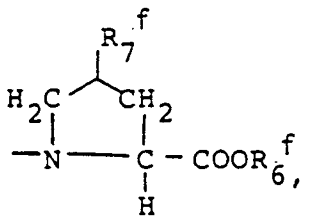

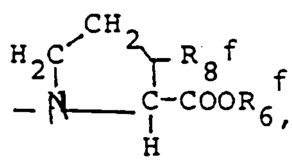

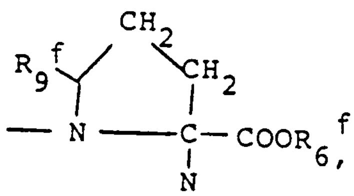

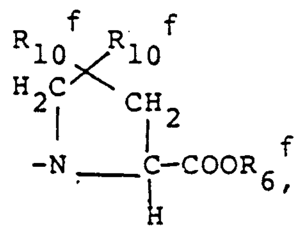

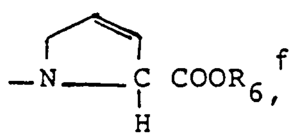

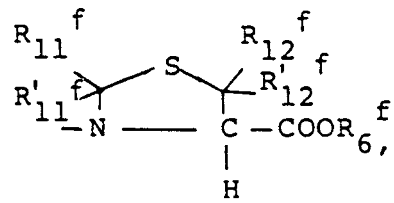

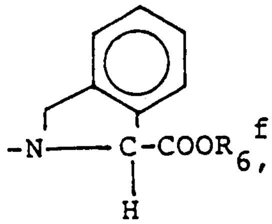

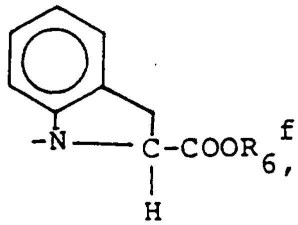

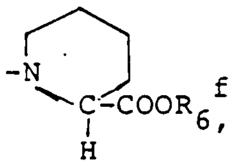

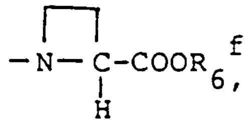

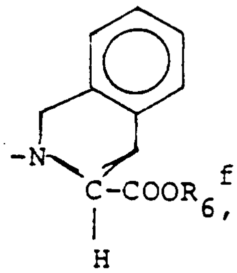

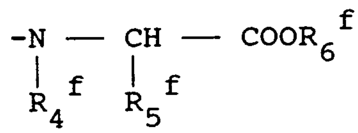 oder

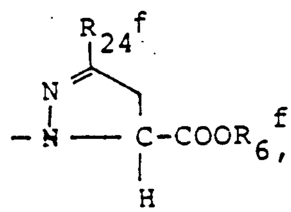

$R_7{}^f$ ist Wasserstoff, Niederalkyl, Halogen, Keto, Hydroxy,

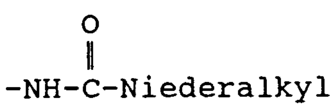 Azido, Amino, 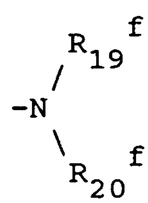 ,

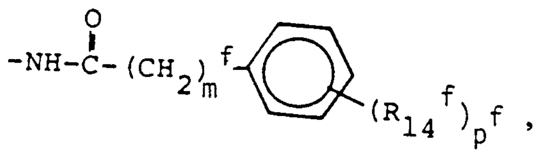

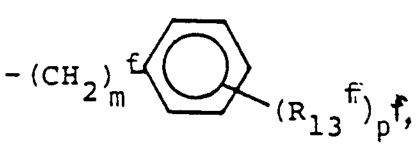

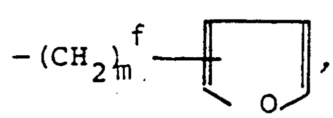

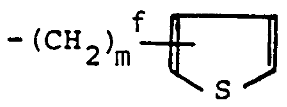 ,

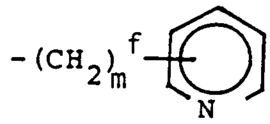 ,

ein 1- oder 2-Naphthyl der Formel

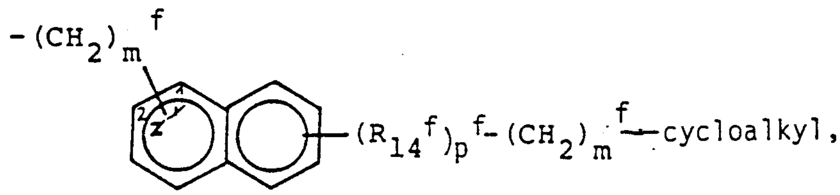

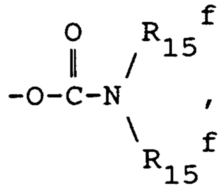 ,

-O-Niederalkyl

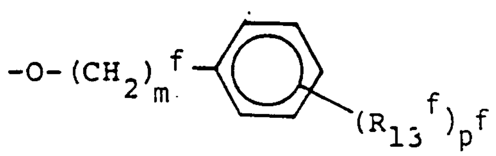

ein 1- oder 2-Naphthyloxy der Formel

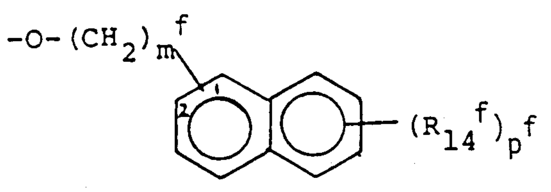

-S-Niederalkyl,

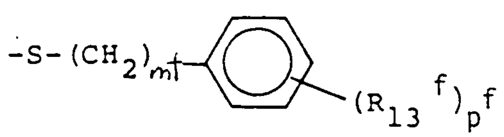

oder ein 1- oder 2-Naphthylthio der Formel

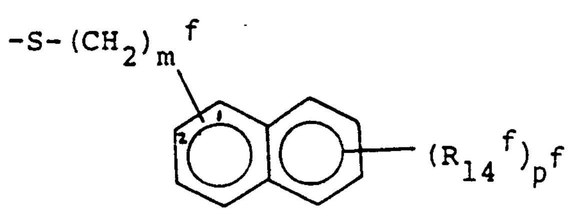

$R_8{}^f$ ist Keto, Halogen,

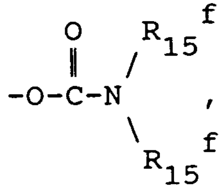 , 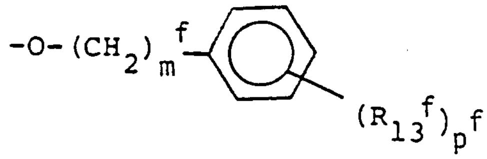

-O-Niederalkyl, ein 1- oder 2-Naphthyloxy der Formel

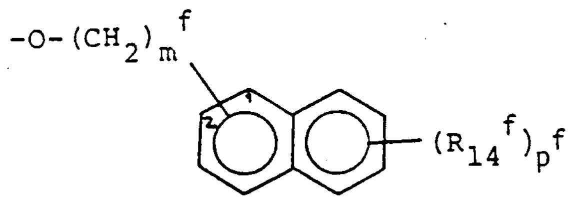

-S-Niederalkyl,

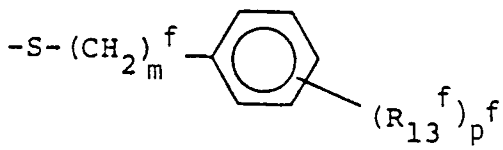

oder ein 1- oder 2-Naphthylthio der Formel

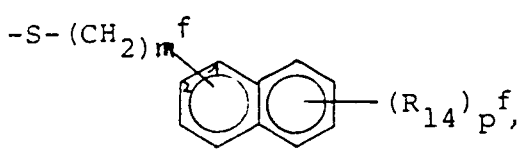

$R_9{}^f$ ist Keto oder

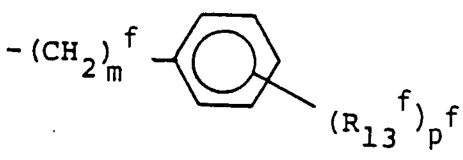

$R_{10}{}^f$ ist Halogen oder $-Y^f-R_{16}{}^f$,

$R_{11}{}^f$, $R'_{11}{}^f$, $R_{12}{}^f$ und $R'_{12}{}^f$ sind unabhängig ausgewählt aus Wasserstoff und Niederalkyl, oder $R'_{11}{}^f$, $R_{12}{}^f$ und $R'_{12}{}^f$ sind Wasserstoff und $R_{11}{}^f$ ist

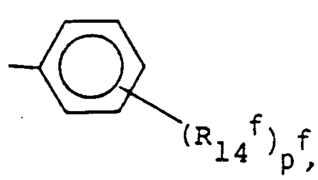

$R_{13}{}^f$ ist Wasserstoff, Niederalkyl mit 1 bis 4 Kohlenstoff-Atomen, Niederalkoxy mit 1 bis 4 Kohlenstoff-Atomen, Niederalkylthio mit 1 bis 4 Kohlenstoff-Atomen, Chlor, Brom, Fluor, Trifluormethyl, Hydroxy, Phenyl, Phenoxy, Phenylthio oder Phenylmethyl;
$R_{14}{}^f$ ist Wasserstoff, Niederalkyl mit 1 bis 4 Kohlenstoff-Atomen, Niederalkoxy mit 1 bis 4 Kohlenstoff-Atomen, Niederalkylthio mit 1 bis 4 Kohlenstoff-Atomen, Chlor, Brom, Fluor, Trifluormethyl oder Hydroxy;
$m^f$ ist null, eins, zwei, drei oder vier;
$p^f$ ist eins, zwei oder drei, vorausgesetzt, daß $p^f$ nur größer als eins ist wenn $R_{13}{}^f$ oder $R_{14}{}^f$ Wasserstoff, Methyl, Methoxy, Chlor oder Fluor ist;
$R_{15}{}^f$ ist Wasserstoff oder Niederalkyl mit 1 bis 4 Kohlenstoff-Atomen;
$Y^f$ ist Sauerstoff oder Schwefel;
$R_{16}{}^f$ ist Niederalkyl mit 1 bis 4 Kohlenstoff-Atomen;

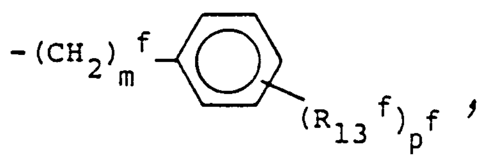

oder die $R_{16}{}^f$-Gruppen sind verknüpft, um einen unsubstituierten 5- oder 6gliedrigen Ring zu vervollständigen oder den Ring, worin eines oder mehrere der Kohlenstoff-Atome einen Niederalkylmit 1 bis 4 Kohlenstoff-Atomen oder einen Diniederalkyl-Substituenten mit 1 bis 4 Kohlenstoff-Atomen aufweist;
$R_4{}^f$ ist Wasserstoff, Niederalkyl,

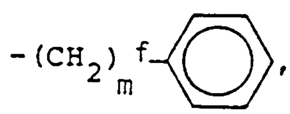 $-(CH_2)_m{}^f$-Cycloalkyl,

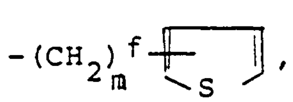 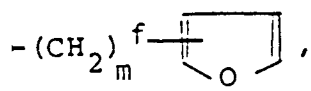

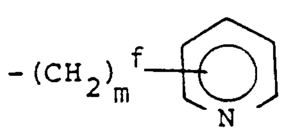 oder 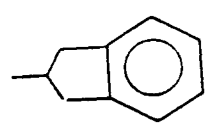

$R_5{}^f$ ist Wasserstoff, Niederalkyl,

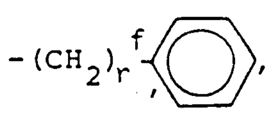 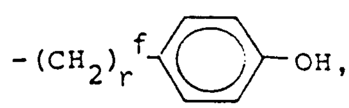

$-(CH_2)_r{}^f-OH$,

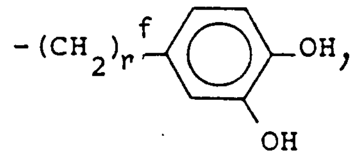

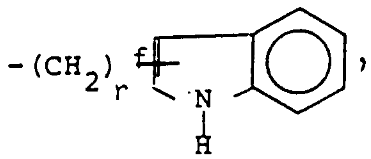

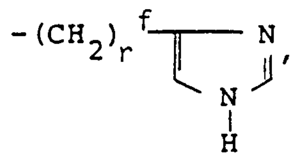

$-(CH_2)_r{}^f-NH_2$, $-(CH_2)_r{}^f-SH$, $-(CH_2)_r{}^f$-S-Niederalkyl,

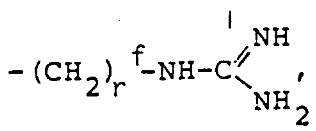

oder

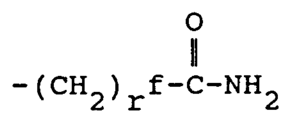

$r^f$ ist eine ganze Zahl von 1 bis 4;
$R_{19}{}^f$ ist Niederalkyl, Benzyl oder Phenethyl;
$R_{20}{}^f$ ist Wasserstoff, Niederalkyl, Benzyl oder Phenethyl;
$R^f$ ist Wasserstoff, Niederalkyl, Cycloalkyl,

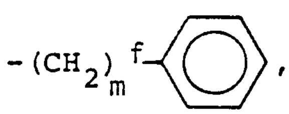

$-(CH_2)_2-NH_2$, $-(CH_2)_3-NH_2$, $-(CH_2)_4-NH_2$, $-(CH_2)_2-OH$, $-(CH_2)_3-OH$, $-(CH_2)_4-OH$, $-(CH_2)_2-SH$, $-(CH_2)_3-SH$ oder $-(CH_2)_4-SH$;
$R_1{}^f$ ist Wasserstoff, Niederalkyl, Halogen-substituiertes Niederalkyl,

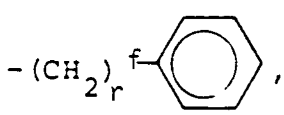

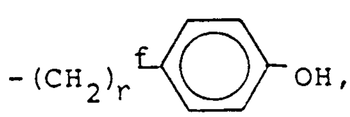

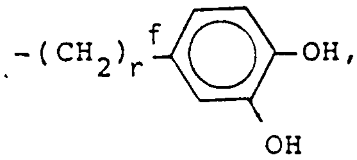 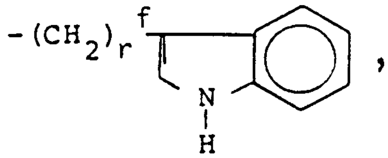 ,

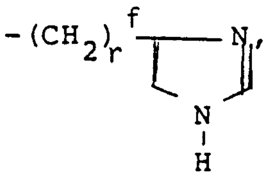 $-(CH_2)_r f-NH_2$, $-(CH_2)_r f-SH$,

$-(CH_2)_r f-OH$, $-(CH_2)_r f-S-Niederalkyl$,

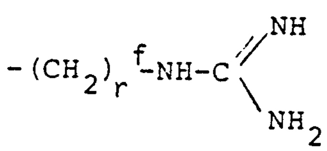 oder 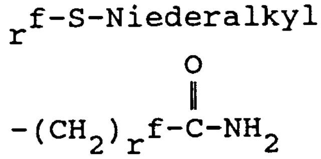

vorausgesetzt, daß $R_1{}^f$ nur Wasserstoff ist, wenn $R^f$ nicht Wasserstoff ist;
$R_2{}^f$ ist

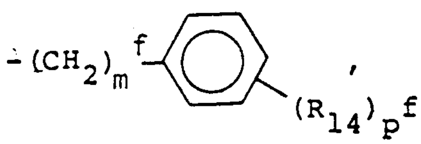 , 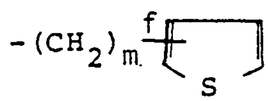 ,

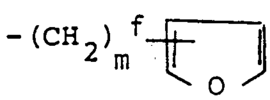 oder 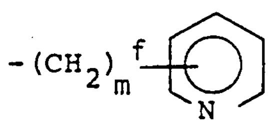

$R_3{}^f$ ist Wasserstoff, Niederalkyl,

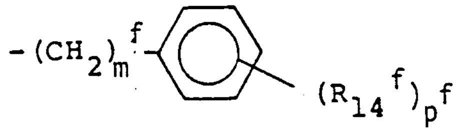

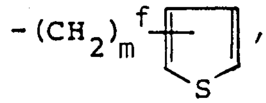

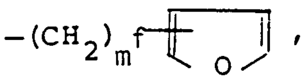

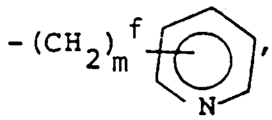

Halogen-substituiertes Niederalkyl, $-(CH_2)_m{}^f$-Cycloalkyl

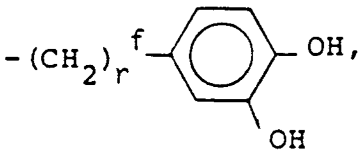

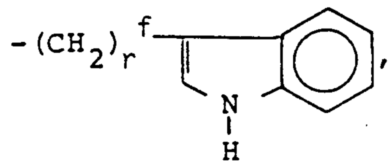

$-(CH_2)_r{}^f-OH$, 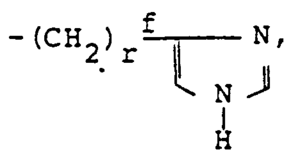 $-(CH_2)_r{}^f-NH_2$, $-(CH_2)_r{}^f-SH$,

$-(CH_2)_r{}^f$-S-Niederalkyl,

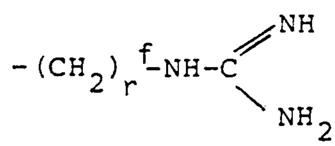

oder 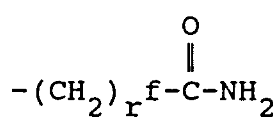

worin $m^f$, $R_{14}{}^f$, $p^f$ und $r^f$ wie vorstehend definiert sind; $R_6{}^f$ ist Wasserstoff, Niederalkyl, Benzyl, Benzhydryl,

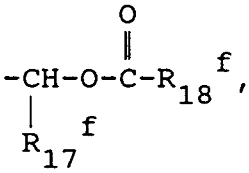

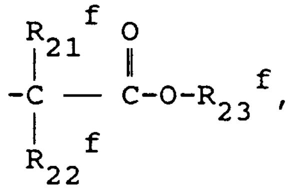

$-CH(CH_2-OH)_2$,

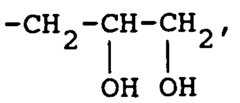, $-(CH_2)_2-N(CH_3)_2$ oder 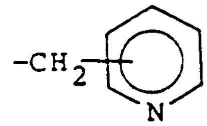

$R_{17}{}^{f}$ ist Wasserstoff, Niederalkyl, Cycloalkyl oder Phenyl;
$R_{18}{}^{f}$ ist Wasserstoff, Niederalkyl, Niederalkoxy oder Phenyl, oder $R_{17}{}^{f}$ und $R_{18}{}^{f}$ sind zusammengenommen $-(CH_2)_2-$, $-(CH_2)_3-$, $-CH=CH-$ oder

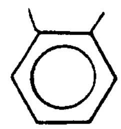,

$R_{21}{}^{f}$ und $R_{22}{}^{f}$ sind unabhängig voneinander ausgewählt aus Wasserstoff und Niederalkyl;
$R_{23}{}^{f}$ ist Niederalkyl;
$R_{24}{}^{f}$ ist Wasserstoff, Niederalkyl;

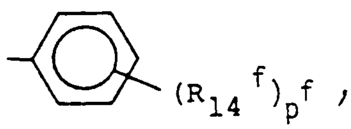,

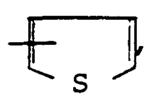, 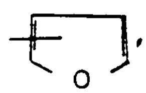, oder 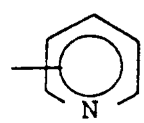

(g) mit der Formel

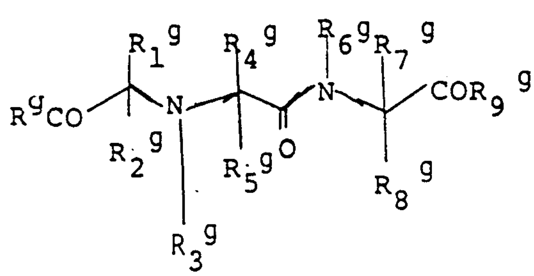

worin entweder

(A) $R^{g}$ und $R_9{}^{g}$ OH, 1-6C-Alkoxy, 2-6C-Alkenyloxy, Di(1-6C-alkyl)amino(1-6C)alkoxy, 1-6C-Hydroxyalkoxy, Acylamino(1-6C)alkoxy, Acyloxy(1-6C)alkoxy,, Aryloxy, Aryloxy(1-6C)alkoxy, $NH_2$, Mono- oder Di(1-6C-alkyl)amino, Hydroxyamino oder Aryl(1-6C)alkylamino sind;

$R_1{}^{g}$-$R_5{}^{g}$, $R_7{}^{g}$ und $R_8{}^{g}$ 1-20C-Alkyl, 2-20C-Alkenyl, 2-20C-Alkinyl, Aryl, Aryl(1-6C)alkyl mit 7-12 C- oder Heterocyclyl(1-6C)alkyl mit 7-12 C-Atomen sind;

$R_6{}^{g}$ Cycloalkyl, Polycycloalkyl, teilweise gesättigtes Cycloalkyl oder Polycycloalkyl, Cycloalkyl(1-

6C)alkyl mit 3-20C, 6-10C-Aryl, Aryl(1-6C)alkyl, Aryl(2-6C)alkenyl oder Aryl(2-6C)alkinyl ist; oder $R_2{}^g$ und $R_3{}^g$, zusammen mit den C- und N-Atomen woran sie geknüpft sind, oder $R_3{}^g$ und $R_5{}^g$, zusammen mit den N- und C-Atomen woran sie geknüpft sind, einen N-Heteroring bilden, der 3-5C oder 2-4C und ein S-Atom enthält;

alle Alkyl, Alkenyl und Alkinyl gegebenenfalls substituiert sind durch OH, 1-6C-Alkoxy, SH, 1-6C-Alkylthio, $NH_2$, Mono- oder Di(1-6C-alkyl)amino, Halogen oder $NO_2$; alle Cycloalkyl-Gruppen (einschließlich Poly und teilweise ungesättigt) gegebenenfalls substituiert sind durch Halogen, 1-6C-Hydroxyalkyl, 1-6C-Alkoxy, Amino(1-6C-alkyl)amino, Di(1-6C-alkyl)amino, SH, 1-6C-Alkylthio, $NO_2$ oder $CF_3$; und

Aryl-Gruppen gegebenenfalls sind substituiert durch OH, 1-6C-Alkoxy, $NH_2$, Mono- oder Di(1-6C-alkyl)amino, SH, 1-6C-Alkylthio, 1-6C-Hydroxyalkyl, 1-6C-Aminoalkyl, 1-6C-Thioalkyl, $NO_2$, Halogen, $CF_3$, $OCH_2O$, Ureido oder Guanidino; oder

(B) $R^g$ und $R_9{}^g$ H oder 1-6C-Alkoxy sind;

$R_1{}^g$ und $R_2{}^g$ H, 1-6C-Alkyl, Aryl(1-6C)alkyl mit 7-12 C-Atomen oder Heterocyclyl(1-6C)alkyl mit 6-12 C-Atomen sind;

$R_3{}^g$-$R_5{}^g$, $R_7{}^g$ und $R_8{}^g$ H oder 1-6C-Alkyl sind;

$R_6{}^g$ Cycloalkyl, Polycycloalkyl, teilweise gesättigtes Cycloalkyl oder Polycycloalkyl, Cycloalkyl(1-6C)alkyl mit 3-20 C-Atomen, Aryl oder Aryl(1-6C)alkyl ist; und

Aryl 6-10 C aufweist und gegebenenfalls substituiert ist durch 1-6C-Alkyl, 2-6C-Alkenyl, 2-6C-Alkinyl, OH, 1-6C-Alkoxy, $NH_2$, Mono- oder Di(1-6C-alkyl)amino, SH, 1-6C-Alkylthio, 1-6C-Hydroxyalkyl, 1-6C-Aminoalkyl, 1-6C-Thioalkyl, $NO_2$, Halogen, $CF_3$, $OCH_2O$, Ureido oder Guanidino;

(h) mit der Formel

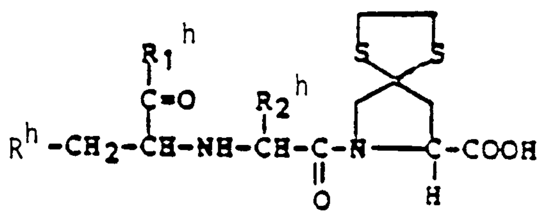

worin

$R^h$ Niederalkyl, Benzyl, Benzylthio, Benzyloxy, Phenylthio oder Phenoxy ist;

$R_1{}^h$ Hydroxy, oder Niederalkoxy ist;

$R_2{}^h$ Wasserstoff, Niederalkyl oder Amino-Niederalkyl ist;

sowie die pharmazeutisch annehmbaren Salze davon;

(i) mit der Formel

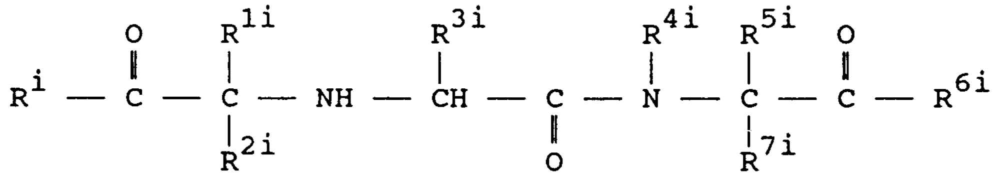

oder ein pharmazeutisch annehmbares Salz davon, worin $R^i$ und $R^{6i}$ gleich oder verschieden sind und Hydroxy, Niederalkoxy, Niederalkenyloxy, Diniederalkylamino-niederalkoxy, Acylamino-niederalkoxy, Acyloxy-niederalkoxy, Aryloxy, Aryl-niederalkoxy, Amino, Niederalkylamino, Diniederalkylamino, Hydroxyamino, Aryl-niederalkylamino oder substituiertes Aryloxy oder substituiertes Arylniederalkoxy sind, worin der Substituent Methyl, Halogen oder Methoxy ist;

$R^{1i}$ Wasserstoff, Alkyl mit 1 bis 10 Kohlenstoff-Atomen, substituiertes Niederalkyl ist, worin der Substituent Hydroxy, Niederalkoxy, Aryloxy, substituiertes Aryloxy, Heteroaryloxy, substituiertes Heteroaryloxy, Amino, Niederalkylamino, Diniederalkylamino, Acylamino, Arylamino, substituiertes Arylamino, Guanidino, Imidazolyl, Indolyl, Niederalkylthio, Arylthio, substituiertes Arylthio, Carboxy, Carbamoyl, Niederalkoxycarbonyl, Aryl, substituiertes Aryl, Aralkyloxy, substituiertes Aralkyloxy, Aralkylthio oder substituiertes Aralkylthio ist, worin der Aryl- oder Heteroaryl-Teil der substituierten

Aryloxy-, Heteroaryloxy-, Arylamino-, Arylthio-, Aryl-, Aralkyloxy-, Aralkylthio-Gruppe substituiert ist mit einer Gruppe, ausgewählt aus Halogen, Niederalkyl, Hydroxy, Niederalkoxy, Amino, Aminomethyl, Carboxyl, Cyan oder Sulfamoyl;
$R^{2i}$ und $R^{7i}$ gleich oder verschieden sind und Wasserstoff oder Niederalkyl sind;
$R^{3i}$ Wasserstoff, Niederalkyl, Phenyl-niederalkyl, Aminomethylphenyl-niederalkyl, Hydroxyphenyl-niederalkyl, Hydroxy-niederalkyl, Acylamino-niederalkyl, Amino-niederalkyl, Dimethylamino-niederalkyl, Guanidino-niederalkyl, Imidazolyl-niederalkyl, Indolyl-niederalkyl oder Niederalkylthio-niederalkyl ist;
$R^{4i}$ und $R^{5i}$ gleich oder verschieden sind und Wasserstoff, Niederalkyl oder $Z^i$ sind, oder $R^{4i}$ und $R^{5i}$ zusammengenommen eine Gruppe bilden, dargestellt durch $Q^i$, $U^i$, $V^i$, $Y^i$, $D^i$ oder $E^i$, worin $Z^i$

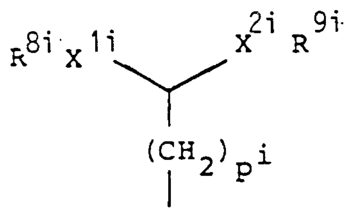

ist, worin $X^{1i}$ und $X^{2i}$ unabhängig voneinander O, S oder $CH_2$ sind; $R^{8i}$ und $R^{9i}$ unabhängig voneinander Niederalkyl, Niederalkenyl, Niederalkinyl, Cycloalkyl mit 3 bis 8 Kohlenstoff-Atomen, Hydroxy-niederalkyl oder $-(CH_2)_n iAr^i$ sind, worin $n^i$ 0, 1, 2 oder 3 ist und $Ar^i$ unsubstituiertes oder substituiertes Phenyl, Furyl, Thienyl oder Pyridyl ist, wobei die substituierten Phenyl-, Furyl-, Thienyl- oder Pyridyl- mit wenigstens einer Gruppe substituiert sind, die unabhängig ausgewählt ist aus $C_1$- bis $C_4$-Alkyl, Niederalkoxy, Niederalkylthio, Halogen, $CF_3$ und Hydroxy, oder $R^{8i}$ und $R^{9i}$ zusammengenommen eine Brücke $W^i$ bilden, wobei $W^i$ eine Einfachbindung oder eine Methylen-Brücke oder eine substituierte Methylen-Brücke ist, wenn wenigstens eines der $X^{1i}$ und $X^{2i}$ Methylen ist, oder $W^i$ eine Alkylen- oder substituierte Alkylen-Brücke mit 2 bis 3 Kohlenstoff-Atomen ist, wobei die substituierte Methylen-Brücke oder die substituierte Alkylen-Brücke einen oder zwei Substituenten aufweist, ausgewählt aus Niederalkyl-, Aryl- und Aryl-niederalkyl-Gruppen, und $p^i$ 0, 1 oder 2 ist; mit der Maßgabe, daß wenigstens eines der $R^4$ und $R^5$ $Z^i$ ist, mit der Maßgabe, daß, wenn $R^4$ $Z^i$ ist und $p^i$ 0 ist, $X^{1i}$ und $X^{2i}$ beide Methylen sein müssen, und mit der Maßgabe, daß, wenn $X^{1i}$ und $X^{2i}$ beide Methylen sind, $R^{8i}$ und $R^{9i}$ eine Alkylen-Brücke $W^i$ bilden müssen;
$Q^i$

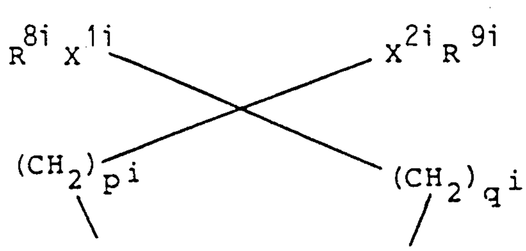

ist, worin $R^{8i}$, $R^{9i}$, $X^{1i}$ und $X^{2i}$ wie vorstehend definiert sind, $p^i$ 0, 1 oder 2 ist, $q^i$ 0, 1 oder 2 ist, mit der Maßgabe, daß die Summe von $p^i$ und $q^i$ 1, 2 oder 3 sein muß, mit der Maßgabe, daß, wenn $p^i$ 0 ist, $X^{1i}$ und $X^{2i}$ Methylen sein müssen, und mit der Maßgabe, daß, wenn $X^{1i}$ und $X^{2i}$ Methylen sind, $R^{8i}$ und $R^{9i}$ zusammengenommen eine Brücke $W^i$ bilden, worin $W^i$ wie vorstehend definiert ist;
$V^i$

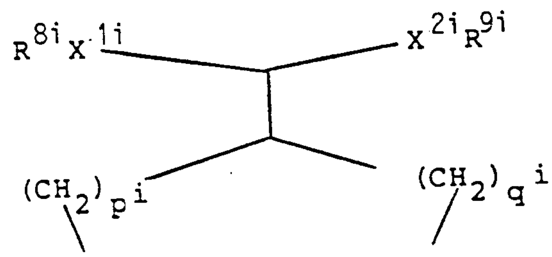

ist, worin $R^{8i}$, $R^{9i}$, $X^{1i}$ und $X^{2i}$ wie vorstehend definiert sind, $p^i$ 0, 1 oder 2 ist, und $q^i$ 0, 1 oder 2 ist, mit der Maßgabe, daß die Summe von $p^i$ und $q^i$ 1, 2 oder 3 ist, mit der Maßgabe, daß, wenn $X^{1i}$ und $X^{2i}$ $CH_2$ sind, $R^{8i}$ und $R^{9i}$ zusammengenommen eine Brücke $W^i$ bilden, worin $W^i$ wie vorstehend definiert ist;

$U^i$

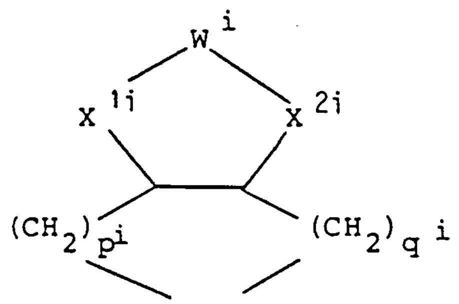

ist, worin $W^i$ wie vorstehend definiert ist (außer, daß $W^i$ auch eine Methylen-Brücke sein kann, wenn $X^{1i}$ und $X^{2i}$ Sauerstoff oder Schwefel sind), $X^{1i}$ und $X^{2i}$ wie vorstehend definiert sind, $p^i$ 0, 1 oder 2 ist, $q^i$ 0, 1 oder 2 ist, mit der Maßgabe, daß die Summe von $p^i$ und $q^i$ 1 oder 2 ist, mit der Maßgabe, daß, wenn $p^i$ 0 ist, $X^{1i}$ $CH_2$ sein muß;

$Y^i$

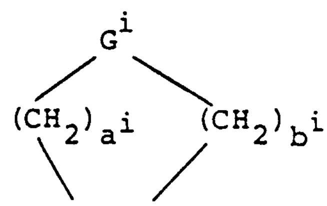

ist, worin $G^i$ Sauerstoff, Schwefel oder $CH_2$ ist, $a^i$ 2, 3 oder 4 ist, und $b^i$ 1, 2, 3, 4 oder 5 ist, mit der Maßgabe, daß die Summe von $a^i$ und $b^i$ 5, 6 oder 7 ist, oder $G^i$ $CH_2$ ist, $a^i$ 0, 1, 2 oder 3 ist, $b^i$ 0, 1, 2 oder 3 ist, mit der Maßgabe, daß die Summe von $a^i$ und $b^i$ 1, 2 oder 3 ist, mit der Maßgabe, daß die Summe von $a^i$ und $b^i$ nur 1, 2 oder 3 sein kann, wenn $R^{1i}$ mit Aralkylthio oder Aralkyloxy substituiertes Niederalkyl ist;

$D^i$

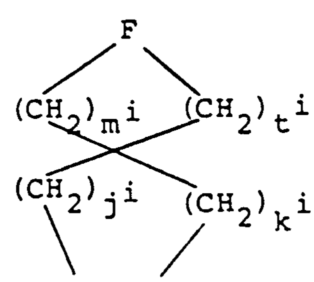

ist, worin $F^i$ O oder S ist, $j^i$ 0, 1 oder 2 ist, und $k^i$ 0, 1 oder 2 ist, mit der Maßgabe, daß die Summe von $j^i$ und $k^i$ 1, 2 oder 3 sein muß, und $m^i$ 1, 2 oder 3 ist, und $t^i$ 1, 2 oder 3 ist, mit der Maßgabe, daß die Summe von $m^i$ und $t^i$ 2, 3 oder 4 sein muß;
$E^i$

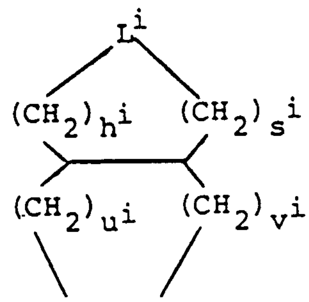

ist, worin $L^i$ O oder S ist, $u^i$ 0, 1 oder 2 ist, und $v^i$ 0, 1 oder 2 ist, mit der Maßgabe, daß die Summe von $u^i$ und $v^i$ 1 oder 2 sein muß, und $h^i$ 1 oder 2 ist, und $s^i$ 1 oder 2 ist, mit der Maßgabe, daß die Summe von $h^i$ und $s^i$ 2 oder 3 sein muß;
(j) mit der Formel

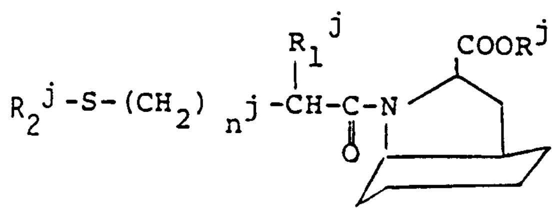

worin $R^j$ Wasserstoff oder Niederalkyl ist; $R_1{}^j$ Wasserstoff, Niederalkyl oder Benzyl ist; $R_2{}^j$ Wasserstoff oder

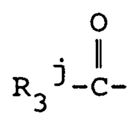

ist, worin $R_3{}^j$ Niederalkyl, Heteroaryl, enthaltend 4 bis 9 Kohlenstoff-Atome und ein oder zwei Stickstoff-, Sauerstoff- oder Schwefel-Atome, ist; Phenyl, substituiertes Phenyl mit 1 oder 2 Substituenten, ausgewählt aus Fluor, Chlor, Brom, Niederalkyl oder -alkoxy; und $n^j$ 0 oder 1 ist; wobei Niederalkyl und Niederalkoxy lineare oder verzweigte Gruppen, enthaltend 1 bis 4 Kohlenstoff-Atome, einschließen, und pharmazeutisch annehmbare Salze der Verbindungen, wenn $R^j$ Wasserstoff ist und wenn $R_3{}^j$ Heteroaryl, enthaltend 1 oder 2 Stickstoff-Atome, ist, werden offenbart;
(k) mit der Formel

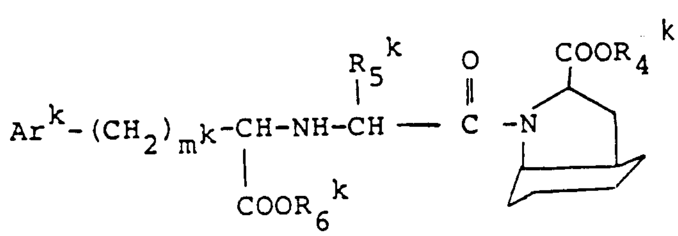

worin $R_4{}^k$ Wasserstoff oder Niederalkyl ist; $R_5{}^k$ Wasserstoff, Niederalkyl oder Benzyl ist; $R_6{}^k$ Wasserstoff oder Niederalkyl ist; $Ar^k$ Phenyl ist oder Phenyl, das substituiert ist mit 1 oder 2

Substituenten, ausgewählt aus Fluor, Chlor, Brom, Niederalkyl, Niederalkoxy, Hydroxy oder Amino; und m 0 bis 3 ist; wobei Niederalkyl und Niederalkoxy - linear oder verzweigt - 1 bis 4 Kohlenstoff-Atome enthalten; und pharmazeutisch annehmbare Salze davon;

(l) mit der Formel

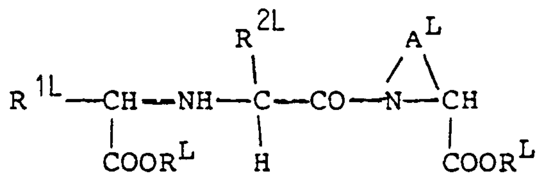

(I)$^L$

und

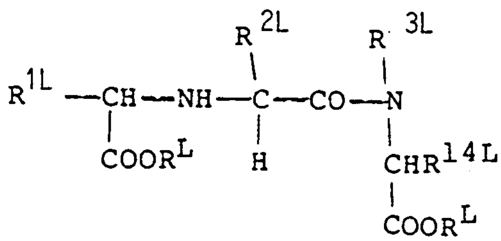

(Ia)$^L$

worin

$R^L$ und $R^{2L}$ unabhängig voneinander Wasserstoff, Niederalkyl, Aralkyl oder Aryl sind;

$R^{1L}$ Wasserstoff ist; verzweigt- oder geradkettiges $C_1$-$C_{12}$-Alkyl und -Alkenyl; $C_3$-$C_9$-Cycloalkyl und Benzokondensiertes Alkyl; substituiertes Niederalkyl, worin die Substituenten Halogen, Hydroxy, Niederalkoxy, Aryloxy, Amino, Mono- oder Diniederalkylamino, Acylamino, Arylamino, Guanidino, Mercapto, Niederalkylthio, Arylthio, Carboxy, Carboxamido oder Niederalkoxycarbonyl sind; Aryl; substituiertes Aryl, worin die Substituenten Niederalkyl, Niederalkoxy oder Halogen sind; Ar-niederalkyl; Ar-niederalkenyl; Heteroar-niederalkyl; Heteroar-niederalkenyl; substituiertes Ar-niederalkyl, substituiertes Ar-niederalkenyl, substituiertes Heteroar-niederalkyl oder substituiertes Heteroar-niederalkenyl, worin die Aryl- und Heteroaryl-Substituenten Halogen, Dihalogen, Niederalkyl, Hydroxy, Niederalkoxy, Amino, Aminoniederalkyl, Acylamino, Mono- oder Diniederalkylamino, Carboxyl, Halogen-niederalkyl, Nitro, Cyan oder Sulfonamido sind, und worin der Niederalkyl-Teil von Ar-niederalkyl substituiert sein kann durch Amino, Acylamino oder Hydroxyl;

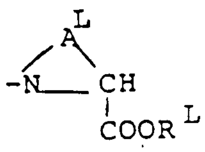

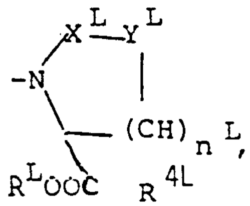

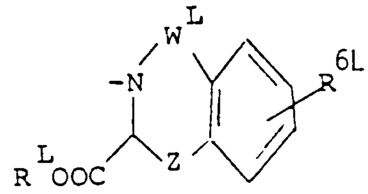

oder

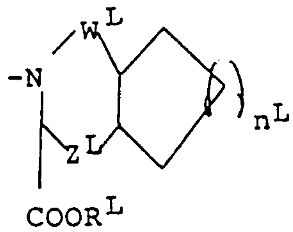

ist,

worin

$X^L$ und $Y^L$ zusammengenommen $-CH_2-CH_2-$;

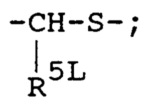

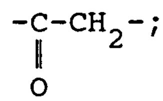

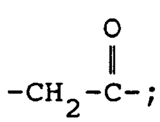

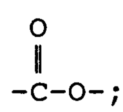

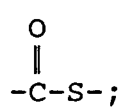

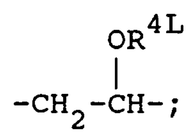

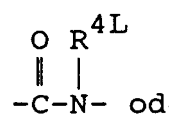

oder

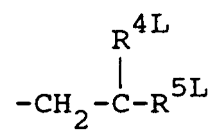

sind;

$R^{4L}$ Wasserstoff, Niederalkyl, Aryl, substituiertes Aryl ist;

$R^{5L}$ Wasserstoff, Niederalkyl, Aryl oder substituiertes Aryl ist;

$n^L$ 1 bis 3 ist;

$W^L$ nicht vorhanden,

$-CH_2-$ oder

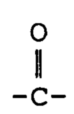

ist;

$Z^L$ $-(CH_2)_mL$ ist, worin $m^L$ 0 bis 2 ist, vorausgesetzt, daß $m^L$ nicht O ist und $W^L$ nicht gleichzeitig abwesend sein darf; und

$R^{6L}$ Wasserstoff, Niederalkyl, Halogen oder $OR^{4L}$ ist;

$R^{2L}$ $—(CH_2)_rL—B^L—(CH_2)_sL—NR^{7L}R^{15L}$ ist,

worin

$r^L$ und $s^L$ unabhängig voneinander 0 bis 3 sind;

$B^L$ nicht vorhanden, -O-, -S- oder $-NR^{8L}$ ist; worin $R^{8L}$ Wasserstoff, Niederalkyl, Alkanoyl oder Aroyl ist; und $R^{7L}$

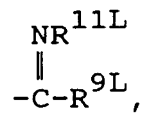, 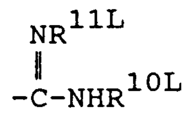 oder 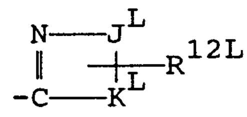

ist,
worin
$R^{9L}$ Niederalkyl, Aralkyl, Aryl, Heteroaryl oder Heteroar-niederalkyl ist und diese Gruppen substituiert sind durch Hydroxy, Niederalkoxy oder Halogen, Carboxyl, Carboxamido, Nitromethenyl;
$R^{10L}$ Wasserstoff, Niederalkyl, Aryl oder Amidino ist;
$R^{11L}$ Wasserstoff, Niederalkyl, Cyan, Amidino, Aryl, Aroyl, Niederalkanoyl,

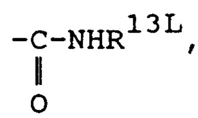, 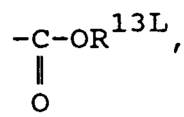,

$-NO_2$, $-SO_2NH_2$ oder $SO_2R^{13}L$ ist;
$R^{12}L$ Wasserstoff, Niederalkyl, Halogen, Aralkyl, Amino, Cyan, Mono- oder Diniederalkylamino oder $OR^{4L}$ ist;
$R^{13L}$ Wasserstoff, Niederalkyl oder Aryl ist;
$R^{15L}$ Wasserstoff, Niederalkyl, Aralkyl oder Aryl ist;

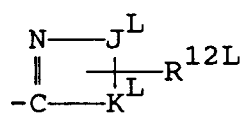

einen basischen Heteroring mit 5 bis 6 Atomen oder Benzo-kondensierte Analoga desselben bildet, gegebenenfalls 1-3 N-Atome, ein Sauerstoff-, ein Schwefel-Atom, eine SO- oder eine $SO_2$-Gruppe enthaltend, gegebenenfalls substituiert durch Amino-, Niederalkylamino-, Diniederalkylamino-, Niederalkoxy- oder Aralkyl-Gruppen;
$R^{3L}$ $C_{3-8}$-Cycloalkyl und Benzo-kondensiertes $C_{3-8}$-Cycloalkyl, Perhydrobenzo-kondensiertes $C_{3-8}$-Cycloalkyl, Aryl, substituiertes Aryl, Heteroaryl, substituiertes Heteroaryl ist;
$R^{14L}$ Wasserstoff oder Niederalkyl ist; und ein pharmazeutisch annehmbares Salz davon;
(m) mit der Formel

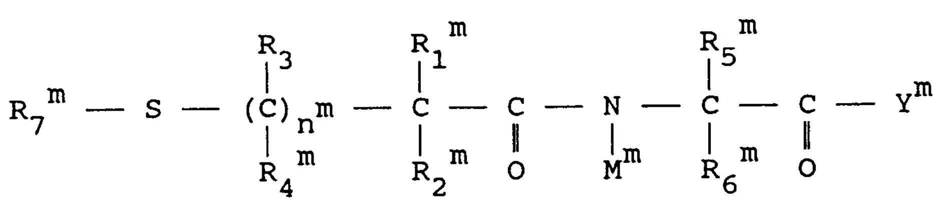

worin
$R_1{}^m$, $R_2{}^m$, $R_3{}^m$, $R_4{}^m$, $R_5{}^m$ und $R_6{}^m$ Wasserstoff, Alkyl, Alkenyl, Alkinyl, Phenylalkyl und Cycloalkyl sind und gleich oder verschieden sein können;
$n^m$ eine ganze Zahl von 0 bis einschließlich 4 ist;
$M^m$ Alkenyl, Alkinyl, Cycloalkyl, Cycloalkylalkyl, Polycycloalkyl, Polycycloalkylalkyl, Aryl, Aralkyl, Heteroaryl, Heteroarylalkyl, Heterocycloalkyl, Heterocycloalkylalkyl, kondensiertes Arylcycloalkyl, kondensiertes Arylcycloalkylalkyl, kondensiertes Heteroarylcycloalkyl, kondensiertes Heteroarylcycloalkylalkyl, Alkoxyalkyl, Alkylthioalkyl, Alkylaminoalkyl oder Dialkylaminoalkyl ist;
$Y^m$ Hydroxy, Alkoxy, Amino oder substituiertes Amino, Aminoalkanoyl, Aryloxy, Aminoalkoxy oder Hydroxyalkoxy ist; und

$R_7{}^m$ Wasserstoff, Alkanoyl, Carboxyalkanoyl, Hydroxyalkanoyl, Aminoalkanoyl, Cyan, Amidino, Carbalkoxy, $Z^m$S oder

$Z^m$SCO-

ist, worin $Z^m$ Wasserstoff, Alkyl, Hydroxyalkyl oder der Rest

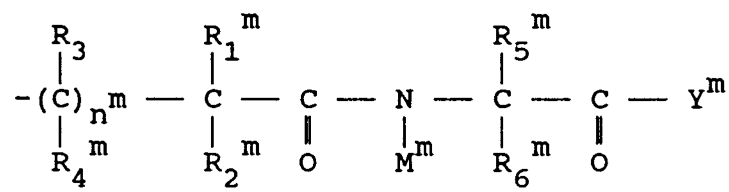

worin
$R_1{}^m$, $R_2{}^m$, $R_3{}^m$, $R_4{}^m$, $R_5{}^m$, $R_6{}^m$, $n^m$, $M^m$ und $Y^m$ wie vorstehend beschrieben sind, und worin $Y^m$ Hydroxy ist; ihre nicht-toxischen, pharmazeutisch annehmbaren Alkalimetall-, Erdalkalimetall- und Amin-Salze; oder
(n) mit der Formel

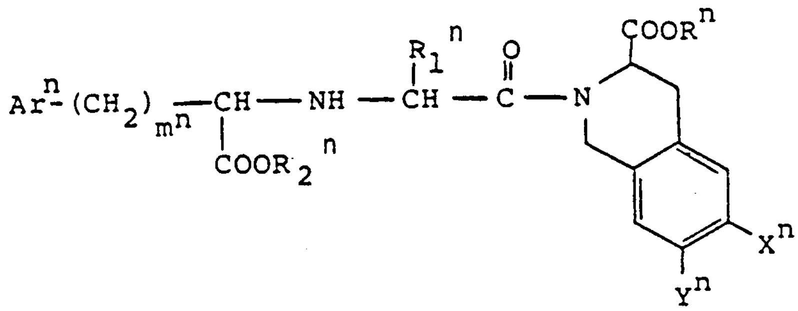

worin $R^n$ Wasserstoff, Niederalkyl oder Aralkyl ist; $R_1{}^n$ Wasserstoff, Niederalkyl oder Benzyl ist; $R_2{}^n$ Wasserstoff oder Niederalkyl ist, und Ar Phenyl ist oder Phenyl, das substituiert ist mit 1 oder 2 Substituenten, ausgewählt aus Fluor, Chlor, Brom, Niederalkyl, Niederalkoxy, Hydroxy oder Amino; X und Y unabhängig voneinander Wasserstoff, Niederalkyl, Niederalkoxy, Niederalkylthio, Niederalkylsulfinyl, Niederalkylsulfonyl, Hydroxy sind, oder X und Y zusammen Methylendioxy sind; m 0 bis 3 ist; und die pharmazeutisch annehmbaren Salze davon;
vorzugsweise mit der Formel wie in Abschnitt (i) definiert, insbesondere
7-{N-[1(S)-Carboxy-3-phenylpropyl]-(S)-alanyl}-1,4-dithia-7-azasprio[4.4]nonan-8(S)-carbonsäure;
(o) mit der Formel

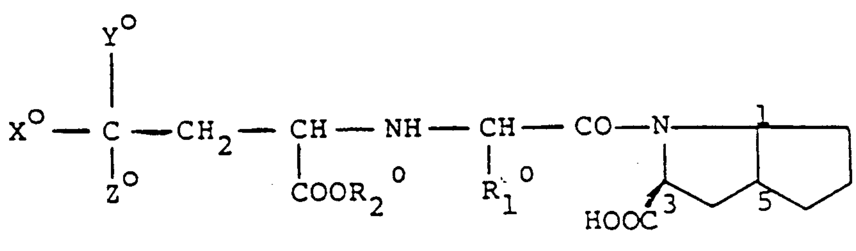

worin die Wasserstoff-Atome an den Ringpositionen 1 und 5 cis zueinander stehen und die 3-Carboxy-Gruppe endo-Orientierung aufweist;
$R_1{}^o$ H, Allyl, Vinyl oder die Seitenkette einer gegebenenfalls geschützten, natürlich vorkommenden $\alpha$-Aminosäure ist;
$R_2{}^o$ H, 1-6C-Alkyl, 2-6C-Alkenyl oder Aryl(1-4C)alkyl ist;

$Y^o$ H oder OH ist, und $Z^o$ H ist, oder $Y^o$ und $Z^o$ zusammen Sauerstoff sind;
$X^o$ 1-6C-Alkyl, 2-6C-Alkenyl, 5-9C-Cycloalkyl, 6-12C-Aryl [gegebenenfalls substituiert durch ein bis drei 1-4C-Alkyl oder Alkoxy, OH, Halogen, Nitro, Amino (gegebenenfalls substituiert durch ein oder zwei 1-4C-Alkyl) oder Methylendioxy] oder Indol-3-yl ist;
(p) mit der Formel

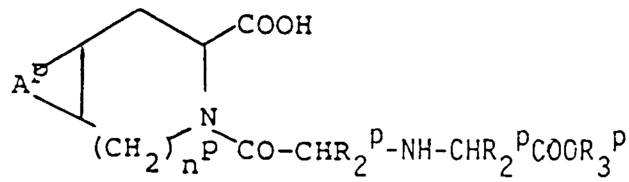

worin
$n^p$ 0 oder 1 ist;

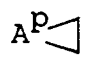

ein Benzol- oder Cyclohexan-Ring ist;
$R_1{}^p$ und $R_2{}^p$ jeweils 1-6C-Alkyl, 2-6C-Alkenyl, 5-7C-Cycloalkyl, 5-7C-Cycloalkenyl, 7-12C-Cycloalkyalkyl, gegebenenfalls teilweise hydriertes 6-10C-Aryl, 7-14C-Aralkyl oder 5-7gliedriges monocyclisches oder 8-10gliedriges bicyclisches Heterocyclyl, enthaltend 1 oder 2 S- oder O- und/oder 1-4 N-Atome; alle Gruppen $R_1{}^p$ und $R_2{}^p$ gegebenenfalls substituiert sind;
$R_3{}^p$ H, 1-6C-Alkyl, 2-6C-Alkenyl oder 7-14C-Aralkyl ist.

3. Zusammensetzung nach Anspruch 1 oder 2, worin der $\beta$-adrenerge Blocker Atenolol, Metoprolol, Nadolol, Pindolol, Propranolol, Timolol, Labetalol, Betaxolol, Carteolol, Dilevolol oder Butonolol ist, vorzugsweise Timolol.

4. Zusammensetzung nach irgendeinem der Ansprüche 1 bis 3, wobei pro Menge von 0,005 $\mu$g bis 50 $\mu$g des ACE-Inhibitors eine Menge von 5 $\mu$g bis 500 $\mu$g des $\beta$-adrenergen Blockers vorhanden ist, wobei die folgenden Anteile bevorzugt sind:
(a) 50 $\mu$g bis 250 $\mu$g (insbesondere 50 $\mu$g bis 125 $\mu$g) des $\beta$-adrenergen Blockers zu 0,025 $\mu$g bis 5 $\mu$g (insbesondere 0,05 $\mu$g bis 0,5 $\mu$g) des ACE-Inhibitors;
(b) 5 $\mu$g bis 125 $\mu$g (insbesondere 50 $\mu$g) des $\beta$-adrenergen Blockers zu 0,005 $\mu$g bis 1 $\mu$g (insbesondere 0,05 $\mu$g bis 0,5 $\mu$g) des ACE-Inhibitors; und
(c) 25 $\mu$g bis 500 $\mu$g (insbesondere 50 $\mu$g bis 250 $\mu$g) des $\beta$-adrenergen Blockers zu 0,005 $\mu$g bis 1 $\mu$g (insbesondere 0,005 $\mu$g bis 0,05 $\mu$g) des ACE-Inhibitors.

5. Kit, das in getrennten Behältnissen pharmazeutische Zusammensetzungen für die Kombinationstherapie umfaßt, zur Herabsetzung und/oder Kontrolle des mit Glaukom bei Säugern verbundenen erhöhten Intraokulardrucks, das in dem einen Behältnis eine topische ophthalmologische pharmazeutische Zusammensetzung umfaßt, umfassend einen ophthalmologisch annehmbaren Inhibitor für Angiotensin umwandelndes Enzym, und im zweiten Behältnis eine topische ophthalmologische pharmazeutische Zusammensetzung, umfassend einen ophthalmologisch annehmbaren $\beta$-adrenergen Blocker.

6. Kit nach Anspruch 5, worin der ACE-Inhibitor ein ACE-Inhibitor wie in Anspruch 2 definiert ist, vorzugsweise 7-{N-[1(S)-Carboxy-3-phenylpropyl]-(S)-alanyl}-1,4-dithia-7-azasprio[4.4]nonan-8(S)-carbonsäure.

7. Kit nach Anspruch 5 oder 6, worin der $\beta$-adrenerge Blocker Atenolol, Metoprolol, Nadolol, Pindolol, Propranolol, Timolol, Labetalol, Betaxolol, Carteolol, Dilevolol oder Bunolol ist, vorzugsweise Timolol.

8. Kit nach irgendeinem der Ansprüche 5 bis 7, wobei pro Menge von 0,005 $\mu$g bis 50 $\mu$g des ACE-Inhibitors eine Menge von 5 $\mu$g bis 500 $\mu$g des $\beta$-adrenergen Blockers vorhanden ist, wobei die

folgenden Anteile bevorzugt sind:

(a) 50 µg bis 250 µg (insbesondere 50 µg bis 125 µg) des β-adrenergen Blockers zu 0,025 µg bis 5 µg (insbesondere 0,05 µg bis 0,5 µg) des ACE-Inhibitors;

(b) 5 µg bis 125 µg (insbesondere 50 µg) des β-adrenergen Blockers zu 0,005 µg bis 1 µg (insbesondere 0,05 µg bis 0,5 µg) des ACE-Inhibitors; und

(c) 25 µg bis 500 µg (insbesondere 50 µg bis 250 µg) des β-adrenergen Blockers zu 0,005 µg bis 1 µg (insbesondere 0,005 µg bis 0,05 µg) des ACE-Inhibitors.

**9.** Kit, umfassend (a) eine topische ophthalmologische pharmazeutische Zusammensetzung, umfassend einen ophthalmologisch annehmbaren Inhibitor für Angiotensin umwandelndes Enzym, und (b) eine topische ophthalmologische pharmazeutische Zusammensetzung, umfassend einen ophthalmologisch annehmbaren β-adrenergen Blocker, zusammen mit (c) Instruktionen für die Anwendung der Zusammensetzungen für die Kombinationstherapie zur Herabsetzung und/oder Kontrolle des mit Glaukom bei Säugern verbundenen erhöhten Intraokulardrucks.

**10.** Kit nach Anspruch 9, worin der ACE-Inhibitor ein ACE-Inhibitor wie in Anspruch 2 definiert ist, vorzugsweise 7-{N-[1(S)-Carboxy-3-phenylpropyl]-(S)-alanyl}-1,4-dithia-7-azasprio[4.4]nonan-8(S)-carbonsäure.

**11.** Kit nach Anspruch 9 oder 10, worin der β-adrenerge Blocker Atenolol, Metoprolol, Nadolol, Pindolol, Propranolol, Timolol, Labetalol, Betaxolol, Carteolol, Dilevolol oder Bunolol ist, vorzugsweise Timolol.

**12.** Kit nach irgendeinem der Ansprüche 9 bis 11, wobei pro Menge von 0,005 µg bis 50 µg des Inhibitors für Angiotensin umwandelndes Enzym eine Menge von 5 µg bis 500 µg des β-adrenergen Blockers vorhanden ist, wobei die folgenden Anteile bevorzugt sind:

(a) 50 µg bis 250 µg (insbesondere 50 µg bis 125 µg) des β-adrenergen Blockers zu 0,025 µg bis 5 µg (insbesondere 0,05 µg bis 0,5 µg) des ACE-Inhibitors;

(b) 5 µg bis 125 µg (insbesondere 50 µg) des β-adrenergen Blockers zu 0,005 µg bis 1 µg (insbesondere 0,05 µg bis 0,5 µg) des ACE-Inhibitors; und

(c) 25 µg bis 500 µg (insbesondere 50 µg bis 250 µg) des β-adrenergen Blockers zu 0,005 µg bis 1 µg (insbesondere 0,005 µg bis 0,05 µg) des ACE-Inhibitors.

**13.** Verwendung eines ophthalmologisch annehmbaren ACE-Inhibitors und eines ophthalmologisch annehmbaren β-adrenergen Blockers zur Herstellung von Medikamenten für die Kombinationstherapie zur Herabsetzung und/oder Kontrolle des mit Glaukom bei Säugern verbundenen erhöhten Intraokulardrucks.

**14.** Verwendung nach Anspruch 13, wobei der ACE-Inhibitor ein ACE-Inhibitor wie in Anspruch 2 definiert ist, vorzugsweise 7-{H-[1(S)-Carboxy-3-phenylpropyl]-(S)-alanyl}-1,4-dithia-7-azasprio[4.4]nonan-8(S)-carbonsäure.

**15.** Verwendung nach Anspruch 13 oder 14, wobei der β-adrenerge Blocker Atenolol, Metoprolol, Nadolol, Pindolol, Propranolol, Timolol, Labetalol, Betaxolol, Carteolol, Dilevolol oder Bunolol ist, vorzugsweise Timolol.

**16.** Verwendung nach irgendeinem der Ansprüche 13 bis 15, wobei pro Menge von 0,005 µg bis 50 µg des ACE-Inhibitors eine Menge von 5 µg bis 500 µg des β-adrenergen Blockers verwendet wird, wobei die folgenden Anteile bevorzugt sind:

(a) 50 µg bis 250 µg (insbesondere 50 µg bis 125 µg) des β-adrenergen Blockers zu 0,025 µg bis 5 µg (insbesondere 0,05 µg bis 0,5 µg) des ACE-Inhibitors;

(b) 5 µg bis 125 µg (insbesondere 50 µg) des β-adrenergen Blockers zu 0,005 µg bis 1 µg (insbesondere 0,05 µg bis 0,5 µg) des ACE-Inhibitors; und

(c) 25 µg bis 500 µg (insbesondere 50 µg bis 250 µg) des β-adrenergen Blockers zu 0,005 µg bis 1 µg (insbesondere 0,005 µg bis 0,05 µg) des ACE-Inhibitors.

**17.** Verfahren zur Herstellung einer topischen, ophthalmologisch annehmbaren Zusammensetzung, brauchbar zur Herabsetzung und/oder Kontrolle des mit Glaukom verbundenen erhöhten Intraokulardrucks, umfassend das Mischen eines ophthalmologisch annehmbaren Inhibitors für Angiotensin umwandelndes Enzym und eines ophthalmologisch annehmbaren β-adrenergen Blockers mit einem ophthalmologisch annehmbaren Träger zur topischen Anwendung.

**18.** Verfahren nach Anspruch 17, wobei der ACE-Inhibitor ein ACE-Inhibitor wie in Anspruch 2 definiert ist, vorzugsweise 7-{N-[1(S)-Carboxy-3-phenylpropyl]-(S)-alanyl}-1,4-dithia-7-azasprio[4.4]nonan-8(S)-carbonsäure.

**19.** Verfahren nach Anspruch 17 oder 18, wobei der β-adrenerge Blocker Atenolol, Metoprolol, Nadolol, Pindolol, Propranolol, Timolol, Labetalol, Betaxolol, Carteolol, Dilevolol oder Bunolol ist, vorzugsweise Timolol.

**20.** Verfahren nach irgendeinem der Ansprüche 17 bis 19, wobei pro Menge von 0,005 µg bis 50 µg des ACE-Inhibitors eine Menge von 5 µg bis 500 µg des β-adrenergen Blockers verwendet wird, wobei die folgenden Anteile bevorzugt sind:
(a) 50 µg bis 250 µg (insbesondere 50 µg bis 125 µg) des β-adrenergen Blockers zu 0,025 µg bis 5 µg (insbesondere 0,05 µg bis 0,5 µg) des ACE-Inhibitors;
(b) 5 µg bis 125 µg (insbesondere 50 µg) des β-adrenergen Blockers zu 0,005 µg bis 1 µg (insbesondere 0,05 µg bis 0,5 µg) des ACE-Inhibitors; und
(c) 25 µg bis 500 µg (insbesondere 50 µg bis 250 µg) des β-adrenergen Blockers zu 0,005 µg bis 1 µg (insbesondere 0,005 µg bis 0,05 µg) des ACE-Inhibitors.

**Revendications**

**1.** Composition topique ophtalmologiquement acceptable utile pour réduire et/ou contrôler la pression intra-oculaire élevée associée au glaucome, qui comprend un inhibiteur de l'enzyme de conversion de l'angiotensine ophtalmologiquement acceptable et un bêta bloquant adrénergique ophtalmologiquement acceptable en combinaison ave un support ophtalmologiquement acceptable pour usage topique.
**2.** Composition selon la revendication 1 qui comprend un inhibiteur de l'enzyme de conversion de l'angiotensine ayant pour formule
a)

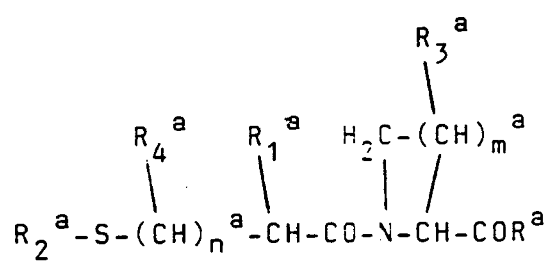

où
$R^a$ est hydroxy, $NH_2$ ou alcoxy inférieur ;
chacun de $R_1{}^a$ et $R_4{}^a$ est hydrogène, alkyle inférieur, phényle ou phényl-alkyle inférieur ;
$R_2{}^a$ est hydrogène, alkyle inférieur, phényle, phényle substitué où le substituant du phényle est halo, alkyle inférieur ou alcoxy inférieur, phénylalkyle inférieur, diphényl-alkyle inférieur , triphényl-alkyle inférieur, alkyl inférieur thiométhyle, phényl-alkyl inférieur thiométhyle, alcanoyl inférieur-amidométhyle,

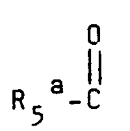 , 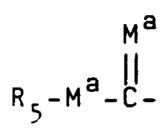 , 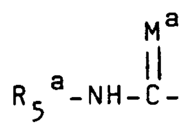 ,

$R_6{}^a$-S- , ou $R_7{}^a$ :
$R_3{}^a$ est hydrogène, hydroxy ou alkyle inférieur ;
$R_5{}^a$ est alkyle inférieur, phényle ou phényl-alkyle inférieur ;

$R_6{}^a$ est alkyle inférieur, phényle, phényle substitué (où le substituant du phényle est halo, alkyle inférieur ou alcoxy inférieur), hydroxy-alkyle inférieur ou amino(carboxy)-alkyle inférieur ;
$R_7{}^a$ est

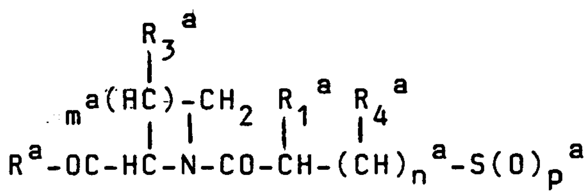

$M^a$ est O ou S ;
$m^a$ est 1 à 3 ;
chacun de $n^a$ et $p^a$ est 0 à 2 ;
(b) ayant pour formule

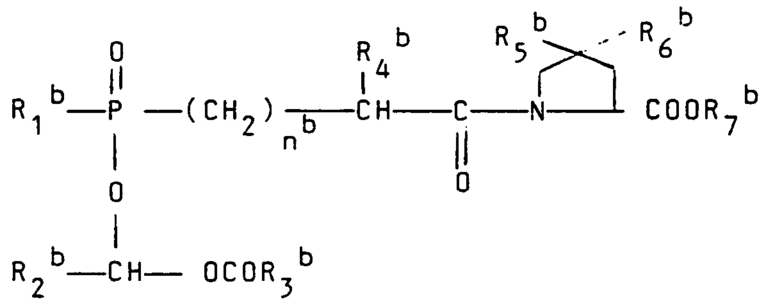

$R_1{}^b$ est alkyle, aryle, aralkyle, cycloalkyle ou cycloalkylalkyle ;
$R_2{}^b$ est cycloalkyle, 3-cyclohexényle ou 2-alkyl-3-cyclohexényle ;
$R_3{}^b$ est alkyle, cycloalkyle ou phényle ;
$R_4{}^b$ est H ou alkyle ;
L'un de $R_5{}^b$ et $R_6{}^b$ est H et l'autre est alkyl-$X^b$-, phényl-$X^b$-alcoxy, phénoxy, phényle, cycloalkyle, alkyle ou phénylalkyle ; ou
$R_5{}^b$ et $R_6{}^b$ forment ensemble -$X^bCH_2CH_2X^b$-, $X^b$ est S, SO ou $SO_2$ ;
$R_7{}^b$ est H ou $CH(R_2{}^b)OCOR_3{}^b$ ;
$n^b$ est 0 ou 1 ;
"aryle" est phényle facultativement substitué par halo, alkyle, alcoxy, alkylthio, OH, alcanoyle, $NO_2$, amino, dialkylamino et/ou $CF_3$ ;
alkyle a 1-10C, cycloalkyle 3-7C, alcoxy 1-8C et alcanoyle 2-9C(atomes) ;
(c) ayant pour formule

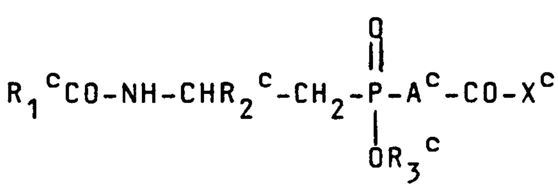

$R^{1c}$ et $R_2{}^c$ sont H, alkyle 1-7C, haloalkyle 1-7C, $(CH_2)_m{}^c$-$D^c$, aminoalkyle 1-7C ou un groupe de la formule

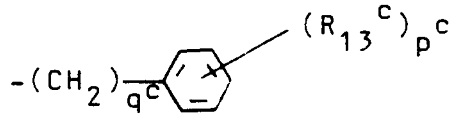

$D^c$ est cycloalkyle, furyle, thiényle ou pyridinyle;
$A^c$ est $(CH_2)_n$-$CHR_{21}{}^c$ , $NR_{22}{}^c$-$CHR_{23}{}^c$ ou O-$CHR_{23}{}^c$ ;
$n^c$ est 0 ou 1 ;
$q^c$ est 0-7 ;
$R_{21}{}^c$ est H, alkyle 1-7C, haloalkyle 1-7C, benzyle ou phénéthyle ;
$R_{22}{}^c$ est H ou alkyle 1-7C ;
$R_{23}{}^c$ est H, alkyle 1-7C, haloalkyle 1-7C ou $(CH_2)_r{}^cD_1{}^c$ ;
$D_1{}^c$ est phényle, 4-hydroxyphényle, 3,4-dihydroxyphényle, 3-indolyle, 4-imidazolyle, $NH_2$, SH, alkylthio 1-7C, guanidino ou $CONH_2$ ;
$X^c$ est un groupe de formule $(II)^c$-$(V)^c$, ou $NR_4{}^c$-$CHR_5{}^c$-$COOR_6{}^c$ ;

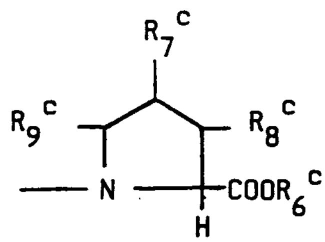
$(II)^c$

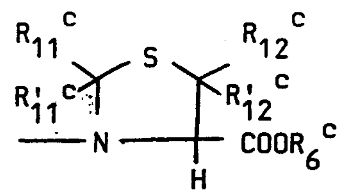
$(III)^c$

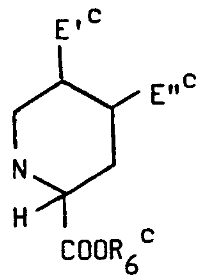
$(IV)^c$

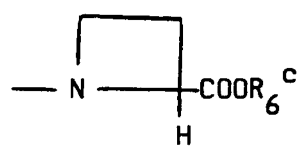
$(V)^c$

où
$R_9{}^c$ et $R_8{}^c$ sont H et $R_7{}^c$ est tel que défini ci-dessous ;
$R_9{}^c$ et $R_7{}^c$ sont H et $R_8{}^c$ est tel que défini ci-dessous ;
$R_7{}^c$ et $R_8{}^c$ sont H et $R_9{}^c$ est tel que défini ci-dessous ;
$R_9{}^c$ et $R_8{}^c$ sont H et -$CHR_7{}^c$ est remplacé par -$CR_{10}{}^cR_{10}{}^c$ ;
$R_9{}^c$ est H et $R_7{}^c$ + $R_8{}^c$ forment une double liaison ;
$R_9{}^c$ et $R_7{}^c$ + $R_8{}^c$ forment un noyau benzène fusionné ; ou
$R_8{}^c$ est H et $R_9{}^c$ + $R_7{}^c$ forment un noyau benzène fusionné ;
$E'^c$ et $E''^c$ sont H ou bien $E'^c$ + $E''^c$ forment un noyau benzène fusionné ;
$R_7{}^c$ est H, alkyle 1-7C, halogène, céto, OH, alcanoyl 2-8C-amino, $N_3$, $NH_2$, $NR_{19}{}^cR_{20}{}^c$, $(CH_2)_m{}^c$-$D^c$, O-$CONR_{15}{}^cR_{15}{}^c$, alcoxy 1-7C, alkylthio 1-7C ou bien un groupe de la formule $(VI)^c$, $(VII)^c$ ou $(VIII)^c$:

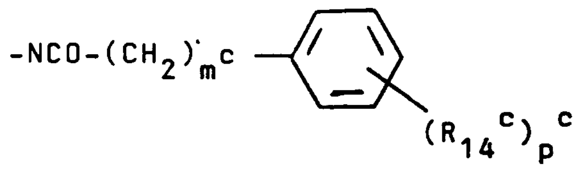

(VI)$^c$

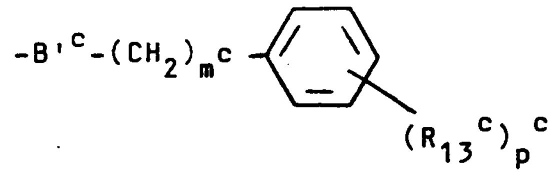

(VII)$^c$

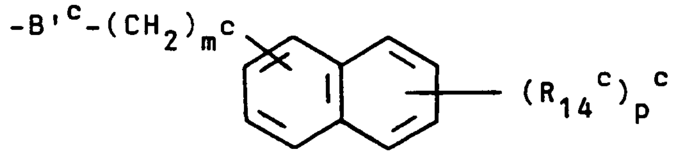

(VIII)$^c$

$B'^c$ est une liaison ou bien c'est O ou S ; $R_8{}^c$ est céto, halogène, O-$CONR_{15}{}^cR_{15}{}^c$, alcoxy 1-7C, alkylthio 1-7C ou bien un groupe (VII)$^c$ ou (VIII)$^c$ où $B'^c$ est O ou S ;

$R_9{}^c$ est céto ou un groupe (VII)$^c$ où $B'^c$ est une liaison ;

$R_{10}{}^c$ est halogène ou $Y^cR_{16}{}^c$ ;

$R_{11}{}^c$, $R'_{11}{}^c$, $R_{12}{}^c$ et $R'_{12}{}^c$ sont H ou alkyle 1-7C, ou bien $R_{11}{}^c$ est un groupe de la formule (IX)$^c$ et les 3 autres sont H ;

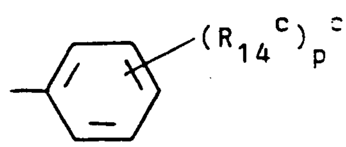

(IX)$^c$

$R_{13}{}^c$ est H, alkyle 1-4C, alcoxy 1-4C, alkylthio 1-4C, Cl, Br, F, $CF_3$, OH, Ph, PhO, PhS ou $PhCH_2$ ;

$R_{14}{}^c$ est H, alkyle 1-4C, alcoxy 1-4C, alkylthio 1-4C, Cl, Br, F, $CF_3$ ou OH ;

$m^c$ est 0-3 ;

$p^c$ est 1-3 mais c'est 2 ou 3 uniquement lorsque $R_{13}{}^c$ ou $R_{14}{}^c$ est H, Me, MeO, Cl ou F ;

$R_{15}{}^c$ est H ou alkyle 1-4C ;

$Y^c$ est O ou S ;

$R_{16}{}^c$ est alkyle 1-4C ou bien le groupe (VII)$^c$ où $B'^c$ est une liaison : ou bien les groupes $R_{16}{}^c$ forment un noyau à 5 ou 6 membres où chacun des atomes de C peut être porteur de 1 ou 2 alkyles ayant 1 à 4 atomes de C ;

$R_4{}^c$ est H, alkyle 1-7C, cycloalkyle ou $(CH_2)_r{}^cPh$;

$R_5{}^c$ est H, alkyle 1-7C ou $(CH_2)_r{}^c$-$D_1{}^c$ ;

$r^c$ est 1-4 ;

$R_3{}^c$ et $R_6{}^c$ sont H, alkyle 1-7C, $PhCH_2$, PhCH ou $CHR_{17}c$-O-$COR_{18}{}^c$ ;

$R_{17}{}^c$ est H, alkyle 1-7C ou Ph ;

$R_{18}{}^c$ est H, alkyle 1-7C, alcoxy 1-7C ou Ph ; ou

$R_{17}{}^c$ + $R_{18}{}^c$ forment $(CH_2)_2$, $(CH_2)_3$, -CH=CH- ou o-phénylène ;

$R_{19}{}^c$ est alkyle 1-7C, benzyle ou phénétyle ; et

$R_{20}{}^c$ est H ou bien est choisi comme pour $R_{19}{}^c$ ;

(d) ayant pour formule

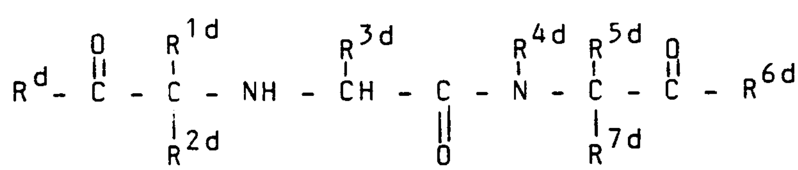

dans laquelle

$R^d$ et $R^{6d}$ sont identiques ou différents et sont hydroxy, alcoxy inférieur, alcénoxy inférieur, dialkylamino inférieur alcoxy inférieur (diméthylaminoéthoxy), acylamino alcoxy inférieur (acétylaminoéthoxy), acyloxy alcoxy inférieur (pivaloyloxyméthoxy), aryloxy comme phénoxy, aralcoxy inférieur comme benzyloxy, aryloxy substitué ou bien aralcoxy inférieur substitué où le substituant est méthyle, halo ou méthoxy, amino, alkylamino inférieur, dialkylamino inférieur, hydroxyamino, aralkylamino inférieur comme benzylamino ;

$R^{1d}$ est hydrogène, alkyle de 1 à 20 atomes de carbone qui comprennent des groupes alkyles ramifiés et cycliques et insaturés (comme allyle), alkyle inférieur substitué où le substituant peut être halo, hydroxy, alcoxy inférieur, aryloxy comme phénoxy, amino, dialkylamino inférieur, acylamino comme acétamido et benzamido, arylamino, guanidino, imidazolyle, indolyle, mercapto, alkylthio inférieur, arylthio comme phénylthio, carboxy ou carboxamido, carboalcoxy inférieur, aryle comme phényle ou naphtyle, aryle substitué comme phényle où le substituant est alkyle inférieur, alcoxy inférieur ou halo, aralkyle inférieur, aralkényle inférieur, hétéroaralkyle inférieur ou hétéroaralkényle inférieur comme benzyle, styryle ou indolyl éthyle, aralkyle inférieur substitué, aralkényle inférieur substitué, hétéroaralkyle inférieur substitué ou hétéroaralkényle inférieur substitué, où le ou les substituants est ou sont halo, dihalo, alkyle inférieur, hydroxy, alcoxy inférieur, amino, aminométhyle, acylamino (acétylamino ou benzoylamino), dialkylamino inférieur, alkylamino inférieur, carboxyle, haloalkyle inférieur, cyano ou sulfonamido ; aralkyle inférieur ou hétéroaralkyle inférieur substitué sur la portion alkyle par amino ou acylamino (acétylamino ou benzoylamino) ;

$R^{2d}$ et $R^{7d}$ sont identiques ou différents et sont hydrogène ou alkyle inférieur ;

$R^{3d}$ est hydrogène, alkyle inférieur, phényl-alkyle inférieur, aminométhyl phényl-alkyle inférieur, hydroxy phényl-alkyle inférieur, hydroxy-alkyle inférieur, acylamino-alkyle inférieur (comme benzoylamino-alkyle inférieur, acétylamino-alkyle inférieur), amino-alkyle inférieur, diméthylaminoalkyle inférieur, halo-alkyle inférieur, guanidino-alkyle inférieur, imidazolyl-alkyle inférieur, indolyl-alkyle inférieur, mercapto-alkyle inférieur, alkylthio inférieur - alkyle inférieur ;

$R^{4d}$ est hydrogène ou alkyle inférieur ;

$R^{5d}$ est hydrogène, alkyle inférieur, phényle, phényl-alkyle inférieur, hydroxy phényl-alkyle inférieur, hydroxy-alkyle inférieur, amino-alkyle inférieur, guanidino-alkyle inférieur, imidazolylalkyle inférieur, indolyl-alkyle inférieur, mercapto-alkyle inférieur ou alkylthio inférieur-alkyle inférieur ;

$R^{4d}$ et $R^{5d}$ peuvent être connectés l'un à l'autre pour former un pont alkylène de 2 à 4 atomes de carbone, un pont alkylène de 2 à 3 atomes de carbone et un atome de soufre, un pont alkylène de 3 à 4 atomes de carbone contenant une double liaison ou un pont alkylène tel que ci-dessus substitué par hydroxy, alcoxy inférieur, alkyle inférieur ou dialkyle inférieur ; et leurs sels acceptables en pharmacie ;

(e) ayant pour formule

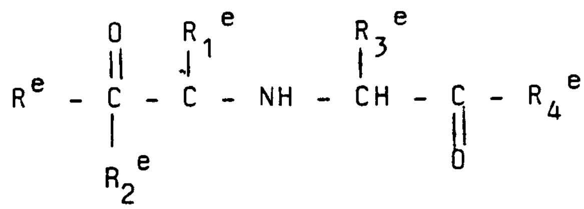

$R_4{}^e$ est une proline substituée de la formule

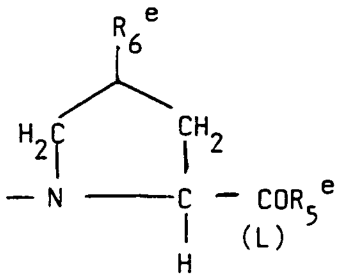 ,

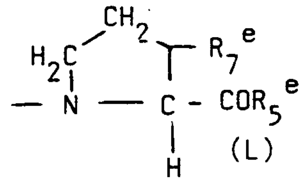 ,

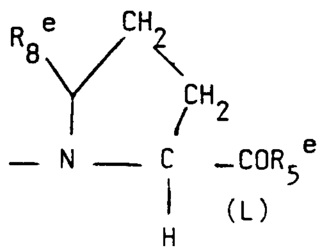 , ou

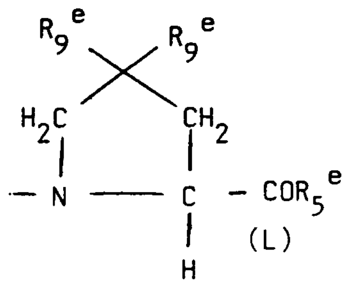 ,

$R_6{}^e$ est halogène, céto, azido,

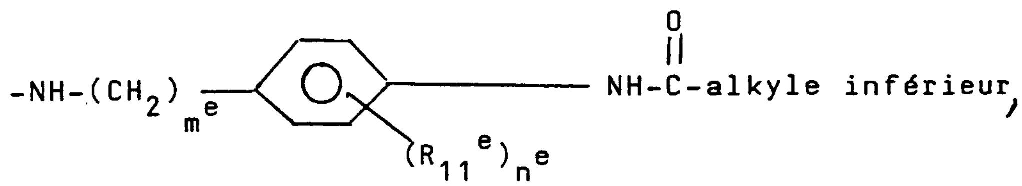

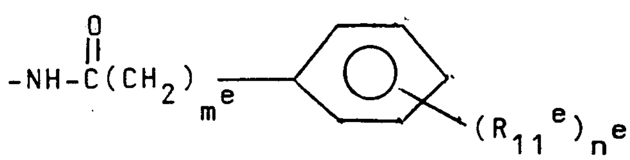

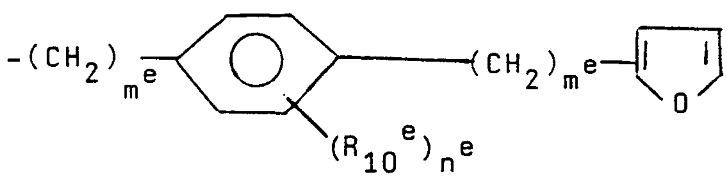 ,

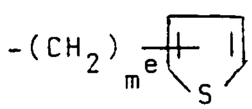 ,

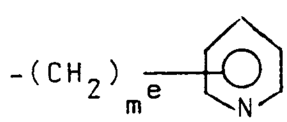 ,

un 1- ou 2-naphtyle de la formule

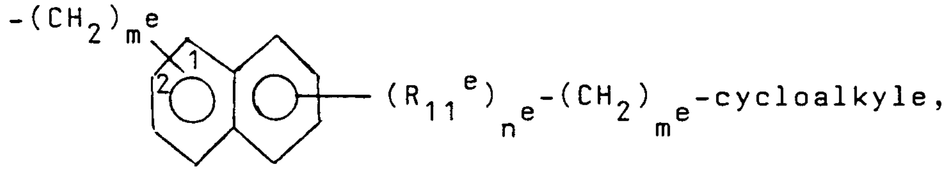

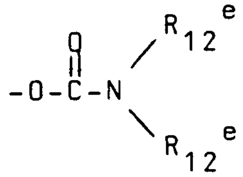
,
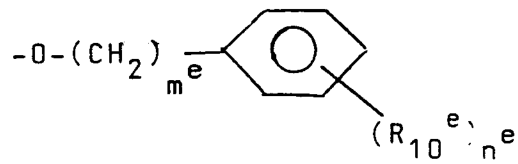

un 1- ou 2-naphtyloxy de la formule

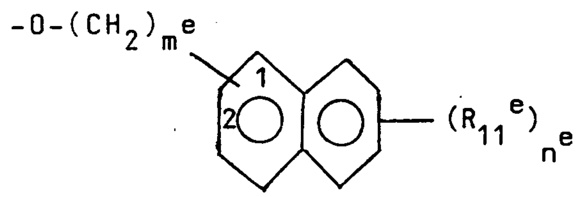

-S-alkyle inférieur,

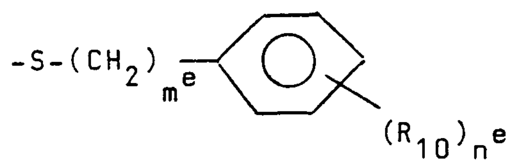

ou un 1- ou 2-naphtylthio de la formule

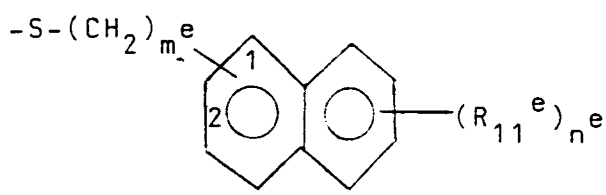

$R_7^e$ est céto, halogène,

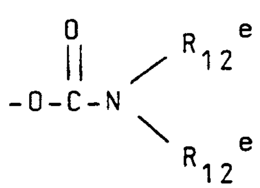
,
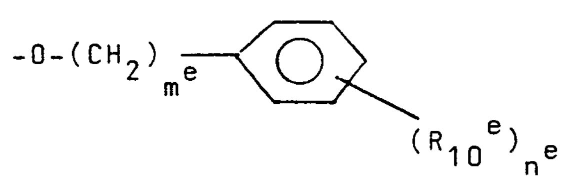

un 1- ou 2-naphtyloxy de la formule

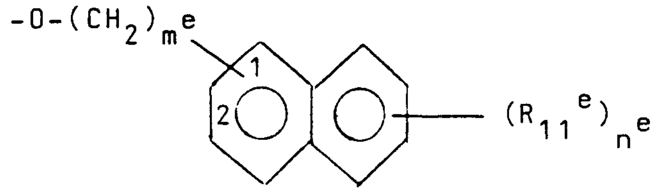

-S-alkyle inférieur

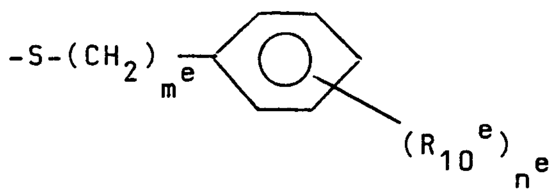

ou un 1- ou 2-naphtylthio de la formule

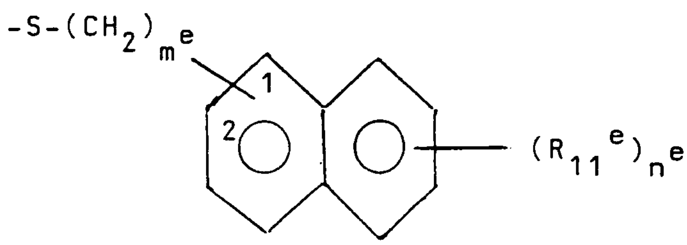

$R_8{}^e$ est céto ou

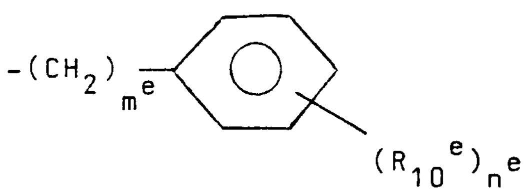

$R_9{}^e$ est halogène ou $-Y^e-R_{13}{}^e$.
$m^e$ est zéro, un, deux ou trois.
$R_{10}{}^e$ est hydrogène, alkyle inférieur de 1 à 4 atomes de carbone, alcoxy inférieur de 1 à 4 atomes de carbone, alkylthio inférieur de 1 à 4 atomes de carbone, chloro, bromo, fluoro, trifluorométhyle, hydroxy, phényle, phénoxy, phénylthio ou phénylméthyle.
$R_{11}{}^e$ est hydrogène, alkyle inférieur de 1 à 4 atomes de carbone, alcoxy inférieur de 1 à 4 atomes de carbone, alkylthio inférieur de 1 à 4 atomes de carbone, bromo, fluoro, trifluorométhyle ou hydroxy.
$n^e$ est un, deux ou trois à condition que n soit supérieur à un uniquement si $R_{10}{}^e$ ou $R_{11}{}^e$ est hydrogène, méthyle, méthoxy, chloro ou fluoro.
$R_{12}{}^e$ est hydrogène ou alkyle inférieur de 1 à 4 atomes de carbone.
$Y^e$ est oxygène ou soufre.
$R_{13}{}^e$ est alkyle inférieur de 1 à 4 atomes de carbone.

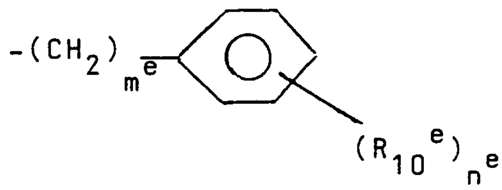

ou les groupes $R_{13}^e$ se joignant pour former un noyau non substitué à 5 ou 6 membres ou bien ledit noyau dans lequel un ou plusieurs des atomes de carbone a un substituant alkyle inférieur de 1 à 4 atomes de carbone ou di(alkyle inférieur) de 1 à 4 atomes de carbone.

$R^e$ et $R_5^e$ sont indépendamment choisis parmi hydroxy, alcoxy inférieur, di (alkyl inférieur)-amino-alcoxy inférieur, comme diméthylaminoéthoxy, alkyl inférieurcarbonyl-amino-alcoxy inférieur, comme acétylaminoéthoxy, alkyl inférieur-carbonyloxy-alcoxy inférieur comme pivaloyloxyméthoxy,

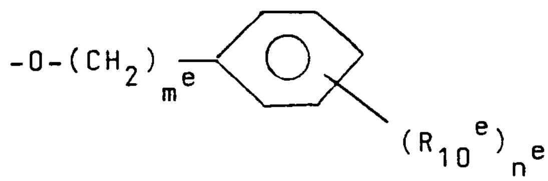

où $m^e$, $n^e$ et $R_{10}^e$ sont tels que définis ci-dessus, amino, alkyl inférieur-amino, di(alkyl inférieur)amino, hydroxyamino, benzylamino ou phénéthylamino.

$R_1^e$ est hydrogène, alkyle inférieur,

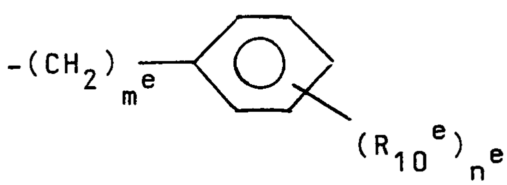

alkyle inférieur halo-substitué, alkyle inférieur hydroxy-substitué, $-(CH_2)_q^e$-cycloalkyle, $-(CH_2)_q^e$-carboxy, $-(CH_2)_q^e$-S-alkyle inférieur,

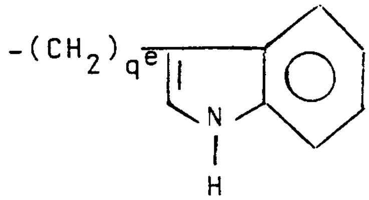 , 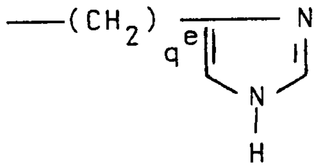 ,

$-(CH_2)_{q^e}$-guanidinyle, $-CH_2)_{q^e}-NH_2$, $-(CH_2)_{q^e}-N$(alkyle inférieur)$_2$ ,

$-(CH_2)_{q^e}-NH-C(O)$-alkyle inférieur,

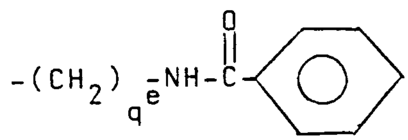 , $-(CH_2)_{q^e}-SH$ ,

$-(CH_2)_{q^e}$-alcoxy inférieur, 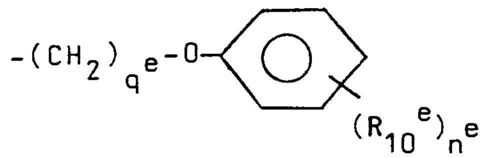

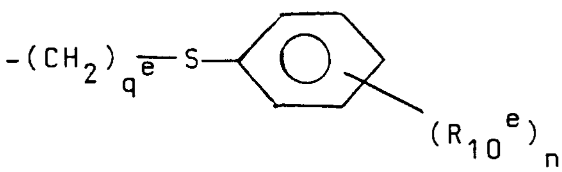 $-(CH_2)_{q^e}-C(O)-NH_2$

$-(CH_2)_{q^e}-C(O)$-alcoxy inférieur, ou

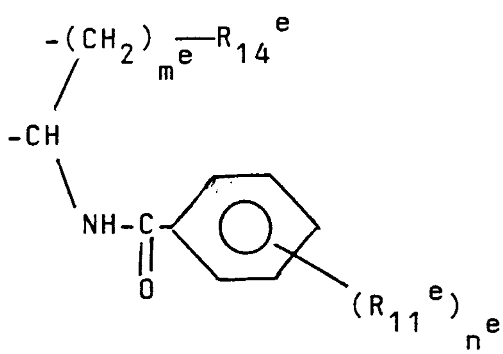

ou $m^e$, $n^e$, $R_{10}{}^e$ et $R_{11}{}^e$ sont tels que définis ci-dessus, $R_{14}{}^e$ est alkyle inférieur, cycloalkyle ou

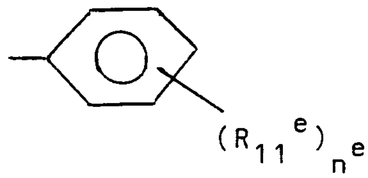

et $q^e$ est un nombre entier de 1 à 4.

$R_2{}^e$ est hydrogène ou alkyle inférieur.

$R_3{}^e$ est hydrogène, alkyle inférieur, alkyle inférieur halo-substitué, alkyle inférieur hydroxysubstitué, $-(CH_2)_q{}^e-NH_2$, $-(CH_2)_q{}^e$-N-(alkyle inférieur)$_2$ , $-(CH_2)_q{}^e$-guanidinyle, $-(CH_2)_q{}^e$-SH, $-(CH_2)_q{}^e$-S-alkyle inférieur,

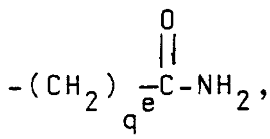 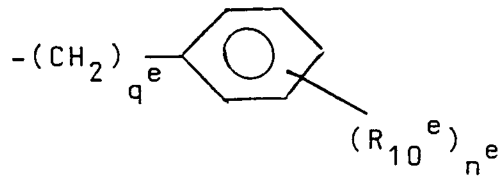 ,

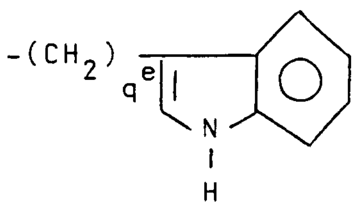 , 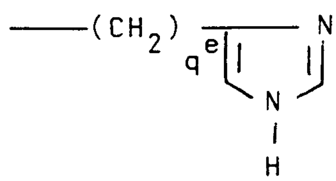 ,

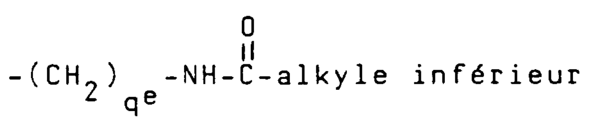 , ou

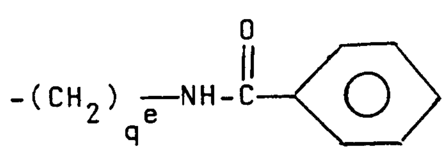

où $R_{10}{}^e$, $n^e$ et $q^e$ sont tels que définis ci-dessus;

(f) ayant pour formule

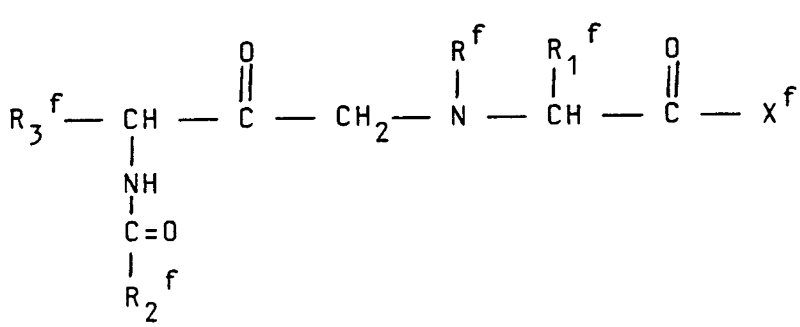

$X^f$ est un amino ou iminoacide de la formule

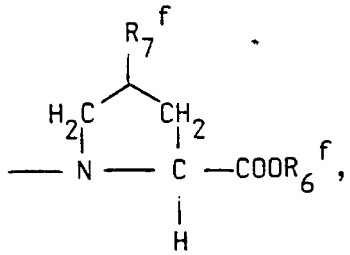

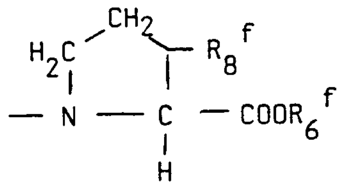
,

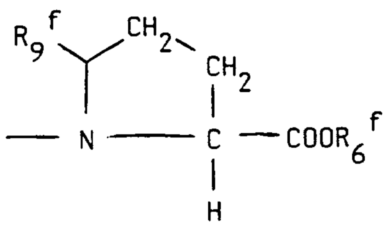

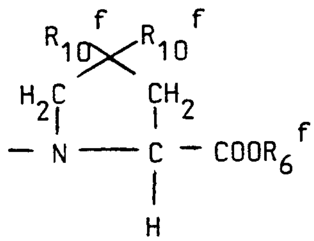
,

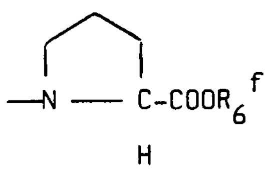
,

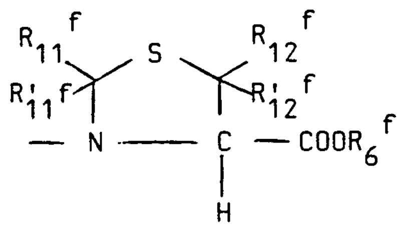
,

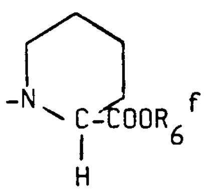
,

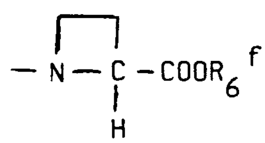
,

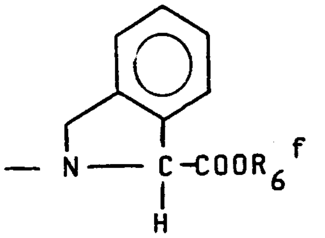 ,

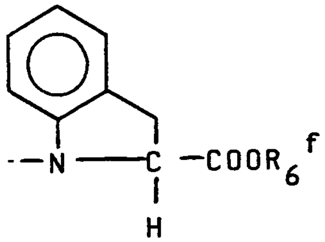 ,

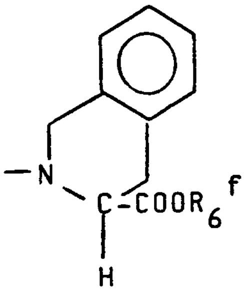 ,

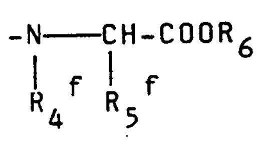 , ou

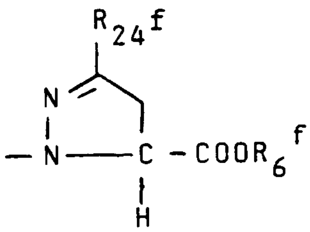 ,

$R_7{}^f$ est hydrogène, alkyle inférieur, halogène, céto, hydroxy.

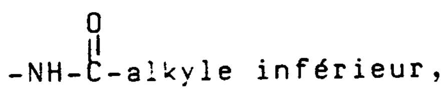

azido, amino,

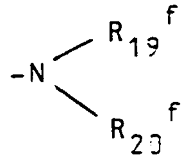 ,

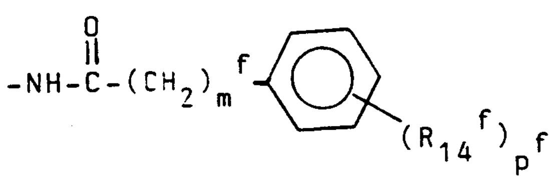 ,

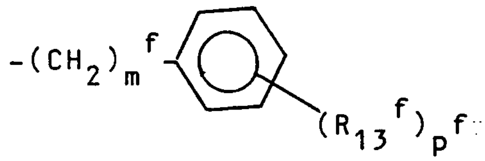 , 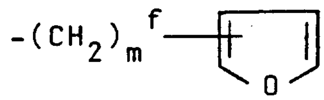 ,

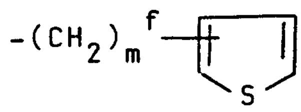 , 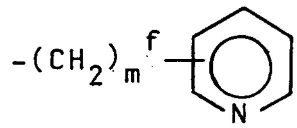 ,

un 1- ou 2-naphtyle de la formule

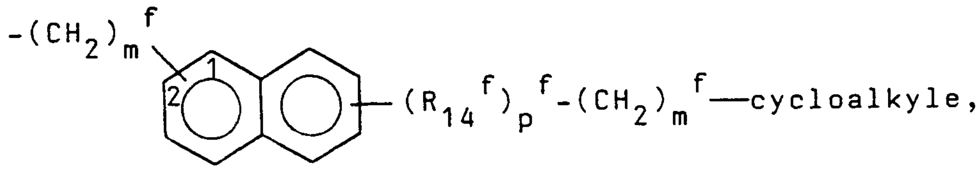

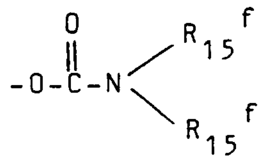 , -O-alkyle inférieur,

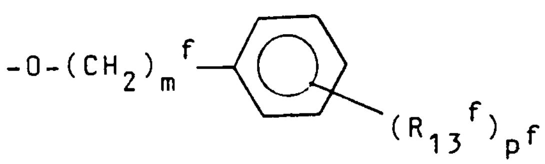 ,

un 1- ou 2-naphtyloxy de la formule

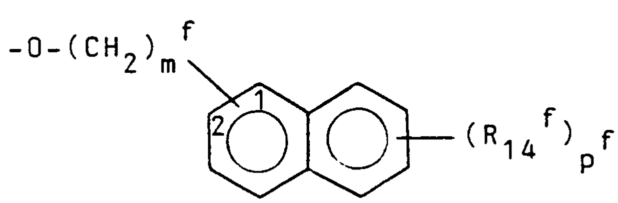 ,

-S-alkyle inférieur,

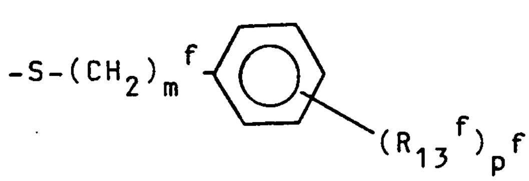 ,

ou un 1- ou 2-naphtylthio de la formule

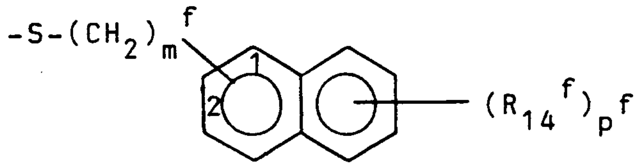

$R_8{}^f$ est céto, halogène,

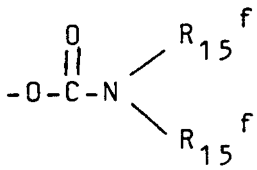

,

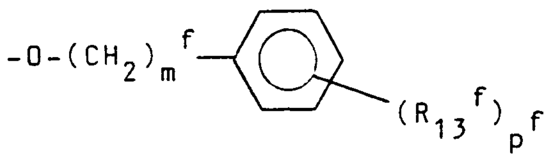

-O-alkyle inférieur, un 1- ou 2-naphtyloxy de la formule

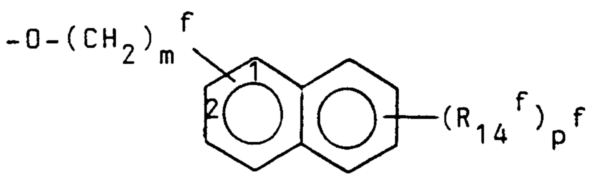

-S-alkyle inférieur,

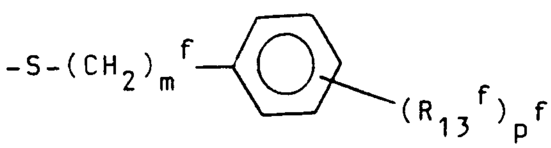

ou bien un 1- ou 2-naphtylthio de la formule

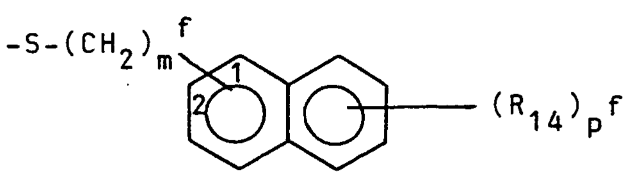

,

$R_9{}^f$ est céto ou

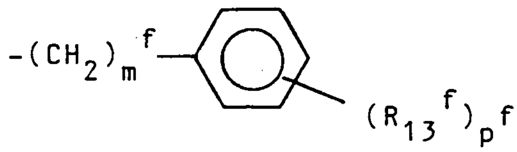

$R_{10}{}^{f}$ est halogène ou $-Y^{f}-R_{16}{}^{f}$

$R_{11}{}^{f}$, $R'_{11}{}^{f}$, $R_{12}{}^{f}$ et $R'_{12}{}^{f}$ sont indépendamment choisis parmi hydrogène et alkyle inférieur ou bien $R'_{11}{}^{f}$, $R_{12}{}^{f}$ et $R'_{12}{}^{f}$ sont hydrogène et $R_{11}{}^{f}$ est

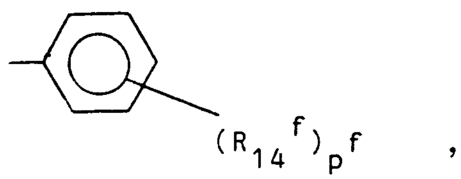

$R_{13}{}^{f}$ est hydrogène, alkyle inférieur de 1 à 4 atomes de carbone, alcoxy inférieur de 1 à 4 atomes de carbone, alkylthio inférieur de 1 à 4 atomes de carbone, chloro, bromo, fluoro, trifluorométhyle, hydroxy, phényle, phénoxy, phénylthio ou phénylméthyle.

$R_{14}{}^{f}$ est hydrogène, alkyle inférieur de 1 à 4 atomes de carbone, alcoxy inférieur de 1 à 4 atomes de carbone, alkylthio inférieur de 1 à 4 atomes de carbone, chloro, bromo, fluoro, trifluorométhyle ou hydroxy.

$m^{f}$ est zéro, un, deux, trois ou quatre.

$p^{f}$ est un, deux ou trois à condition que $p^{f}$ soit plus grand que un uniquement si $R_{13}{}^{f}$ ou $R_{14}{}^{f}$ est hydrogène, méthyle, méthoxy, chloro ou fluoro.

$R_{15}{}^{f}$ est hydrogène ou alkyle inférieur de 1 à 4 atomes de carbone.

$Y^{f}$ est oxygène ou soufre.

$R_{16}{}^{f}$ est alkyle inférieur de 1 à 4 atomes de carbone.

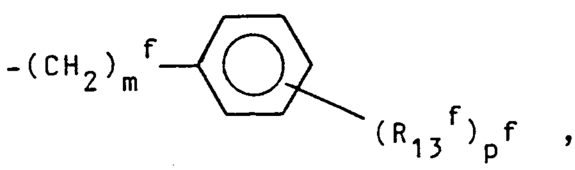

ou les groupes $R_{16}{}^{f}$ se joignent pour former un noyau non substitué à 5 ou 6 membres ou bien ledit noyau dans lequel un ou plusieurs des atomes de carbone a un substituant alkyle inférieur de 1 à 4 atomes de carbone ou un substituant di(alkyle inférieur de 1 à 4 atomes de carbone).

$R_{4}{}^{f}$ est hydrogène, alkyle inférieur,

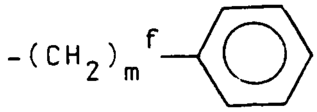, $-(CH_2)_m^f$-cycloalkyle,

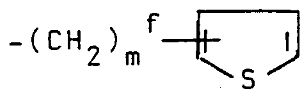, 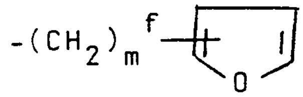,

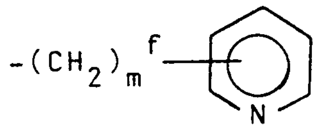, ou 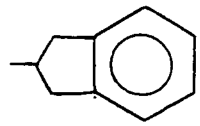

$R_5^f$ est hydrogène, alkyle inférieur,

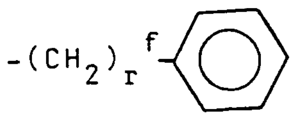, 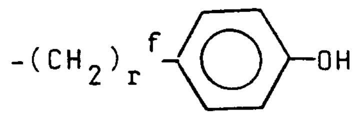,

$-(CH_2)_r^f-OH$, 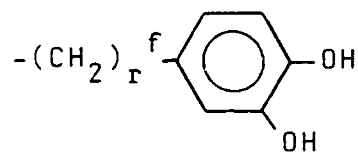,

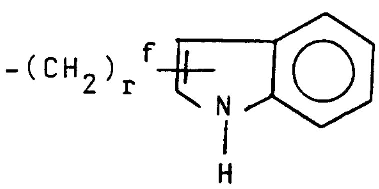, 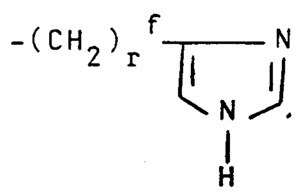,

$-(CH_2)_r^f-NH_2-(CH_2)_r^f-SH$, $-(CH_2)_r^f$-alkyle inférieur

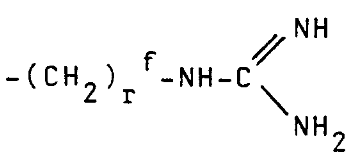, ou 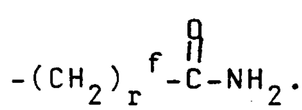.

$r^f$ est un nombre entier de 1 à 4.
$R_{19}^f$ est alkyle inférieur, benzyle ou phénéthyle.
$R_{20}^f$ est hydrogène, alkyle inférieur, benzyle ou phénéthyle.
$R^f$ est hydrogène, alkyle inférieur, cycloalkyle,

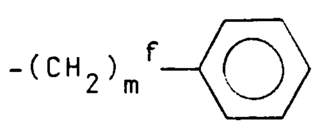,

$-(CH_2)_2-NH_2$, $-(CH_2)_3-NH_2$, $-(CH_2)_4-NH_2$, $-(CH_2)_2-OH$, $-(CH_2)_3-OH$, $-(CH_2)_4-OH$, $-(CH_2)_2-SH$, $-(CH_2)_3-SH$, ou $-(CH_2)_4-SH$.

$R_1^f$ est hydrogène, alkyle inférieur, alkyle inférieur halo-substitué,

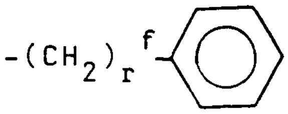 , 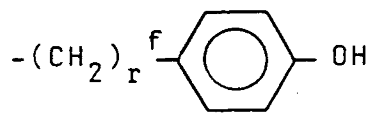 ,

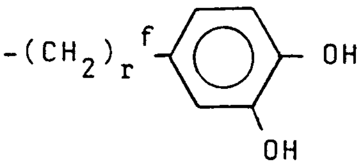 , 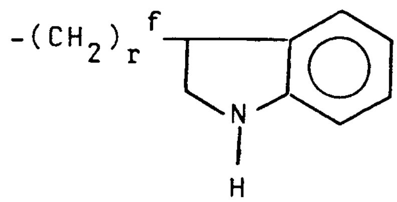 ,

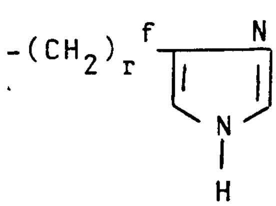 , $-(CH_2)_r^f-NH_2$, $-(CH_2)_r^f-SH$ ,

$-(CH_2)_r^f-OH$, $-(CH_2)_r^f$-S-alkyle inférieur,

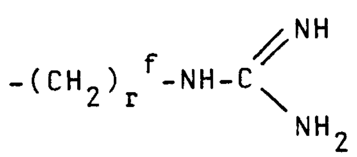 , ou 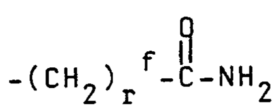

à condition que $R_1^f$ soit hydrogène uniquement si $R^f$ est autre qu'hydrogène.

$R_2^f$ est

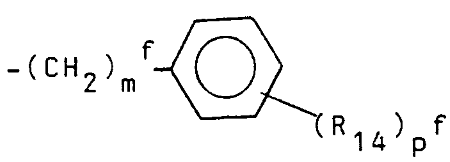 , 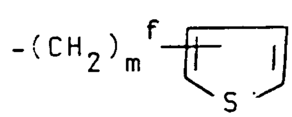 ,

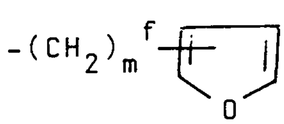 , ou 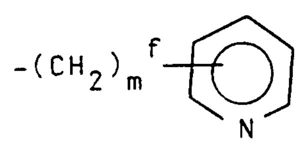

$R_3^f$ est hydrogène, alkyle inférieur,

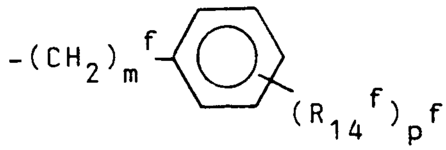 , 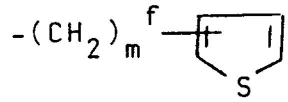 ,

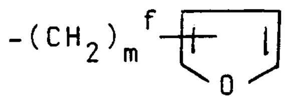 , 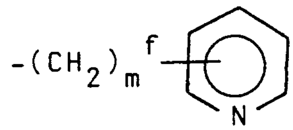 ,

alkyle inférieur, $-(CH_2)_{m}{}^{f}$-cycloalkyle,

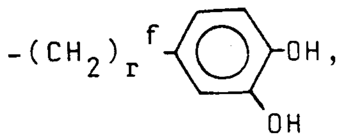

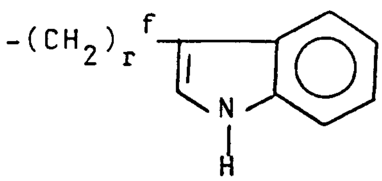 , $-(CH_2)_r{}^f-OH$ ,

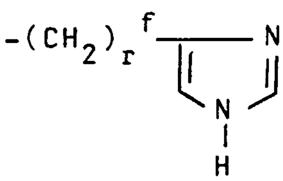 , $-(CH_2)_r{}^f-NH_2$, $-(CH_2)_r{}^f-SH$,

$-(CH_2)_r{}^f$-S-alkyle inférieur, 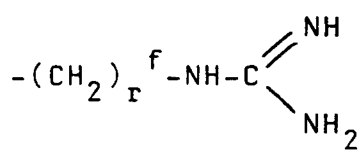 ,

ou 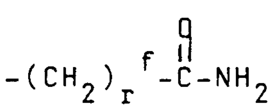

où $m^f$, $R_{14}{}^f$, $p^f$ et $r^f$ sont tels que définis ci-dessus.
$R_6{}^f$ est hydrogène, alkyle inférieur, benzyle, benzhydryle,

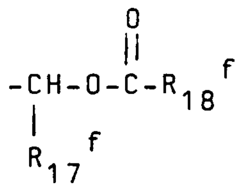 , 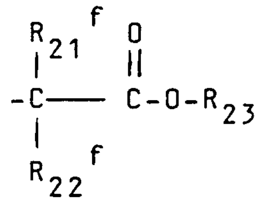 , $-CH-(CH_2-OH)_2$ ,

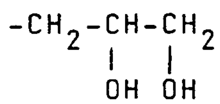 , $-(CH_2)_2-N(CH_3)_2$ , ou 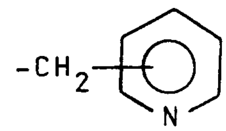

$R_{17}{}^f$ est hydrogène, alkyle inférieur, cycloalkyle ou phényle.
$R_{18}{}^f$ est hydrogène, alkyle inférieur, alcoxy inférieur ou phényle ou bien $R_{17}{}^f$ et $R_{18}{}^f$ pris ensemble sont $-(CH_2)_2-$, $-(CH_2)_3-$, $-CH=CH-$ ou

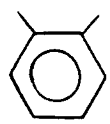 ,

$R_{21}{}^f$ et $R_{22}{}^f$ sont indépendamment choisis parmi hydrogène et alkyle inférieur,
$R_{23}{}^F$ est alkyle inférieur,
$R_{24}{}^f$ est hydrogène, alkyle inférieur,

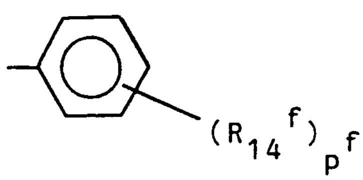 ,

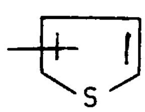 , 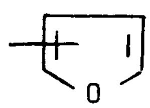 , ou 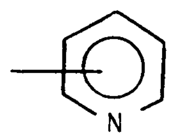

(g) ayant pour formule

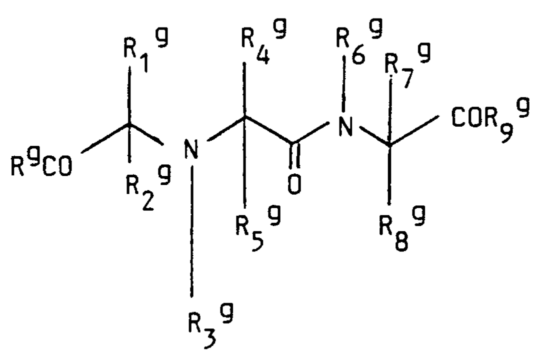

où
soit

(A) $R^g$ et $R_9{}^g$ sont OH, alcoxy 1-6C, alkényloxy 2-6C, di-(alkyl 1-6C)amino-alcoxy(1-6C), hydroxyalcoxy 1-6C, acylamino-alcoxy(1-6C), acyloxy-alcoxy(1-6C), aryloxy, aryloxy-alcoxy(1-6C), $NH_2$, mono- ou di-alkylamino(1-6C), hydroxyamino ou aryl-alkylamino(1-6C) ;

$R_1{}^g$-$R_5{}^g$, $R_7{}^g$ et $R_8{}^g$ sont alkyle 1-20C, alkényle 2-20C, alkynyle 2-20C, aryle, aryl-alkyle (1-6C) ayant 7-12C ou hétérocyclique-alkyl(1-6C) ayant 7-12 atomes de C.

$R_6{}^g$ est cycloalkyle, polycycloalkyle, cycloalkyle ou polycycloalkyle partiellement saturé, cycloalkylalkyle(1-6C) ayant 3-20C, aryle 6-10C, aryl-alkyle(1-6C), aryl-alkényle(2-6C) ou aryl-alkynyle(2-6C) ; ou

$R_2{}^g$ et $R_3{}^g$, avec les atomes de C et de N auxquels ils sont attachés, ou bien $R_3{}^g$ et $R_5{}^g$, avec les atomes de N et de C auxquels ils sont attachés, forment un N-hétérocycle contenant 3-5C ou 2-4C et un atome de S ;

tous les alkyle, alkényle et alkynyle sont facultativement substitués par OH, alcoxy 1-6C, SH, alkylthio 1-6C, $NH_2$, mono- ou di-(alkyl 1-6C)-amino, halogène ou $NO_2$ ;

tous les groupes cycloalkyle (comprenant poly et partiellement insaturés) sont facultativement substitués par halogène, hydroxyalkyle 1-6C, alcoxy 1-6C, amino-alkylamino (1-6C),di(alkyl 1-6C)-amino, SH, alkylthio 1-6C, $NO_2$ ou $CF_3$ ; et les groupes aryle sont facultativement substitués par OH, alcoxy 1-6C, $NH_2$, mono- ou di-(alkyl 1-6C)amino, SH, alkylthio 1-6C, hydroxyalkyle 1-6C, aminoalkyle 1-6C, thioalkyle 1-6C, $NO_2$, halogène, $CF_3$, $OCH_2O$, uréido ou guanidino ; ou bien

(B) $R^g$ et $R_9{}^g$ sont H ou alcoxy 1-6C ;

$R_1{}^g$ et $R_2{}^g$ sont H, alkyle 1-6C,aryl-alkyl (1-6C) ayant 7-12 atomes de carbone ou bien hétérocyclyl-alkyle (1-6C) ayant 6-12 atomes de carbone ;

$R_3{}^g$-$R_5{}^g$, $R_7{}^g$ et $R_8{}^g$ sont H ou alkyle 1-6C ;

$R_6{}^g$ est cycloalkyle, polycycloalkyle, cycloalkyle ou polycycloalkyle partiellement saturé, cycloalkylalkyle(1-6C) ayant 3-20 atomes de carbone, aryle ou aryl-alkyle(1-6C) ; et

aryle a 6-10C et est facultativement substitué par alkyle 1-6C, alkényle 2-6C, alkynyle 2-6C, OH, alcoxy 1-6C, $NH_2$ , mono- ou di-(alkyl 1-6C)amino, SH, alkylthio 1-6C, hydroxyalkyle 1-6C , aminoalkyle 1-6C, thioalkyle 1-6C, $NO_2$, halogène, $CF_3$, $OCH_2O$, uréido ou guanidino :

(h) ayant pour formule

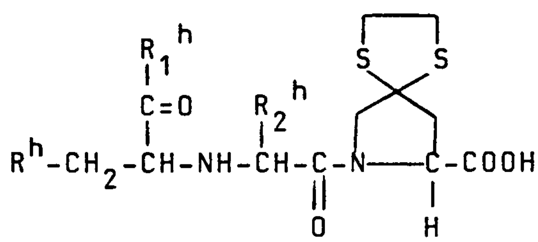

où

$R^h$ est alkyle inférieur, benzyle, benzylthio, benzyloxy, phénylthio ou phénoxy ;

$R_1{}^h$ est hydroxy ou alcoxy inférieur ;

$R_2{}^h$ est hydrogène, alkyle inférieur ou aminoalkyle inférieur ; et leurs sels acceptables en pharmacie;

(i) ayant pour formule

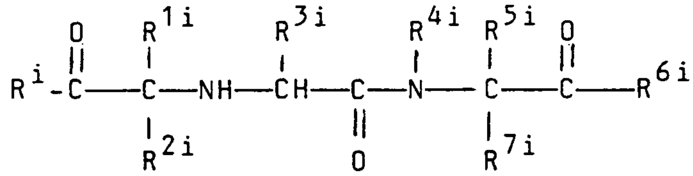

ou un sel acceptable en pharmacie, où $R^i$ et $R^{6i}$ sont identiques ou différents et sont hydroxy, alcoxy inférieur, alkényloxy inférieur, dialkylamino inférieur alcoxy inférieur, acylamino alcoxy inférieur, acyloxy alcoxy inférieur, aryloxy, arylalcoxy inférieur, amino, alkylamino inférieur, dialkylamino inférieur, hydroxyamino, arylalkylamino inférieur, ou aryloxy substitué ou arylalcoxy inférieur substitué où le substituant est méthyle, halo ou méthoxy ; $R^{1i}$ est hydrogène, alkyle de 1 à 10 atomes de carbone,

alkyle inférieur substitué où le substituant est hydroxy, alcoxy inférieur, aryloxy, aryloxy substitué , hétéroaryloxy, hétéroaryloxy substitué, amino, alkylamino inférieur, dialkylamino inférieur, acylamino, arylamino, arylamino substitué, guanidino, imidazolyle, indolyle, alkylthio inférieur, arylthio, arylthio substitué, carboxy, carbamoyle, alcoxycarbonyle inférieur, aryle, aryle substitué, aralkyloxy, aralkyloxy substitué, aralkylthio ou aralkylthio substitué, où la portion aryle ou hétéroaryle dudit groupe substitué aryloxy, hétéroaryloxy, arylamino, arylthio, aryle, aralkyloxy, aralkylthio est substituée par un groupe choisi parmi halo, alkyle inférieur, hydroxy, alcoxy inférieur, amino, aminométhyle, carboxyle, cyano ou sulfamoyle ; $R^{2i}$ et $R^{7i}$ sont identiques ou différents et sont hydrogène ou alkyle inférieur ; $R^{3i}$ est hydrogène, alkyle inférieur, phényl-alkyle inférieur, aminométhylphényl-alkyle inférieur, hydroxyphényl-alkyle inférieur, hydroxy-alkyle inférieur, acylamino-alkyle inférieur, aminoalkyle inférieur, diméthylamino-alkyle inférieur, guanidino-alkyle inférieur, imidazolyl-alkyle inférieur, indolyl-alkyle inférieur ou alkyl inférieur thio-alkyle inférieur ; $R^{4i}$ et $R^{5i}$ sont identiques ou différents et sont hydrogène, alkyle inférieur ou bien $Z^i$ ou bien $R^{4i}$ et $R^{5i}$ ,pris ensemble,forment un groupe représenté par $Q^i$, $U^i$, $V^i$, $Y^i$, $D^i$ ou $E^i$, où :

$Z^i$ est

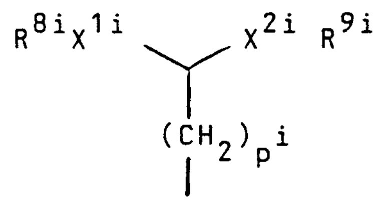

où $X^{1i}$ et $X^{2i}$ sont, indépendamment l'un de l'autre,O, S ou $CH_2$, $R^{8i}$ et $R^{9i}$ sont, indépendamment l'un de l'autre, alkyle inférieur, alkényle inférieur, alkynyle inférieur, cycloalkyle ayant 3 à 8 atomes de carbone, hydroxy-alkyle inférieur ou $-(CH_2)_nAr^i$, où $n^i$ est 0,1, 2 ou 3 et $Ar^i$ est phényle, furyle, thiényle ou pyridyle non substitué ou substitué où lesdits groupes phényle, furyle, thiényle ou pyridyle substitués sont substitués par au moins un groupe qui est indépendamment choisi parmi alkyle $C_1$ à $C_4$, alcoxy inférieur, alkylthio inférieur, halo, $CF_3$ et hydroxy, ou bien $R^{8i}$ et $R^{9i}$ , pris ensemble, forment un pont $W^i$, où $W^i$ est une simple liaison ou un pont méthylène ou bien un pont méthylène substitué quand au moins l'un de $X^{1i}$ et $X^{2i}$ est méthylène ou bien $W^i$ est un alkylène ou un pont alkylène substitué ayant 2 ou 3 atomes de carbone, ledit pont méthylène substitué ou ledit pont alkylène substitué ayant un ou deux substituants choisis parmi les groupes alkyle inférieur, aryle et aryl-alkyle inférieur, et $p^i$ est 0, 1 ou 2 ; à condition qu'au moins l'un de $R^4$ et $R^5$ soit $Z^i$, à condition que si $R^4$ est $Z^i$ et $p^i$ est 0 alors $X^{1i}$ et $X^{2i}$ soient tous deux méthylène et à condition que si $X^{1i}$ et $X^{2i}$ sont deux méthylène, alors $R^{8i}$ et $R^{9i}$ forment un pont alkylène $W^i$ ;

$Q^i$ est

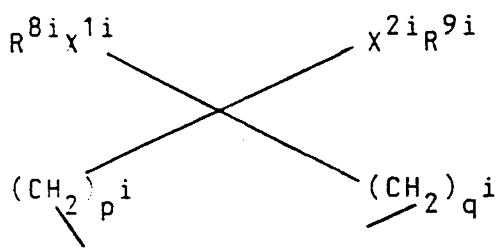

où $R^{8i}$, $R^{9i}$, $X^{1i}$ et $X^{2i}$ sont tels que définis ci-dessus, $p^i$ est 0, 1 ou 2, $q^i$ est 0, 1 ou 2, à condition que la somme de $p^i$ et $q^i$ soit 1, 2 ou 3, à condition que si $p^i$ est 0, $X^{1i}$ et $X^{2i}$ soient méthylène, et à condition que si $X^1$ et $X^{2i}$ sont méthylène alors $R^{8i}$ et $R^{9i}$, pris ensemble, forment un pont $W^i$, où $W^i$ est tel que défini ci-dessus ;

$V^i$ est

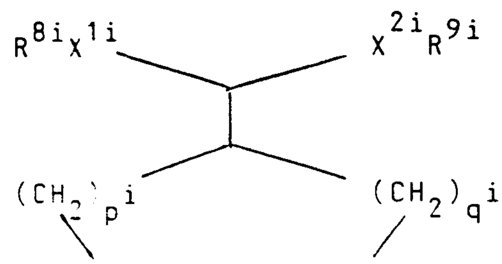

où $R^{8i}$, $R^{9i}$, $X^{1i}$ et $X^{2i}$ sont tels que définis ci-dessus, $p^i$ est 0, 1 ou 2 et $q^i$ est 0, 1 ou 2, à condition que la somme de $p^i$ et $q^i$ soit 1, 2 ou 3, à condition que si $X^{1i}$ et $X^{2i}$ sont $CH_2$, alors $R^{8i}$ et $R^{9i}$, pris ensemble, forment un pont $W^i$ où $W^i$ est tel que défini ci-dessus;
$U^i$ est

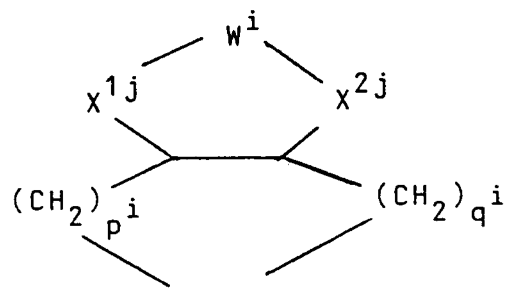

où $W^i$ est tel que défini ci-dessus (à l'exception que $W^i$ peut également être un pont méthylène quand $X^{1i}$ et $X^{2i}$ sont oxygène ou soufre), $X^{1i}$ et $X^{2i}$ sont tels que définis ci-dessus, $p^i$ est 0, 1 ou $q_i$ est 0, 1 ou 2, à condition que la somme de $p^i$ et $q^i$ soit 1 ou 2 et à condition que si $p^i$ est O, $X^{1i}$ soit $CH_2$ ;
$Y^i$ est

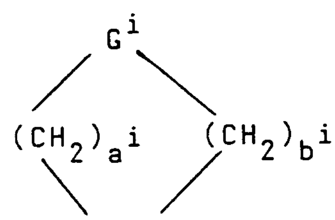

où $G^i$ est oxygène, soufre ou $CH_2$, $a^i$ est 2, 3 ou 4 et $b^i$ est 1, 2, 3, 4 ou 5, à condition que la somme de $a^i$ et $b^i$ soit 5, 6 ou 7 ou
$G^i$ est $CH_2$, $a^i$ est 0, 1, 2 ou 3, $b^i$ est 0, 1 , 2 ou 3, à condition que la somme de $a^i$ et $b^i$ soit 1, 2 ou 3, à condition que la somme de $a^i$ et $b^i$ puisse être 1, 2 ou 3 uniquement si $R^{1i}$ est alkyle inférieur substitué par alkylthio ou aralkyloxy ;
$D^i$ est

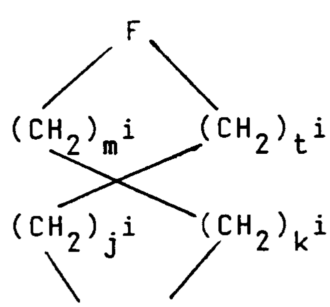

où $F^i$ est O ou S, $j^i$ est 0, 1 ou 2 et $k^i$ est 0, 1 ou 2 à condition que la somme de $j^i$ et $k^i$ soit 1, 2 ou 3 et $m^i$ est 1, 2 ou 3 et $t^i$ est 1, 2 ou 3, à condition que la somme de $m^i$ et $t^i$ soit 2, 3 ou 4 ;
$E^i$ est

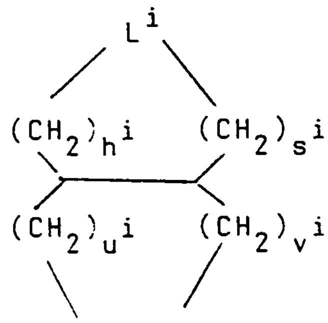

où $L^i$ est O ou S, $u^i$ est 0, 1 ou 2 et $v^i$ est 0, 1 ou 2, à condition que la somme de $u^i$ et $v^i$ soit 1 ou 2 et $h^i$ est 1 ou 2 et $s^i$ est 1 ou 2, à condition que la somme de $h^i$ et $s^i$ soit 2 ou 3.
(j) ayant pour formule

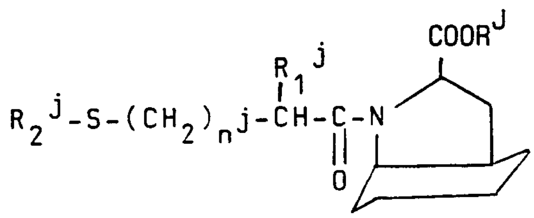

où $R^j$ est hydrogène ou alkyle inférieur ; $R_1{}^j$ est hydrogène, alkyle inférieur ou benzyle ; $R_2{}^j$ est hydrogène, ou bien

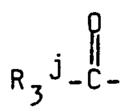

où $R_3{}^j$ est alkyle inférieur, hétéroaryle contenant 4 à 9 atomes de carbone et un ou deux atomes d'azote, d'oxygène ou de soufre ; phényle, phényle substitué ayant 1 ou 2 substituants choisis parmi fluor, chlore, brome, alkyle ou alcoxy inférieur ;
et $n^j$ est 0 ou 1 ; où alkyle inférieur et alcoxy inférieur comprennent des groupes droits ou ramifiés contenant 1 à 4 atomes de carbone, et
les sels acceptables en pharmacie des composés quand $R^j$ est hydrogène et quand $R_3{}^j$ est hétéroaryle contenant 1 ou 2 atomes d'azote, sont révélés ;
(k) ayant pour formule

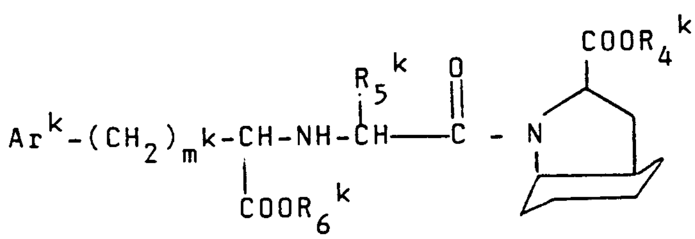

où $R_4{}^k$ est hydrogène ou alkyle inférieur ; $R_5{}^k$ est hydrogène, alkyle inférieur ou benzyle ; $R_6{}^k$ est hydrogène ou alkyle inférieur ; $Ar^k$ est phényle ou phényle substitué par 1 ou 2 substituants choisis

parmi fluor, chlore, brome, alkyle inférieur, alcoxy inférieur, hydroxy ou amino ; et m est 0 à 3 ; où alkyle inférieur et alcoxy inférieur contiennent 1 à 4 atomes de carbone droits ou ramifiés ; et leurs sels acceptables en pharmacie ;

(l) ayant les formules

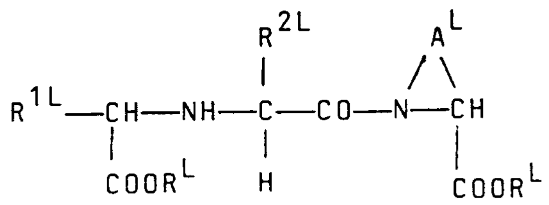

$(I)^L$

et

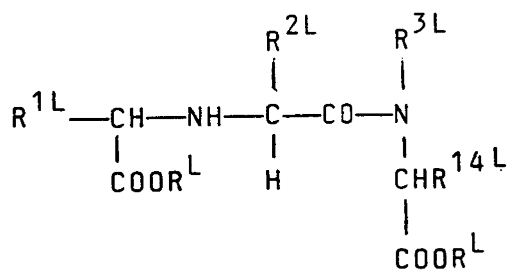

$(Ia)^L$

où :

$R^L$ et $R^{2L}$ sont indépendamment hydrogène ; alkyle inférieur; aralkyle ou aryle ;

$R^{1L}$ est hydrogène ; alkyle $C_{1-12}$ à chaîne droite ou ramifiée et alkényle ; cycloalkyle $C_3$-$C_9$ et alkyle benzo-fusionné ; alkyle inférieur substitué où les substituants sont halo, hydroxyalcoxy inférieur, aryloxy, amino, mono- ou di-alkyl inférieur amino, mono- ou di-alkyl inférieur amino, acylamino, arylamino, guanidino, mercapto , alkylthio inférieur, arylthio, carboxy, carboxamido ou alcoxycarbonyle inférieur ; aryle; aryle substitué où les substituants sont alkyle inférieur, alcoxy inférieur ou halo ; aralkyle inférieur ; aralkényle inférieur ; hétéroaralkyle inférieur; hétéroaralkényle inférieur ; aralkyle inférieur substitué, aralkényle inférieur substitué, hétéroaralkyle inférieur substitué ou hétéroaralkényle inférieur substitué où les substituants aryle et hétéroaryle sont halo, dihalo, alkyle inférieur, hydroxy, alcoxy inférieur, amino, aminoalkyle inférieur, acylamino, mono- ou di-alkylamino inférieur, carboxyle, haloalkyle inférieur, nitro, cyano ou sulfonamido et où la portion alkyle inférieur ou aralkyle inférieur peut être substituée par amino, acylamino ou hydroxyle ;

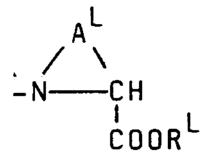 est 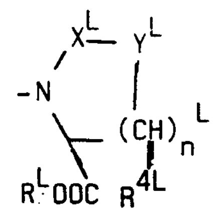 , 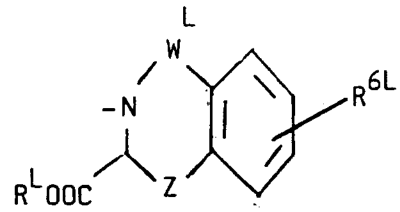

ou 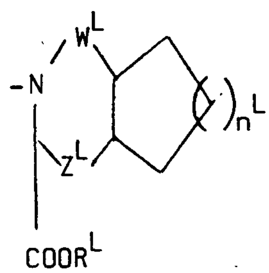

où

$X^L$ et $Y^L$ , pris ensemble, sont $-CH_2-CH_2-$ ;

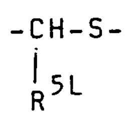 ; 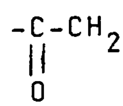 ; 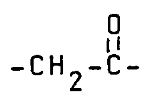 ; 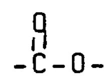 ; 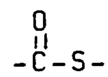 ;

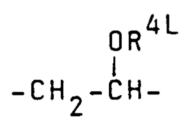 ; 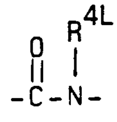 ; ou 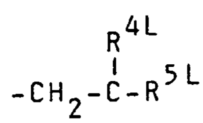 ;

$R^{4L}$ est hydrogène, alkyle inférieur ; aryle ; aryle substitué ;

$R^{5L}$ est hydrogène ; alkyle inférieur ; aryle ou aryle substitué ;

$n^L$ est 1 à 3 ;

$W^L$ est absent ; $-CH_2$ ; ou

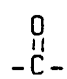

$Z^L$ est $-(CH_2)_m{}^L$, où $m^L$ est 0 à 2, à condition que $m^L$ ne soit pas O et $W^L$ ne soit pas absent en même temps; et

$R^{6L}$ est hydrogène ; alkyle inférieur ; halo ; ou $OR^{4L}$

$R^{2L}$ est $-(CH_2)_r{}^L-B^L-(CH_2)_s{}^L-NR^{7L}R^{15L}$

où

$r^L$ et $s^L$ sont indépendamment 0 à 3 ; $B^L$ est absent ; -O- ; -S- : ou $-NR^{8L}$ ;

où $R^{8L}$ est hydrogène ; alkyle inférieur ; alcanoyle ou aroyle ; et

$R^{7L}$ est

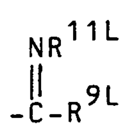 ; 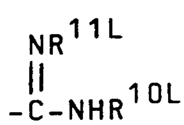 ; ou 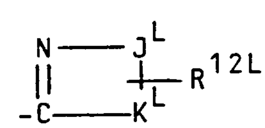

où

| | |
|---|---|
| $R^{9L}$ est | alkyle inférieur ; aralkyle ; aryle; hétéroaryle ou hétéroaralkyle inférieur et ces groupes sont substitués par hydroxy, alcoxy inférieur ou halo ; carboxyle ; carboxamido ; nitrométhényle. |
| $R^{10L}$ est | hydrogène ; alkyle inférieur ; aryle ou amidino ; |
| $R^{11L}$ est | hydrogène ; alkyle inférieur ; cyano ; amidino; aryle ; aroyle ; alcanoyle inférieur ; |

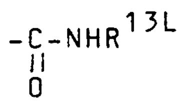 ; 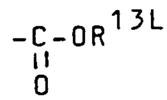 ;

$-NO_2$ ; $-SO_2NH_2$ ;
ou
$SO_2R^{13L}$ ;

| | |
|---|---|
| $R^{12L}$ est | hydrogène ; alkyle inférieur ; halo ; aralkyle; amino ; cyano ; mono- ou di-alkylamino inférieur; ou $OR^{4L}$ ; |
| $R^{13L}$ est | hydrogène ; alkyle inférieur ; ou aryle. |
| $R^{15L}$ est | hydrogène ; alkyle inférieur ; aralkyle ; ou aryle |

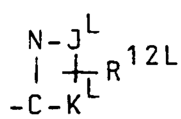

constitue un hétérocycle basique de 5 ou 6 atomes ou leurs analogues benzo-fusionnés et contenant facultativement 1-3 atomes de N, un atome d'oxygène un de soufre, un groupe SO-ou un groupe $SO_2$- facultativement substitué par des groupes amino, alkylamino inférieur, dialkylamino inférieur, alcoxy inférieur ou aralkyle ;

| | |
|---|---|
| $R^{3L}$ est | cycloalkyle $C_{3-8}$ et cycloalkyle $C_{3-8}$ benzofusionné ; cycloalkyle $C_{3-8}$ perhydrobenzofusionné ; aryle ; aryle substitué ; hétéroaryle ; hétéroaryle substitué ; |
| $R^{14L}$ est | hydrogène ou alkyle inférieur ; et un sel acceptable en pharmacie ; |

(m) ayant pour formule

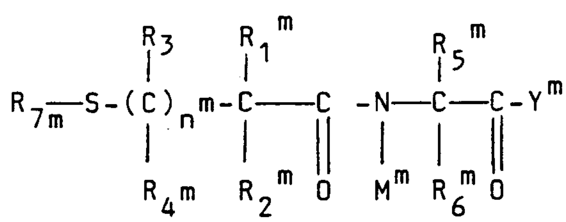

où
$R_1{}^m$, $R_2{}^m$, $R_3{}^m$, $R_4{}^m$, $R_5{}^m$ et $R_6{}^m$ sont hydrogène, alkyle, alkényle, alkynyle, phényl-alkyle et cycloalkyle et peuvent être identiques ou différents,
$n^m$ est un nombre entier de 0 à 4 inclus,
$M^m$ est alkényle, alkynyle, cycloalkyle, cycloalkyl-alkyle, polycycloalkyle, polycycloalkyl-alkyle, aryle, aralkyle, hétéroaryle, hétéroaryle-alkyle, hétérocycloalkyle, hétérocycloalkyl-alkyle, aryl-cycloalkyle fusionné, arylcycloalkyl-alkyle fusionné, hétéroaryle-cycloalkyle fusionné, hétéroaryl-cycloalkyl-alkyle fusionné, alcoxy-alkyle, alkylthio-alkyle, alkylamino-alkyle ou dialkylamino-alkyle,
$Y^m$ est hydroxy, alcoxy, amino ou aminoalcanoyle, aryloxy, aminoalcoxy, hydroxyalcoxy ou amino substitué et
$R_7{}^m$ est hydrogène, alcanoyle, carboxyalcanoyle, hydroxyalcanoyle, aminoalcanoyle, cyano, amidino, carbalcoxy, $Z^mS$ ou

$Z^mSCO$-

où $Z^m$ est hydrogène, alkyle, hydroxyalkyle ou bien le radical

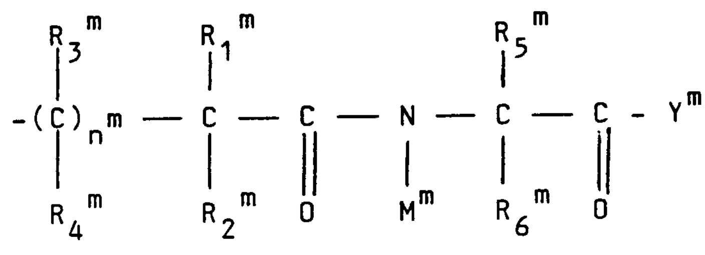

où $R_1{}^m$, $R_2{}^m$, $R_3{}^m$, $R_4{}^m$, $R_5{}^m$, $R_6{}^m$, $n^m$, $M^m$ et $Y^m$ sont tels que décrits ci-dessus ; et où $Y^m$ est hydroxy, leurs sels de métal alcalin, de métal alcalino-terreux et d'amine non toxiques et acceptables en pharmacie ; ou
(n) ayant pour formule

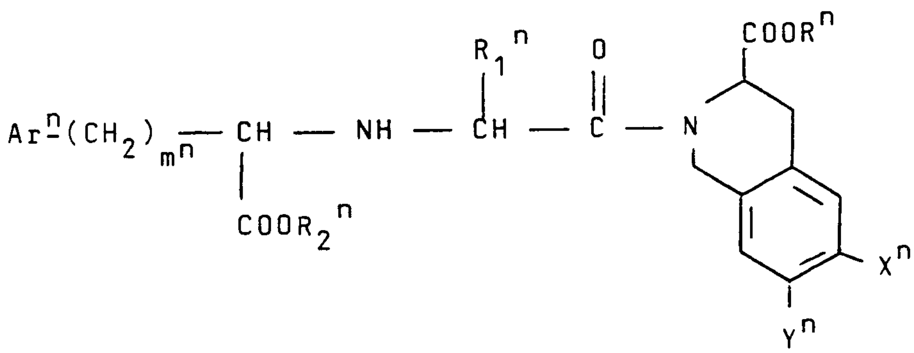

où $R^n$ est hydrogène, alkyle inférieur ou aralkyle ;
$R_1{}^n$ est hydrogène, alkyle inférieur ou benzyle ;
$R_2{}^n$ est hydrogène ou alkyle inférieur et Ar est phényle ou phényle substitué par 1 ou 2 substituants choisis parmi fluor, chlore, brome, alkyle inférieur, alcoxy inférieur, hydroxy ou amino ;X et Y sont indépendamment hydrogène, alkyle inférieur, alcoxy inférieur, alkylthio inférieur, alkylsulfinyle inférieur, alkylsulfonyle inférieur, hydroxy, ou X et Y, ensemble, sont méthylènedioxy; m est 0 à 3 ; et leurs sels d'acide acceptables en pharmacie ;
ayant de préférence la formule telle que définie au paragraphe (i) et étant en particulier acide 7-[N-(1(S)-carboxy-3-phénylpropyl)-(S)-alanyl]-1,4-dithia-7-azaspiro[4.4]nonane-8(S)-carboxylique.
(o) ayant pour formule

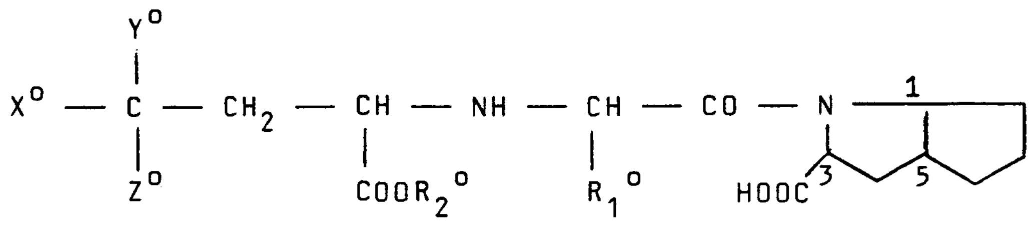

les atomes d'hydrogène aux positions 1 et 5 sur le noyau sont cis l'un à l'autre et le groupe 3-carboxy a l'orientation endo ;
$R_1{}^o$ est H, allyle, vinyle ou bien la chaîne latérale d'un acide $\alpha$-aminé naturel éventuellement protégé ;
$R_2{}^o$ est H, alkyle 1-6C, alkényle 2-6C ou aryl(alkyle 1-4C) ;
$Y^o$ est H ou OH et $Z^o$ est H, ou bien $Y^o$ et $Z^o$ sont ensemble oxygène ;
$X^o$ est alkyle 1-6C, alkényle 2-6C, cycloalkyle 5-9C, aryle 6-12C (facultativement substitué par un à trois alkyle ou alcoxy 1-4C, OH, halo, nitro, amino (facultativement substitué par un ou deux alkyle 1-

4C) ou méthylènedioxy ou indol-3-yle ;
(p) ayant pour formule

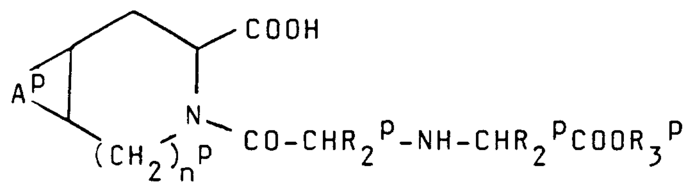

$n^p$ est 0 ou 1 ;

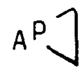

est un noyau benzène ou cyclohexane ; chacun de $R_1{}^p$ et $R_2{}^p$ est alkyle 1-6C, alkényle 2-6C, cycloalkyle 5-7C, cycloalkényle 5-7C, cycloalkyl-alkyle 7-12C, aryle 6-10C éventuellement partiellement hydrogéné, aralkyle 7-14C ou hétérocyclyle monocyclique à 5-7 membres ou bicyclique à 8-10 membres contenant 1 ou 2 S ou 0 et/ou 1-4 atomes de N ; tous les groupes $R_1{}^p$ et $R_2{}^p$ sont facultativement substitués.
$R_3{}^p$ est H, alkyle 1-6C, alkényle 2-6C ou aralkyle 7-14C.

**3.** Composition selon la revendication 1 ou 2, où le bêta bloquant adrénergique est aténolol, métoprolol, nadolol, pindolol, propanolol, timolol, labétalol, bétaxolol, cartéolol, dilévolol ou butonolol, de préférence timolol.

**4.** Composition selon l'une quelconque des revendications 1 à 3 où, pour une quantité de 0,005 µg à 50 µg de l'inhibiteur de ACE, il y a une quantité de 5 µg à 500 µg du bêta bloquant adrénergique, les proportions suivantes étant préférées
(a) 50 µg à 250 µg (en particulier 50 µg à 125 µg) du bêta bloquant adrénergique pour 0,025 µg à 5 µg (en particulier 0,05 µg à 0,5 µg) de l'inhibiteur de ACE ;
(b) 5 µg à 125 µg (en particulier 50 µg) du bêta bloquant adrénergique pour 0,005 µg à 1 µg (en particulier 0,05 µg à 0,5 µg) de l'inhibiteur de ACE ; et
(c) 25 µg à 500 µg (en particulier 50 µg à 250 µg) du bêta bloquant adrénergique pour 0,005 µg à 1 µg (en particulier 0,005 µg à 0,05 µg) de l'inhibiteur de ACE.

**5.** Nécessaire comprenant, dans des conteneurs séparés, des compositions pharmaceutiques pour une thérapie en association afin de réduire et/ou de contrôler la pression intra-oculaire élevée associée au glaucome chez les mammifères qui comprend, dans le premier conteneur, une composition pharmaceutique ophtalmologique topique comprenant un inhibiteur de l'enzyme de conversion de l'angiotensine ophtalmologiquement acceptable et, dans le second conteneur, une composition topique ophtalmologique pharmaceutique comprenant un bêta bloquant adrénergique ophtalmologiquement acceptable.

**7.** Nécessaire selon la revendication 5, où l'inhibiteur de ACE est un inhibiteur de ACE tel que défini à la revendication 2, de préférence l'acide [7-N-(1(S)-carboxy-3-phénylpropyl)-(S)-alanyl]-1,4-dithia-7-azaspiro[4.4]nonane-8(S)-carboxylique.

**7.** Nécessaire selon la revendication 5 ou 6, où le bêta bloquant adrénergique est aténolol, métoprolol, nadolol, pindonol, propanolol, timolol, labétalol, bétaxolol, cartéolol, dilevolol ou bunolol , de préférence timolol.

**8.** Nécessaire selon l'une quelconque des revendications 5 à 7 où, pour une quantité de 0,005 µg à 50 µg de l'inhibiteur de ACE, il y a une quantité de 5 µg à 500 µg du bêta bloquant adrénergique, les proportions suivantes étant préférées :
(a) 50 µg à 250 µg (en particulier 50 µg à 125 µg) du bêta bloquant adrénergique pour 0,025 µg à 5 µg (en particulier 0,05 µg à 0,5 µg) de l'inhibiteur de ACE ;
(b) 5 µg à 125 µg (en particulier 50 µg) du bêta bloquant adrénergique pour 0,005 µg à 1 µg (en particulier 0,05 µg à 0,5 µg) de l'inhibiteur de ACE ; et
(c) 25 µg à 500 µg (en particulier 50 µg à 250 µg) du bêta bloquant adrénergique pour 0,005 µg à 1 µg (en particulier 0,005 µg à 0,05 µg) de l'inhibiteur de ACE.

**9.** Nécessaire comprenant (a) une composition topique ophtalmologique pharmaceutique comprenant un inhibiteur de l'enzyme de conversion de l'angiotensine ophtalmologiquement acceptable et (b) une

composition topique ophtalmologique pharmaceutique comprenant un bêta bloquant adrénergique ophtalmologiquement acceptable avec (c) les instructions d'utilisation des compositions pour une thérapie en association afin de réduite et/ou de contrôler la pression intra-oculaire élevée associée au glaucome chez les mammifères.

**10.** Nécessaire selon la revendication 9, où l'inhibiteur de ACE est un inhibiteur de ACE tel que défini à la revendication 2, de préférence

acide 7-[N-(1(S)-carboxy-3-phénylpropyl)-(S)-alanyl]-1,4-dithia-7-azaspiro[4.4]nonane-8(S)-carboxylique.

**11.** Nécessaire selon la revendication 9 ou 10, où le bêta bloquant adrénergique est aténolol, métoprolol, nadolol, pindolol, propanolol timolol, labétalol, bétaxolol, cartéolol, dilévolol ou bunolol , de préférence timolol.

**12.** Nécessaire selon l'une quelconque des revendications 9 à 11 où, pour une quantité de 0,005 µg à 50 µg de l'inhibiteur de ACE, il y a une quantité de 5 µg à 500 µg du bêta bloquant adrénergique, les proportions suivantes étant préférées

(a) 50 µg à 250 µg (en particulier 50 µg à 125 µg) du bêta bloquant adrénergique pour 0,025 µg à 5 µg (en particulier 0,05 µg à 0,5 ug) de l'inhibiteur de ACE ;

(b) 5 µg à 125 µg (en particulier 50 µg) du bêta bloquant adrénergique pour 0,005 µg à 1 µg (en particulier 0,05 µg à 0,5 µg) de l'inhibiteur de ACE ; et

(c) 25 µg à 500 µg (en particulier 50 µg à 250 µg) du bêta bloquant adrénergique pour 0,005 µg à 1 µg (en particulier 0,005 µg à 0,05 µg) de l'inhibiteur de ACE.

**13.** Utilisation d'un inhibiteur de ACE ophtalmologiquement acceptable et d'un bêta bloquant adrénergique ophtalmologiquement acceptable pour la préparation de médicaments pour une thérapie en association afin de réduire et/ou de contrôler la pression intra-oculaire élevée associée au glaucome.

**14.** Utilisation selon la revendication 13, où l'inhibiteur de ACE qui est utilisé est un inhibiteur de ACE tel que défini à la revendication 2, de préférence

acide 7-[N-(1(S)-carboxy-3-phénylpropyl)-(S)-alanyl]-1,4-dithia-azaspiro[4.4]nonane-8(S)-carboxylique.

**15.** Utilisation selon la revendication 13 ou 14, où le bêta bloquant adrénergique est aténolol, métoprolol, nadolol, pindolol, propanolol, timolol, labétalol, bétaxolol, cartéolol, dilevolol ou bunolol, de préférence timolol.

**16.** Utilisation selon l'une quelconque des revendications 13 à 15 où, pour une quantité de 0,005 µg à 50 µg de l'inhibiteur de ACE, on utilise une quantité de 5 µg à 500 µg du bêta bloquant adrénergique, les proportions suivantes étant préférées :

(a) 50 µg à 250 µg (en particulier 50 µg à 125 µg) du bêta bloquant adrénergique pour 0,025 µg à 5 µg (en particulier 0,05 µg à 0,5 µg) de l'inhibiteur de ACE ;

(b) 5 µg à 125 µg (en particulier 50 µg) du bêta bloquant adrénergique pour 0,005 µg à 1 µg (en particulier 0,05 µg à 0,5 µg) de l'inhibiteur de ACE ;

(c) 25 µg à 500 µg (en particulier 50 µg à 250 µg) du bêta bloquant adrénergique pour 0,005 µg à 1 µg (en particulier 0,005 µg à 0,05 µg) de l'inhibiteur de ACE.

**17.** Procédé pour la préparation d'une composition topique ophtalmologiquement acceptable, utile pour réduire et/ou contrôler la pression intra-oculaire élevée associée au glaucome, qui comprend le mélange d'un inhibiteur de l'enzyme de conversion de l'angiotensine ophtalmologiquement acceptable et d'un bêta bloquant adrénergique ophtalmologiquement acceptable avec un support ophtalmologiquement acceptable pour usage topique.

**18.** Procédé selon la revendication 17, où l'inhibiteur de ACE est un inhibiteur de ACE tel que défini à la revendication 2, de préférence l'acide [7-N-(1(S)-carboxy-3-phénylpropyl)-(S)-alanyl]-1,4-dithia-7-azaspiro[4.4]nonane-8(S)-carboxylique.

**19.** Procédé selon la revendication 17 ou 18, où le bêta bloquant adrénergique est aténolol, métoprolol, nadolol, pindolol, propanolol, timolol, labétalol, bétaxolol, cartéolol, dilévolol ou bunolol, de préférence timolol.

**20.** Procédé selon l'une quelconque des revendications 17 à 19 où: pour une quantité de 0,005 µg à 50 µg de l'inhibiteur de ACE, il y a une quantité de 5 µg à 500 µg du bêta bloquant adrénergique, les proportions suivantes étant préférées :

(a) 50 µg à 250 µg (en particulier 50 µg à 125 µg) du bêta bloquant adrénergique pour 0,025 µg à 5 µg (en particulier 0,05 µg à 0,5 µg) de l'inhibiteur de ACE ;

(b) 5 µg à 125 µg (en particulier 50 µg) du bêta bloquant adrénergique pour 0,005 µg à 1 µg (en particulier 0,05 µg à 0,5 µg) de l'inhibiteur de ACE ; et

(c) 25 µg à 500 µg (en particulier 50 µg à 250 µg) du bêta bloquant adrénergique pour 0,005 µg à 1 µg (en particulier 0,005 µg à 0,05 µg) de l'inhibiteur de ACE.